# FASCICULE DE BREVET EUROPEEN

(11) **EP 1 062 364 B1**
(45) Date de publication et mention de la délivrance du brevet: **11.05.2005**
(21) Numéro de dépôt: 99909002.0
(22) Date de dépôt: 11.03.1999
(51) Int. Cl.: C12Q 1/68

(54) **CRIBLAGE DIFFERENTIEL QUALITATIF**
QUALITATIVES DIFFERENTIELLES SCREENING
QUALITATIVE DIFFERENTIAL SCREENING

(30) Priorité: 11.03.1998 FR 9802997
(43) Date de publication de la demande: 27.12.2000
(60) Demande divisionnaire: 05003839.7
(73) Titulaire: Exonhit Therapeutics S.A., 75013 Paris (FR)
(72) Inventeur: SCHWEIGHOFFER, Fabien, F-94300 Vincennes (FR); BRACCO, Laurent, F-75013 Paris (FR); TOCQUE, Bruno, F-92400 Courbevoie (FR)
(74) Mandataire: Becker, Philippe
(86) Numéro de dépôt international: PCT/FR1999/000547
(87) Numéro de publication internationale: WO 1999/046403

(56) Documents cités:
- EP-A- 0 330 781
- EP-A- 0 709 397
- EP-A- 0 791 660
- EP-A- 0 806 478
- WO-A-94/12631
- WO-A-95/27052
- WO-A-96/26272
- WO-A-96/30512
- WO-A-97/04092
- WO-A-97/46679
- WO-A-98/02576
- WO-A1-98/42871
- FR-A- 2 664 287
- US-A- 5 679 541
- COOPER D L ET AL: "GENE THERAPY ADVANCES: UTILIZATION OF ALTERNATIVE SPLICING AS A CONTROL ELEMENT IN THE CHIMERIC ENZYME/PRODRUG THERAPY (CEPT) APPROACH TO PRIMARY AND METASTATIC TUMORS" JOURNAL OF CLINICAL LIGAND ASSAY, vol. 19, no. 1, 1996, pages 80-84, XP002038774
- ALPHEY L: "PCR-based method isolation of full-length clones and splice variants from cDNA libraries" BIOTECHNIQUES, vol. 22, 1997, pages 481-486, XP002087172
- ARDLEY H C ET AL.: "Rapid isolation of genomic clones for individual members of human multigene families: Identification and localisation of UBE2L4, a novel member of a ubiquitin conjugating enzyme dispersed gene family" CYTOGENETICS AND CELL GENETICS, vol. 79, 1997, pages 188-192, XP002087173
- BONASS W A ET AL.: "The rat amelogenin gene - some aspects of evolution and expression" ADVANCES IN DENTAL RESEARCH, vol. 10, no. 2, 1996, pages 182-186, XP002087174
- MILLER R D AND RIBLET R: "Improved phenol emulsion DNA reassociation technique (PERT) using thermal cycling" NUCLEIC ACIDS RESEARCH, vol. 23, no. 12, 1995, pages 2339-2340, XP002087175 cité dans la demande
- DIATCHENKO L ET AL: "SUPPRESSION SUBTRACTIVE HYBRIDIZATION: A METHOD FOR GENERATING DIFFERENTIALLY REGULATED OR TISSUE-SPECIFIC CDNA PROBES AND LIBRARIES" PROCEEDINGS OF THE NATIONAL ACADEMY OF SCIENCES OF USA, vol. 93, 1 juin 1996, pages 6025-6030, XP002911922 cité dans la demande
- PERRET E ET AL: "Improved differential screening approach to analyse transcriptional variations in organized cDNA libraries" GENE: AN INTERNATIONAL JOURNAL ON GENES AND GENOMES, vol. 208, no. 2, 22 février 1998, page 103-115 XP004114934
- PELICCI G ET AL: "A NOVEL TRANSFORMING PROTEIN (SHC) WITH AN SH2 DOMAIN IS IMPLICATEDIN MITOGENIC SIGNAL TRANSDUCTION" CELL, vol. 70, no. 1, 10 juillet 1992, pages 93-104, XP002001630 cité dans la demande
- HARUN R B ET AL.: "Characterization of human SHC p66 cDNA and its processed pseudogene mapping to Xq12-q13.1" GENOMICS, vol. 42, 1997, pages 349-352, XP002107843
- HUBANK ET AL: 'Identifying differences in mRNA expression by representatioal difference analysis of cDNA.' NUCLEIC ACIDS RESEARCH vol. 22, no. 25, 1994, pages 5640 - 5648, XP000672770

### LISTE DE SEQUENCES

<110> EXONHIT THERAPEUTICS SA
<120> CRIBLAGE DIFFERENTIEL QUALITATIF
<130> B3898B - PB/KM
<140>
   <141>
<150> 9802997
   <151> 1998-03-11
<160> 16
<170> PatentIn Ver. 2.1
<210> 1
   <211> 23
   <212> ADN
   <213> Séquence artificielle
<220>
   <223> Description de la séquence artificielle: OLIGO
<400> 1
   gagaagcgtt atnnnnnnna ggn 23
<210> 2
   <211> 24
   <212> ADN
   <213> Séquence artificielle
<220>
   <223> Description de la séquence artificielle: OLIGO
<400> 2
   gagaagcgtt atnnnnnnnn tccc 24
<210> 3
   <211> 23
   <212> ADN
   <213> Séquence artificielle
<220>
   <223> Description de la séquence artificielle: OLIGO
<400> 3
   gagaagcgtt atnnnnnnnn nnn 23
<210> 4
   <211> 20
   <212> ADN
   <213> Séquence artificielle
<220>
   <223> Description de la séquence artificielle: OLIGO
<400> 4
   gagaagcgtt atnnnnncca 20
<210> 5
   <211> 66
   <212> ADN
   <213> Homo sapiens
<400> 5
   ccacacctgg ccagtatgtg ctcactggct tgcagagtgg gcagccagcc taagcatttg 60
   cactgg 66
<210> 6
   <211> 23
   <212> ADN
   <213> Séquence artificielle
<220>
   <223> Description de la séquence artificielle: OLIGO
<400> 6
   gggacctgtt tgacatgaag ccc 23
<210> 7
   <211> 22
   <212> ADN
   <213> Séquence artificielle
<220>
   <223> Description de la séquence artificielle: OLIGO
<400> 7
   cagtttccgc tccacaggtt gc 22
<210> 8
   <211> 96
   <212> ADN
   <213> Homo sapiens
<400> 8
   gtacgggaga gcacgaccac acctggccag tatgtgctca ctggcttgca gagtgggcag 60
   cctaagcatt tgctactggt ggaccctgag ggtgtg                           96
<210> 9
   <211> 441
   <212> PRT
   <213> Homo sapiens
<400> 9
   Met Asn Lys Leu Ser Gly Gly Gly Gly Arg Arg Thr Arg Val Glu Gly
   1               5                   10                  15
   Gly Gln Leu Gly Gly Glu Glu Trp Thr Arg His Gly Ser Phe Val Asn
               20                  25                  30
   Lys Pro Thr Arg Gly Trp Leu His Pro Asn Asp Lys Val Met Gly Pro
           35                  40                  45
   Gly Val Ser Tyr Leu Val Arg Tyr Met Gly Cys Val Glu Val Leu Gln
       50                  55                  60
   Ser Met Arg Ala Leu Asp Phe Asn Thr Arg Thr Gln Val Thr Arg Glu
   65                  70                  75                  80
   Ala Ile Ser Leu Val Cys Glu Ala Val Pro Gly Ala Lys Gly Ala Thr
                   85                  90                  95
   Arg Arg Arg Lys Pro Cys Ser Arg Pro Leu Ser Ser Ile Leu Gly Arg
               100                 105                 110
   Ser Asn Leu Lys Phe Ala Gly Met Pro Ile Thr Leu Thr Val Ser Thr
           115                 120                 125
   Ser Ser Leu Asn Leu Met Ala Ala Asp Cys Lys Gln Ile Ile Ala Asn
       130                 135                 140
   His His Met Gln Ser Ile Ser Phe Ala Ser Gly Gly Asp Pro Asp Thr
   145                 150                 155                 160
   Ala Glu Tyr Val Ala Tyr Val Ala Lys Asp Pro Val Asn Gln Arg Ala
                   165                 170                 175
   Cys His Ile Leu Glu Cys Pro Glu Gly Leu Ala Gln Asp Val Ile Ser
               180                 185                 190
   Thr Ile Gly Gln Ala Phe Glu Leu Arg Phe Lys Gln Tyr Leu Arg Asn
           195                 200                 205
   Pro Pro Lys Leu Val Thr Pro His Asp Arg Met Ala Gly Phe Asp Gly
   210 215 220
   Ser Ala Trp Asp Glu Glu Glu Glu Glu Pro Pro Asp His Gln Tyr Tyr
   225 230 235 240
   Asn Asp Phe Pro Gly Lys Glu Pro Pro Leu Gly Gly Val Val Asp Met
   245 250 255
   Arg Leu Arg Glu Gly Ala Ala Pro Gly Ala Ala Arg Pro Thr Ala Pro
   260 265 270
   Asn Ala Gln Thr Pro Ser His Leu Gly Ala Thr Leu Pro Val Gly Gln
   275 280 285
   Pro Val Gly Gly Asp Pro Glu Val Arg Lys Gln Met Pro Pro Pro Pro
   290 295 300
   Pro Cys Pro Gly Arg Glu Leu Phe Asp Asp Pro Ser Tyr Val Asn Val
   305 310 315 320
   Gln Asn Leu Asp Lys Ala Arg Gln Ala Val Gly Gly Ala Gly Pro Pro
   325 330 335
   Asn Pro Ala Ile Asn Gly Ser Ala Pro Arg Asp Leu Phe Asp Met Lys
   340 345 350
   Pro Phe Glu Asp Ala Leu Arg Val Pro Pro Pro Pro Gln Ser Val Ser
   355 360 365
   Met Ala Glu Gln Leu Arg Gly Glu Pro Trp Phe His Gly Lys Leu Ser
   370 375 380
   Arg Arg Glu Ala Glu Ala Leu Leu Gln Leu Asn Gly Asp Phe Leu Val
   385 390 395 400
   Arg Thr Lys Asp His Arg Phe Glu Ser Val Ser His Leu Ile Ser Tyr
   405 410 415
   His Met Asp Asn His Leu Pro Ile Ile Ser Ala Gly Ser Glu Leu Cys
   420 425 430
   Leu Gln Gln Pro Val Glu Arg Lys Leu
   435 440
<210> 10
   <211> 1326
   <212> ADN
   <213> Homo sapiens
<400> 10
   atgaacaagc tgagtggagg cggcgggcgc aggactcggg tggaaggggg ccagcttggg 60
   ggcgaggagt ggacccgcca cgggagcttt gtcaataagc ccacgcgggg ctggctgcat 120
   cccaacgaca aagtcatggg acccggggtt tcctacttgg ttcggtacat gggttgtgtg 180
   gaggtcctcc agtcaatgcg tgccctggac ttcaacaccc ggactcaggt caccagggag 240
   gccatcagtc tggtgtgtga ggctgtgccg ggtgctaagg gggcgacaag gaggagaaag 300
   ccctgtagcc gcccgctcag ctctatcctg gggaggagta acctgaaatt tgctggaatg 360
   ccaatcactc tcaccgtctc caccagcagc ctcaacctca tggccgcaga ctgcaaacag 420
   atcatcgcca accaccacat gcaatctatc tcatttgcat ccggcgggga tccggacaca 480
   gccgagtatg tcgcctatgt tgccaaagac cctgtgaatc agagagcctg ccacattctg 540
   gagtgtcccg aagggcttgc ccaggatgtc atcagcacca ttggccaggc cttcgagttg 600
   cgcttcaaac aatacctcag gaacccaccc aaactggtca cccctcatga caggatggct 660
   ggctttgatg gctcagcatg ggatgaggag gaggaagagc cacctgacca tcagtactat 720
   aatgacttcc cggggaagga accccccttg gggggggtgg tagacatgag gcttcgggaa 780
   ggagccgctc caggggctgc tcgacccact gcacccaatg cccagacccc cagccacttg 840
   ggagctacat tgcctgtagg acagcctgtt gggggagatc cagaagtccg caaacagatg 900
   ccacctccac caccctgtcc aggcagagag ctttttgatg atccctccta tgtcaacgtc 960
   cagaacctag acaaggcccg gcaagcagtg ggtggtgctg ggccccccaa tcctgctatc 1020
   aatggcagtg caccccggga cctgtttgac atgaagccct tcgaagatgc tcttcgggtg 1080
   cctccacctc cccagtcggt gtccatggct gagcagctcc gaggggagcc ctggttccat 1140
   gggaagctga gccggcggga ggctgaggca ctgctgcagc tcaatgggga cttcttggtt 1200
   cggactaagg atcaccgctt tgaaagtgtc agtcacctta tcagctacca catggacaat 1260
   cacttgccca tcatctctgc gggcagcgaa ctgtgtctac agcaacctgt ggagcggaaa 1320
   ctgtga 1326
<210> 11
   <211> 19
   <212> ADN
   <213> Séquence artificielle
<220>
   <223> Description de la séquence artificielle: OLIGO
<400> 11
   tgcccaaatc aacaagagc 19
<210> 12
   <211> 19
   <212> ADN
   <213> Séquence artificielle
<220>
   <223> Description de la séquence artificielle: OLIGO
<400> 12
   cccctgacaa gcctgaata 19
<210> 13
   <211> 24
   <212> ADN
   <213> Séquence artificielle
<220>
   <223> Description de la séquence artificielle: OLIGO
<400> 13
   atgtctcaga gcaaccggga gctg 24
<210> 14
   <211> 24
   <212> ADN
   <213> Séquence artificielle
<220>
   <223> Description de la séquence artificielle: OLIGO
<400> 14
   gtggctccat tcaccgcggg gctg 24
<210> 15
   <211> 19
   <212> ADN
   <213> Séquence artificielle
<220>
   <223> Description de la séquence artificielle: OLIGO
<400> 15
   tgccaagaag ggaaggagt 19
<210> 16
   <211> 20
   <212> ADN
   <213> Séquence artificielle
<220>
   <223> Description de la séquence artificielle: OLIGO
<400> 16
   tgtcatgact ccagcaatag 20

## Description

Cette invention se rapporte aux domaines techniques de la biotechnologie, de la médecine, de la biologie et de la biochimie. Ses applications concernent les domaines de la santé humaine, animale et végétale. Plus particulièrement, l'invention permet d'identifier des séquences d'acides nucléiques permettant de concevoir de nouveaux cribles pour molécules d'intérêt thérapeutique, de nouveaux outils de thérapie génique ainsi que d'apporter des indications sur le potentiel toxique et le suivi d'efficacité de molécules et des informations de pharmacogénomique.

La présente invention décrit notamment une série de techniques originales d'identification de séquences d'acides nucléiques basée sur la mise en évidence des différences qualitatives entre les ARN issus de deux contextes différents que l'on désire comparer, en particulier issus d'un tissu ou d'un organe malade et leur équivalent sain. Plus précisément, ces techniques sont destinées à cloner spécifiquement les introns et les exons alternatifs épissés différentiellement entre une situation pathologique et un état sain ou entre deux situations physiologiques que l'on désire comparer. Ces différences qualitatives au sein des ARNs peuvent également provenir d'altération(s) du génome, de type insertions ou délétions dans des régions qui seront transcrites en ARN. Cette série de techniques est identifiée par l'acronyme DATAS : Differential Analysis of Transcripts with Alternative Splicing.

La caractérisation des altérations de l'expression génétique qui président ou sont associées à une pathologie donnée suscite un espoir important de découvrir de nouvelles cibles thérapeutiques et de nouveaux outils diagnostiques. Toutefois, l'identification d'une séquence d'ADN génomique ou complémentaire, qu'elie ait lieu par clonage positionnei ou par des techniques de criblage différentiel quantitatif, n'apporte que peu ou pas d'information sur la fonction et encore moins sur les domaines fonctionnels mis en jeu dans les dérégulations liées à la pathologie étudiée. La présente invention décrit une série de techniques originales qui visent à identifier les différences d'épissages des ARNs qui existent entre deux situations physiopathologiques distinctes. L'identification de ces différences apporte des informations sur les différences qualitatives et non sur les différences quantitatives comme c'est le cas pour les techniques décrites jusqu'à présent. L'ensemble des techniques présentées dans la présente invention sont donc regroupées sous l'appellation "criblage différentiel qualitatif' ou DATAS. Les méthodes de l'invention sont utilisables pour l'identification de nouvelles cibles ou produits thérapeutiques, pour la préparation d'outils de recherche génétique et/ou d'outils de diagnostic, pour la construction de banques d'acides nucléiques, et dans des méthodes de détermination du profil toxicologique ou de l'efficacité d'un composé par exemple.

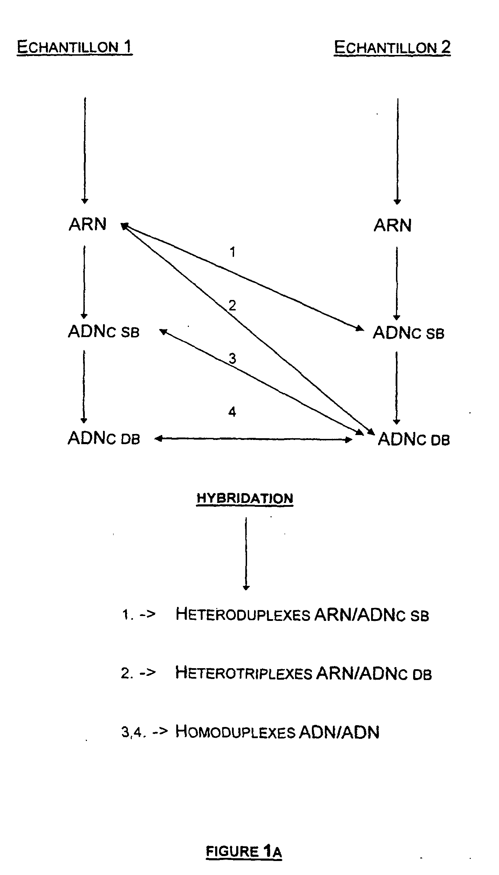

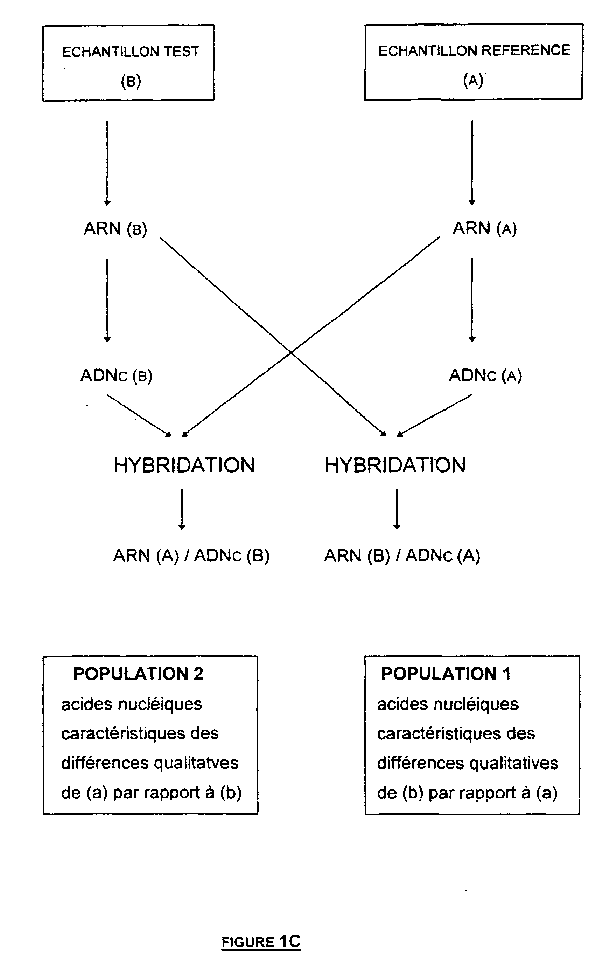

Un premier objet de l'invention réside plus particulièrement dans un procédé d'identification et/ou de clonage de régions d'acides nucléiques représentatives de différences génétiques qualitatives entre deux échantillons biologiques, comprenant une étape d'hybridation d'une population d'ARN ou d'ADNc double-brin provenant d'un premier échantillon biologique avec une population d'ADNc provenant d'un deuxième échantillon biologique (Figure 1A).

Comme indiqué ci-avant, les différences génétiques qualitatives peuvent être dues à des modifications d'épissage des ARN ou à des délétions et/ou insertions dans des régions du génome qui sont transcrites en ARN.

Dans un premier mode de réalisation, il s'agit d'une hybridation entre une population d'ARN provenant d'un premier échantillon biologique et une population d'ADNc (simple-brin ou double-brin) provenant d'un deuxième échantillon biologique.

Dans un autre mode de réalisation, il s'agit d'une hybridation entre une population d'ADNc double-brin provenant d'un premier échantillon biologique et une population d'ADNc (double-brin ou, préférentiellement simple-brin) provenant d'un deuxième échantillon biologique.

Un objet plus particulier de l'invention réside dans un procédé d'identification de régions d'acides nucléiques épissées différentiellement entre deux situations physiologiques, comprenant l'hybridation d'une population d'ARN ou d'ADNc double-brin provenant d'une situation test avec une population d'ADNc provenant d'une situation de référence et l'identification d'acides nucléiques correspondant à des épissages différentiels.

Un autre objet de l'invention concerne un procédé de clonage d'acides nucléiques épissés différentiellement entre deux situations physiologiques, comprenant l'hybridation d'une population d'ARN ou d'ADNc double-brin provenant de la situation test avec une population d'ADNc provenant de la situation de référence et le clonage d'acides nucléiques correspondant à des épissages différentiels.

Dans un mode particulier de mise en oeuvre, le procédé d'identification et/ou de clonage d'acides nucléiques de l'invention comprend deux hybridations parallèles :
(a) l'hybridation des ARN provenant du premier échantillon (situation test) avec les ADNc provenant du deuxième échantillon (situation de référence);
(b) l'hybridation des ARN provenant du deuxième échantillon (situation de référence) avec les ADNc provenant du premier échantillon (situation test); et
(c) l'identification et/ou le clonage, à partir des hybrides formés en (a) et (b) d'acides nucléiques correspondant à des différences génétiques qualitatives.

La présente invention concerne également la préparation de banques d'acides nucléiques, les acides nucléiques et banques ainsi obtenus, ainsi que les utilisations de ces matériels dans tous les domaines de la biologie/biotechnologie, comme illustré plus loin.

A cet égard, l'invention est également relative à un procédé de préparation de compositions ou banques d'acides nucléiques profilées, représentatives des différences qualitatives existant entre deux échantillons biologiques, comprenant une étape d'hybridation d'une population d'ARN provenant d'un premier échantillon biologique avec une population d'ADNc provenant d'un deuxième échantillon biologique.

L'invention concerne en outre une méthode de profilage ("profiling") d'une composition d'ADNc, comprenant une étape d'hybridation de cette composition avec une population d'ARN, ou inversement.

Comme indiqué ci avant, la présente invention concerne en particulier des méthodes d'identification et de clonage d'acides nucléiques représentatifs d'un état physiologique. En outre, les acides nucléiques identifiés et/ou clonés représentent les qualités d'un état physiologique en ce sens que ces acides nucléiques sont généralement en grande partie impliqués dans l'état physiologique observé. De ce fait, les méthodes qualitatives de l'invention donnent accès directement aux éléments génétiques ou à leur produit protéique, ayant un rôle fonctionnel dans le développement d'un état physiopathologique.

Les méthodes selon l'invention reposent en partie sur une étape originale d'hybridation croisée entre des ARN et des ADNc de situations physiologiques différentes. Cette ou ces hybridations croisées permettent avantageusement de mettre en évidence, dans les hybrides formés, des régions non appariées, c'est-à-dire des régions présentes dans les ARNs dans une situation physiologique donnée et pas dans les ARNs dans une autre situation physiologique. Ces régions correspondent essentiellement à des épissages alternatifs caractéristiques d'un état physiologique, mais peuvent également refléter des altérations génétiques de type insertions ou délétions, et constituent ainsi des éléments génétiques particulièrement utiles sur le plan thérapeutique ou diagnostic comme
expliqué ci-après. L'invention consiste donc notamment à conserver les complexes formés après hybridation(s) croisée(s), afin d'en extraire les régions correspondant à des différences qualitatives. Cette méthodologie se différencie des techniques de soustractions quantitatives connues de l'homme de l'art ( Sargent and Dawid (1983), Science, 222, 135-139 ; Davis et.al. (1984), PNAS, 81, 2194-2198 ; Duguid and Dinauer (1990) Nuc. Acid Res., 18, 2789-2792 ; Diatchenko et.al. (1996) PNAS, 93, 6025-6030 WO 98/42781 ; Hubank et al., Nucleic Acid Res. 22 (1994) Vol. 22, No 25, p. 5640-5648), qui après hybridation(s), éliminent les hybrides formés pour ne conserver que les acides nucléiques non complexés. De même, Perret et al. (Gene 208 (1998) p. 103-115), au contraire de l'invention, porte uniquement sur l'étude des niveaux d'expression de gènes dans un échantillon biologique, et n'enseigne nullement l'analyse de régions non appariées dans des hybrides formés entre deux échantillons. EP-A-0 791 660 porte sur la détection d'un site unique d'épissage et n'enseigne aucun procédé d'identification de régions qualitatives ni les méthodes et outils d'analyse selon l'invention.

L'invention concerne donc en premier lieu un procédé d'identification d'acides nucléiques d'intérêt comprenant l'hybridation entre les ARN d'un échantillon test et les ADNc d'un échantillon de référence. Cette hybridation permet de mettre en évidence, au sein des complexes formés, des différences génétiques qualitatives entre les situations testées, et ainsi d'identifier et/ou de cloner par exemple les épissages caractéristiques de la situation test.

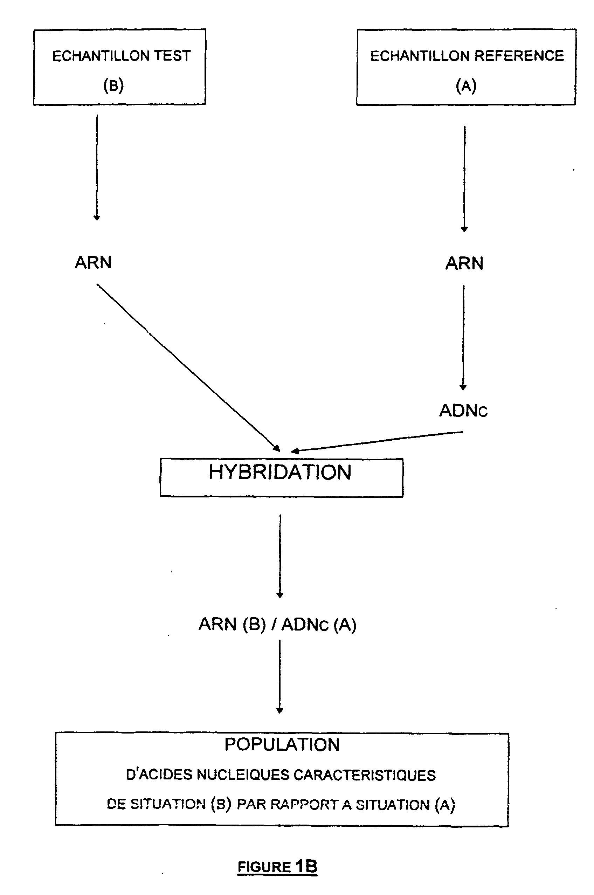

Selon une première variante de l'invention, le procédé permet donc de générer une population d'acides nucléiques caractéristiques des épissages de l'état physiologique test par rapport à l'état de référence (Figure 1A, 1B). Comme indiqué ci-après, cette population peut être utilisée pour le clonage et la caractérisation des acides nucléiques, leur utilisation en diagnostic, criblage, thérapeutique ou pour la production d'anticorps ou de fragments protéiques ou de protéines entières. Cette population peut également servir à la constitution de banques utilisables dans différents domaines d'applications illustrés plus loin et à la réalisation de sondes marquées (Figure 1D).

Selon une autre variante de l'invention, le procédé comprend une première hybridation telle que décrite ci avant et une seconde hybridation, en parallèle, entre les ARN provenant de la situation de référence et les ADNc provenant de la situation test. Cette variante est particulièrement avantageuse puisqu'elle permet de générer deux populations d'acides nucléiques, l'une représentant les qualités de la situation test par rapport à la situation de référence, et l'autre les qualités de la situation de référence par rapport à la situation test (Figure 1C). Ces deux populations peuvent également être utilisées comme source d'acides nucléiques, ainsi que comme banques témoignant de l'empreinte génétique d'une situation physiologique donnée, comme détaillé plus loin (Figure 1D).

La présente invention peut être appliquée à tous types d'échantillons biologiques. En particulier, l'échantillon biologique peut être toute cellule, organe, tissu, prélèvement, biopsie, etc., contenant des acides nucléiques. S'agissant d'un organe, tissu ou biopsie, ils sont éventuellement mis en culture de manière à permettre l'accès aux cellules qui les composent. Il peut s'agir d'échantillons provenant de mammifères (en particulier l'homme), de végétaux, de bactéries ou de cellules eucaryotes inférieures (levures, cellules fongiques, etc.). Des exemples de matériels sont en particulier une biopsie de tumeur, une biopsie de plaques neurodégénératives ou d'aires cérébrales présentant des atteintes neurodégénératives, un échantillon de peau, un échantillon de cellules sanguines obtenues après prise de sang, une biopsie colorectale, des biopsies issues de lavages pulmonaires, etc. Des exemples de cellules sont notamment les cellules musculaires, hépatiques, fibroblastes, nerveuses, de l'épiderme, du derme, des cellules sanguines comme les lymphocytes B, T, les mastocytes, les monocytes, les granulocytes, les macrophages.

Comme indiqué ci avant, le criblage différentiel qualitatif selon la présente invention permet d'identifier des acides nucléiques caractéristiques d'une situation physiologique donnée (situation B) par rapport à une situation physiologique de référence (situation A), en vue de leur clonage ou autres utilisations. A titre illustratif, les situations physiologiques A et B étudiées peuvent être les suivantes :

| SITUATION A | SITUATION B |
|---|---|
| échantillon sain | échantillon pathologique |
| échantillon sain | échantillon apoptotique |
| échantillon sain | échantillon après infection virale |
| échantillon sensible à X | échantillon résistant à X |
| échantillon non traité | échantillon traité (par exemple par composé toxique) |
| échantillon non différencié | échantillon ayant subi une différentiation cellulaire ou tissulaire |

### Populations d'ARN

Pour la mise en oeuvre de la présente invention, il est possible d'utiliser des ARN totaux ou les ARN messagers. Ces ARN peuvent être préparés par toutes méthodes classiques de biologie moléculaire, bien connues de l'homme du métier. Ces méthodes comprennent généralement une lyse des cellules ou tissu ou échantillons et l'isolement des ARNs par des techniques d'extraction. Il peut s'agir en particulier d'un traitement au moyen d'agents chaotropiques tels que le thiocyanate de guanidium (qui détruit les cellules et protège les ARN) suivi d'une extraction des ARN au moyen de solvants (phénol, chloroforme par exemple). De telles méthodes sont bien connues de l'homme du métier (voir Maniatis et al., Chomczynski et al., Anal. Biochem: 162 (1987) 156). Ces méthodes peuvent être aisément pratiquées en utilisant des kits disponibles dans le commerce tels que par exemple le kit US73750 (Amersham) ou le kit Rneasy (Quiagen) pour les ARN totaux. Il n'est pas nécessaire que les ARN utilisés soient parfaitement purs, et notamment il n'est pas gênant que des traces d'ADN génomique ou d'autres composants cellulaires (protéine, etc.) subsistent dans les préparations, dès lors qu'ils n'affectent pas significativement la stabilité des ARNs et que les modes de préparation entre les différents échantillons à comparer soient les mêmes. En outre, de manière facultative, il est possible d'utiliser non pas des préparations d'ARN totaux mais des préparations d'ARN messagers. Ceux-ci peuvent être isolés, soit directement à partir de l'échantillon biologique soit à partir des ARN totaux, au moyen de séquences polyT, selon les méthodes classiques. L'obtention d'ARN messagers peut à cet égard être réalisée au moyen de kits commerciaux tels que par exemple le kit US72700 (Amersham) ou le kit utilisant des billes oligo-(dT) (Dynal). Un mode avantageux de préparation d'ARN consiste à extraire les ARN cytosoliques puis les ARN polyA+ cytosoliques. Des kits permettant la préparation sélective d'ARN cytosoliques non contaminés avec des ARN prémessagers porteurs d'exons et d'introns non épissés sont disponibles dans le commerce. C'est le cas notamment des kits Rneasy commercialisés par Qiagen (exemple de référence : 74103). Les ARN peuvent également être obtenus directement à partir de banques ou autres échantillons préparés à l'avance et/ou accessibles dans des collections, conservés dans des conditions appropriées.

Généralement, les préparations d'ARN utilisées comprennent avantageusement au moins 0,1 µg d'ARN, de préférence au moins 0,5µg d'ARN. Les quantités peuvent varier selon les cellules et les méthodes utilisées, sans modifier la mise en oeuvre de la présente invention. Pour obtenir des quantités d'ARN suffisantes (de préférence 0,1 µg au moins), il est généralement recommandé d'utiliser un échantillon biologique comprenant au moins 10⁵ cellules. A cet égard, une biopsie classique comprend généralement entre 10⁵ et 10⁸ cellules, et une culture cellulaire sur boite de pétri classique (diamètre 6-10 cm) comporte de l'ordre de 10⁶ cellules, ce qui permet d'obtenir aisément des quantités d'ARN suffisantes.

Les préparations d'ARN peuvent être utilisées extemporanément ou être conservées, de préférence au froid, en solution ou congelées, pour des utilisations ultérieures.

### Populations d'ADNc

Les ADNc utilisés dans le cadre de la présente invention peuvent être obtenus par transcription inverse selon les techniques classiques de biologie moléculaire. On peut se référer notamment à Maniatis et al. La transcription inverse est généralement réalisée en utilisant une enzyme, transcriptase inverse ("reverse transcriptase") et une amorce.

A cet égard, de nombreuses transcriptases inverses ont été décrites dans la littérature et sont disponibles dans le commerce (Kit 1483188, Boehringer). On peut citer à titre d'exemples les transcriptases inverses les plus souvent utilisées comme celles du virus aviaire AMV (Avian Myeloblastosis Virus) et du virus de leucémie murine MMLV (Moloney Murine Leukemia Virus). Il convient également de citer certaines DNA polymérase thermostables douées d'activité transcriptase inverse telles celles isolées de Thermus flavus et de Thermus thermophilus HB-8 (commercialement disponibles; références Promega M1941 et M2101). Selon une variante avantageuse, on utilise pour la mise en oeuvre de la présente invention la transcriptase inverse d'AMV puisque cette enzyme, fonctionnant à 42°C (contrairement à celle de MMLV qui fonctionne à 37°C), déstabilise certaines structures secondaires des ARN qui pourraient bloquer l'élongation, et permet ainsi la transcription inverse d'ARN de longueur importante, et permet d'avoir des préparations d'ADNc représentant les ARN avec une grande fidélité et une grande efficacité.

Selon une autre variante avantageuse de l'invention, on utilise une transcriptase inverse dépourvue d'activité RNaseH. L'utilisation de ce type d'enzyme offre plusieurs avantages, et notamment celui d'augmenter le rendement de synthèse des ADNc et de prévenir toute dégradation des ARN, qui seront ensuite engagés dans des hétéroduplex avec les ADNc néosynthétisés, permettant ainsi éventuellement de se passer de l'extraction phénolique de ceux ci. Les transcriptases inverses dépourvues d'activité RNaseH peuvent être préparées à partir de toute transcriptase inverse par délétion(s) et/ou mutagénèse. En outre, de telles enzymes sont également disponibles dans le commerce (par exemple Life Technologies, référence 18053-017).

Les conditions de mise en oeuvre des transcriptases inverses (concentration et température) sont bien connues de l'homme du métier. En particulier, on utilise généralement de 10 à 30 Unités d'enzyme par réaction, en présence d'une concentration optimale en Mg²⁺ de 10 mM.

La ou les amorces utilisées pour la transcription inverse peuvent être de natures différentes. Il peut s'agir en particulier d'un oligonucléotide aléatoire ("random") comprenant de préférence entre 4 et 10 nucléotides, avantageusement un hexanucléotide. L'utilisation de ce type d'amorce aléatoire a été décrite dans la littérature et permet d'initier la transcription inverse en différentes positions au hasard au sein des molécules d'ARN. Cette technique est surtout employée pour la transcription inverse d'ARN totaux (c'est-à-dire comprenant les ARNm, les ARNt et les ARNr notamment). Dans le cas où l'on souhaite réaliser la transcription inverse des ARNm seulement, il est avantageux d'utiliser comme amorce un oligonucléotide oligo dT, qui permet d'initier la transcription inverse à partir des queues polyA spécifiques des ARN messagers. L'oligonucléotide oligo dT peut comprendre de 4 à 20-mères, avantageusement de 15-mères environ. L'emploi de ce type d'amorce constitue un mode de réalisation préféré de l'invention. D'autre part, il peut être avantageux d'utiliser pour la transcription inverse une amorce marquée. Ceci peut en effet permettre de reconnaître et/ou de sélectionner et/ou de trier ultérieurement les ARN des ADNc. Ceci peut également permettre d'isoler les hétéroduplex ARN/ADN dont la formation représente une étape clef de l'invention. Le marquage de l'amorce peut consister en tout système de type ligand-récepteur, c'est-à-dire permettant par affinité de séparer les molécules portant l'amorce. Il peut s'agir par exemple d'un marquage par la biotine, qui peut être séparé par tout support (bille, colonne, plaques, etc.) sur lequel est fixée la streptavidine. Tout autre système de marquage permettant cette séparation sans affecter les propriétés d'amorce peut être utilisé de manière équivalente.

Dans les conditions habituelles de mise en oeuvre, cette transcription inverse génère des ADN complémentaires (ADNc) simple-brins. Ceci constitue un premier mode avantageux de la présente invention.

Dans une deuxième variante de mise en oeuvre, la transcription inverse est réalisée de manière à préparer des ADNc double-brins. Pour ce faire, après transcription du premier brin d'ADNc, le deuxième brin peut être généré selon les techniques classiques de biologie moléculaire faisant intervenir des enzymes de modification de l'ADN comme l'ADN Ligase du phage T4, l'ADN polymérase I et l'ADN polymérase du phage T4.

Les préparations d'ADNc peuvent être utilisées extemporanément ou être conservées, de préférence au froid, en solution ou congelées, pour des utilisations ultérieures.

### Hybridations

Comme expliqué ci-avant, les méthodes selon l'invention reposent en partie sur une étape originale d'hybridation croisée entre les ARN et les ADNc provenant d'échantillons biologiques dans des situations physiologiques ou d'origines différentes. Dans un mode de réalisation préféré, l'hybridation selon l'invention est avantageusement réalisée en phase liquide. En outre, elle peut être effectuée dans tout dispositif approprié, tel que par exemple des tubes (Eppendorff, par exemple), des plaques, ou tout autre support adapté et couramment utilisé en Biologie Moléculaire. L'hybridation est avantageusement réalisée dans des volumes compris entre 10 et 1000 µl, par exemple entre 10 et 500 µl. Il est entendu que le dispositif utilisé et les volumes utilisés peuvent être aisément adaptés par l'homme du métier. Les quantités d'acides nucléiques utilisées pour l'hybridation sont également connues de l'homme du métier. En général, il est suffisant d'utiliser des microgrammes d'acides nucléiques, par exemple de l'ordre de 0,1 à 100 µg.

Un élément plus important dans la mise en oeuvre des hybridations réside dans les quantités respectives d'acides nucléiques utilisées. Ainsi, il est possible d'utiliser les acides nucléiques dans un rapport ADNc/ARN variant de 50 à 0,02 environ, de préférence de 40 à 0,1. De manière plus particulièrement avantageuse, on préfère que le rapport ADNc/ARN soit proche ou supérieur à 1. En effet, dans ces expériences, l'ARN constitue le composé test ("tester") et l'ADNc constitue le porteur ("driver"). De ce fait, dans le but d'améliorer la spécificité de la méthode, il est préférable d'opérer dans des conditions où le "driver" se trouve en excès par rapport au "tester". En effet, dans ces conditions, l'effet de coopérativité entre les acides nucléiques joue et les appariements non-parfaits sont fortement défavorisés. De ce fait, les seuls mésappariements qui apparaissent sont généralement dus à la présence de régions dans les ARN "tester" qui n'existent pas dans l'ADNc "driver" et qui sont donc spécifiques. Pour favoriser la spécificité du procédé, l'hybridation est donc avantageusement réalisée à un rapport ADNc/ARN compris entre 1 environ et 10 environ. Il est bien entendu que ce rapport peut être adapté par l'homme du métier selon les conditions du procédé (quantités d'acides nucléiques disponibles, situations physiologiques, but poursuivi, etc.). Les autres paramètres de l'hybridation (temps, température, force ionique) sont également adaptables par l'homme du métier. De manière générale après dénaturation des "tester" et "driver" (par chauffage par exemple), l'hybridation est réalisée pendant environ 2 à 24 heures, à une température de 37°C environ (éventuellement soumise à des sauts de température comme décrit plus loin), et dans des conditions standard de force ionique (pouvant varier de 0,1 à 5M NaCl par exemple). Il est connu que la force ionique est un des facteurs déterminant la stringence d'une hybridation, notamment dans le cas d'hybridation sur support solide.

Selon un mode de mise en oeuvre particulier de l'invention, l'hybridation est réalisée en émulsion phénolique, par exemple selon la technique PERT ("Phenol Emulsion DNA Reassociation Technique) décrite par Kohne D.E. et al. (Biochemistry, Vol. 16, N° 24, pp 5329-5341, 1977). Avantageusement, on utilise dans le cadre de la présente invention l'hybridation en émulsion phénolique maintenue par thermocycles (sauts de température de 37°C environ à 60/65°C environ) et non par agitation, selon la technique décrite par Miller et Riblet (NAR 23 (1995) 2339). Toute autre technique d'hybridation en phase liquide, notamment en émulsion, peut être utilisée dans le cadre de la présente invention. Ainsi, dans un autre mode particulièrement avantageux, l'hybridation est réalisée dans une solution contenant 80% de formamide, à une température de 40°c par exemple.

L'hybridation peut également se faire avec l'un des partenaires immobilisé sur un support. Avantageusement, c'est l'ADNc qui est immobilisé. Cela peut être réalisé en tirant profit du marquage dont peuvent faire l'objet les ADNc (voir ci-dessus) notamment grâce à des amorces biotinylées. Les groupements biotine sont mis en présence de billes magnétiques sur lesquelles sont fixées des molécules de streptavidine. Les ADNc peuvent ensuite être maintenus grâce à un aimant au contact d'un filtre ou d'un puits de plaque de microtitration. Les ARN sont ensuite, dans les conditions de force ionique requise, mis en présence des ADNc. Les ARN non appariés sont éliminés par lavage. Les ARN hybridés ainsi que les ADNc sont récupérés par retrait du champ magnétique.

Dans le cas où l'ADNc est double-brin, les conditions d'hybridation utilisées sont essentiellement similaires à celles décrites ci-dessus, et adaptables par l'homme du métier. On préfère dans ce cas procéder à l'hybridation en présence de formamide et on expose les complexes à une gamme de températures allant par exemple de 60 à 40 °C, préférentiellement de 56 °C à 44 °C, afin de favoriser la formation de complexes de type R-loop. De plus, il est souhaitable d'ajouter, après l'hybridation, un agent de stabilisation des triplex formés, une fois la formamide retirée du milieu, tels que le glyoxal par exemple (Kaback et.al. (1979) Nuc. Acid Res., 6, 2499-2517).

Ces hybridations croisées selon l'invention génèrent ainsi des compositions comprenant des hétéroduplex ou hétérotriplex ADNc/ARN, représentant les qualités de chacune des situations physiologiques testées. Comme indiqué ci-avant, dans chacune de ces compositions, des acides nucléiques correspondant essentiellement à des épissages alternatifs différentiels ou à d'autres altérations génétiques, spécifiques de chaque situation physiologique, peuvent être identifiés et/ou clonés.

L'invention concerne donc avantageusement un procédé d'identification et/ou de clonage de régions d'acides représentatives de différences génétiques entre deux situations physiologiques, comprenant une étape d'hybridation entre les ARN provenant d'un échantillon biologique dans une première situation physiologique et les ADNc simple-brins provenant d'un échantillon biologique dans une deuxième situation physiologique, et l'identification et/ou le clonage, à partir des hybrides ainsi formés, des régions d'ARN non-appariées.

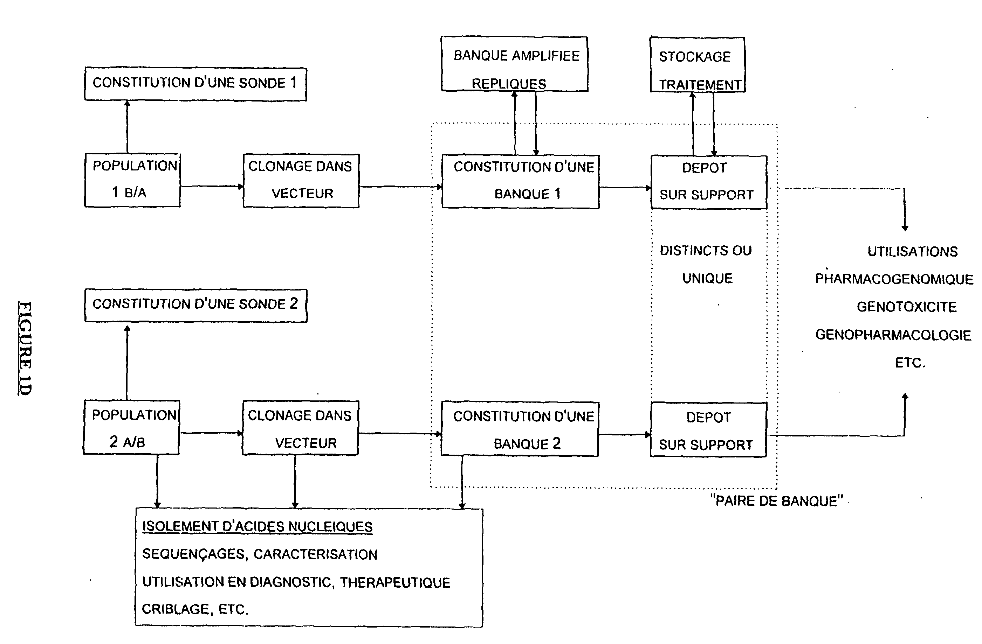

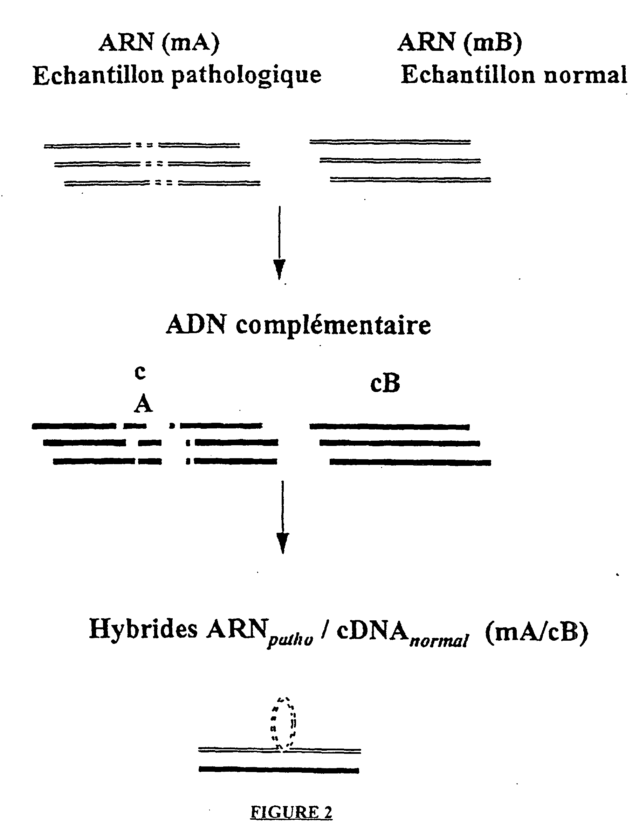

Cette première variante repose plus particulièrement sur la formation d'hétéroduplex entre les ARN et les ADNc simple-brin (voir Figures 2-4). Cette variante est avantageusement mise en oeuvre en utilisant des ARN messagers ou des ADNc produits par transcription inverse des ARN messagers essentiellement, c'est-à-dire en présence d'une amorce oligo dT.

Dans un mode particulier de mise en oeuvre, le procédé d'identification et/ou de clonage d'acides nucléiques de l'invention comprend :
(a) l'hybridation d'ARN provenant de la situation test avec les ADNc simple-brin provenant de la situation de référence;
(b) l'hybridation d'ARN provenant de la situation de référence avec les ADNc simple-brin provenant de la situation test; et
(c) l'identification et/ou le clonage, à partir des hybrides formés en (a) et (b), de régions d'ARN non-appariées.

Dans une variante particulière de mise en oeuvre, le procédé de l'invention comprend les étapes suivantes :
(a) l'obtention d'ARN à partir d'un échantillon biologique dans une situation physiologique A (rA);
(b) l'obtention d'ARN à partir d'un même échantillon biologique dans une situation physiologique B (rB);
(c) la préparation d'ADNc à partir d'une partie des ARN rA obtenus en (a) (ADN cA) et à partir d'une partie des ARN rB obtenus en (b) (ADN cB) au moyen d'amorces polyT,
(d) l'hybridation en phase liquide d'une partie des ARN rA avec une partie des ADN cB (pour générer des hétéroduplex rA/cB)
(e) l'hybridation en phase liquide d'une partie des ARN rB avec une partie des ADN cA (pour générer des hétéroduplex rB/cA),
(f) l'identification et/ou le clonage de régions d'ARN non appariées dans les hétéroduplex rA/cB et rB/cA obtenus en (d) et en (e).

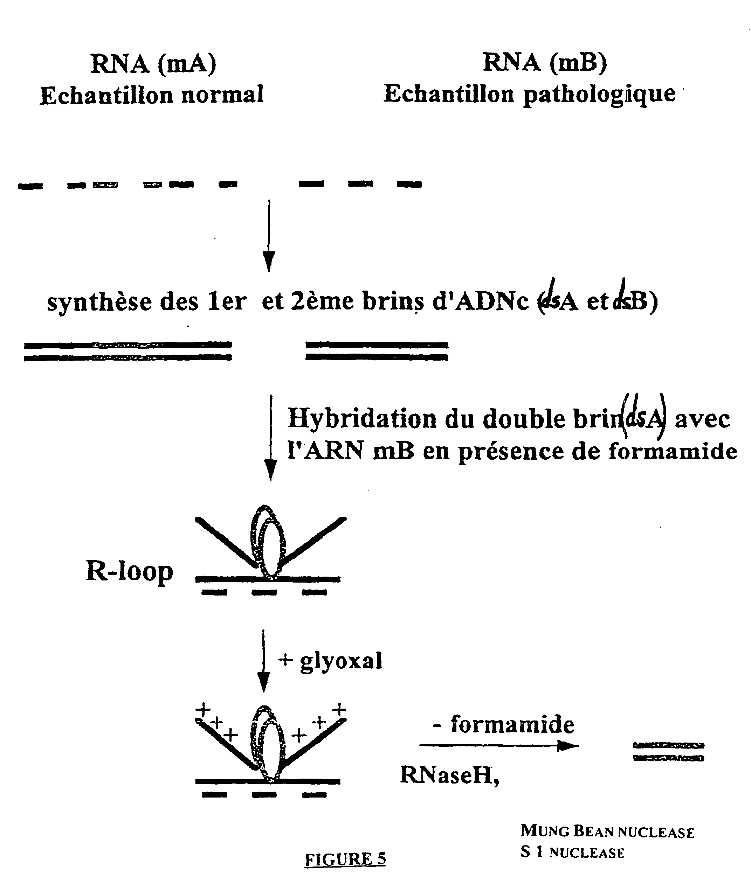

Dans une autre variante particulière de mise en oeuvre, le procédé de l'invention comprend l'hybridation d'ARN provenant de la situation test avec les ADNc double-brins provenant de la situation de référence, et l'identification et/ou le clonage des régions d'ADN double-brin maintenues. Cette deuxième variante repose plus particulièrement sur la formation d'hétérotriplex entre les ARN et les ADNc double-brin, dérivé des structures de type R-loop (voir Figure 5). Cette variante est également préférentiellement mise en oeuvre en utilisant des ARN messagers ou des ADNc produits par transcription inverse des ARN messagers essentiellement, c'est-à-dire en présence d'une amorce polyT. Dans cette variante également, un mode de réalisation particulier comprend deux hybridations parallèles, générant deux populations d'acides nucléiques selon l'invention. Dans cette variante, les régions recherchées, spécifiques des épissages alternatifs, ne sont pas les régions d'ARN non appariées, mais des ADN double-brins qui n'ont pu être déplacés par une séquence ARN homologue (voir Figure 5).

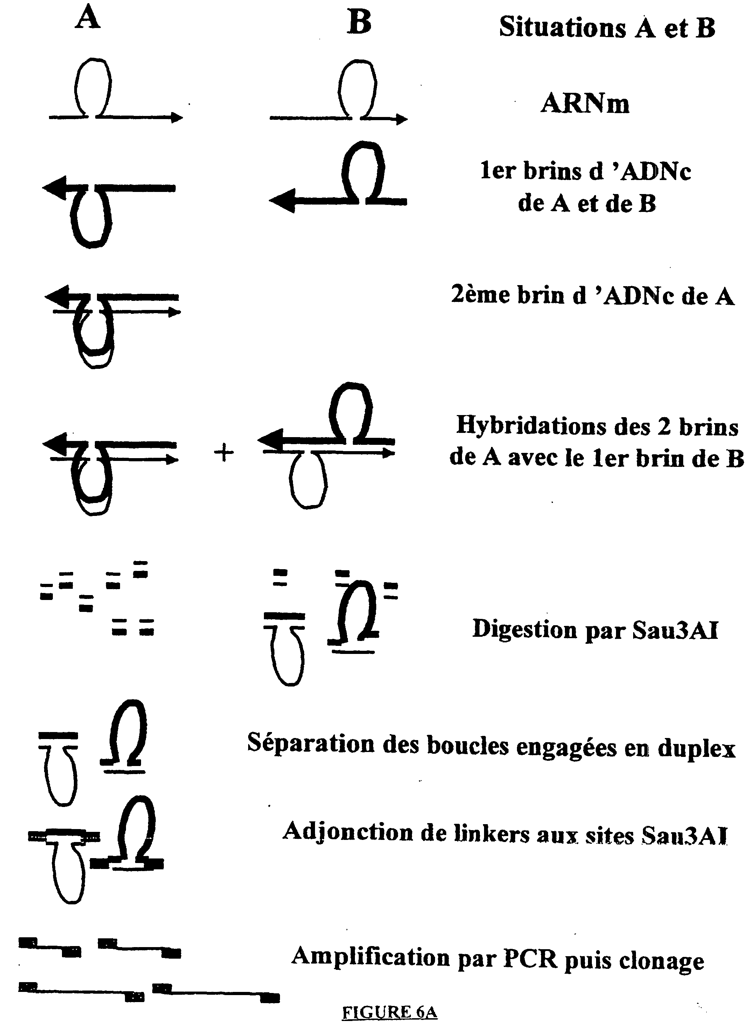

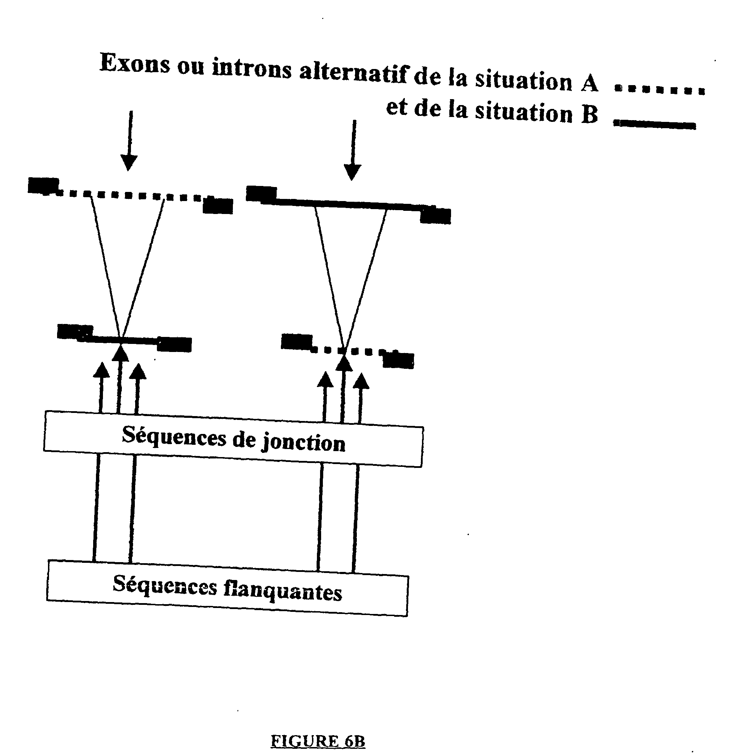

Dans une autre variante de l'invention, le procédé comprend, pour isoler les différences génétiques qualitatives (e.g., les différences d'épissage) qui existent entre deux échantillons, l'hybridation entre une population d'ADNc double-brin provenant d'un premier échantillon biologique et une population d'ADNc (double-brin ou, préférentiellement simple-brin) provenant d'un deuxième échantillon biologique (figure 6).

A la différence des variantes présentées précédemment, celle-ci n'utilise pas des hétéroduplexes ou des hétérotriplexes ADN/ARN mais des homoduplexes ADN/ADN. Cette variante est avantageuse puisqu'elle ne donne pas seulement accès aux exons et aux introns alternatifs mais également, et à l'intérieur d'une même banque d'acides nucléiques, aux jonctions spécifiques créées par délétion d'un exon ou d'un intron. De plus les séquences dans une telle banque donnent accès aux séquences flanquantes des exons et introns alternatifs.

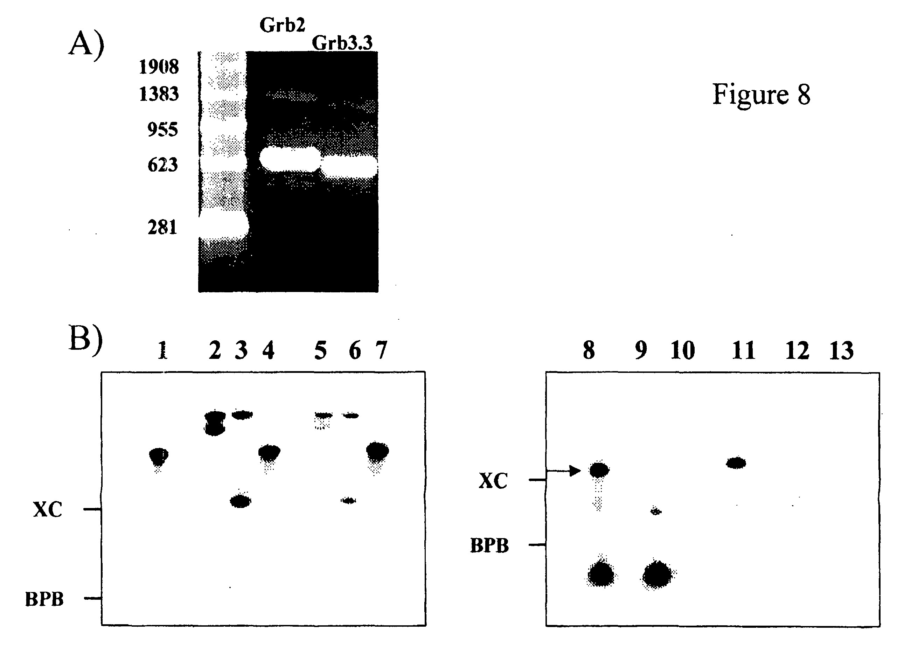

Pour les deux échantillons (i.e., conditions physiopathologiques) étudiés, les ARN cytosoliques polyA⁺ sont extraits selon les techniques connues de l'homme de métier et décrites précédemment. Ceux-ci sont convertis en ADNc par l'action d'une transcriptase inverse dépourvue ou non d'activité RNAse H intrinsèque, comme décrit précédemment. L'un de ces ADNc simple brin est ensuite converti en ADNc double-brin par amorçage à l'aide d'hexamères aléatoires et selon les techniques connues de l'homme de l'art. Pour l'une des situations étudiées nous disposons donc d'un ADNc simple-brin (appelé "driver") et pour l'autre situation d'un ADNc double-brin (appelé "tester"). Ces ADNc sont dénaturés par chauffage puis mélangés de telle façon que le driver est en excès par rapport au tester. Cet excès est choisi entre 1 et 50 fois, avantageusement 10 fois. Dans une expérience donnée, menée à partir de deux situations physiopathologiques, le choix de la situation qui donne le driver est arbitraire et ne doit pas influencer la nature des informations recueillies. En effet, comme dans le cas des approches précédemment présentées, la stratégie d'identification des différences qualitatives qui existent entre deux populations d'ARNm repose sur le clonage de ces différences présentes dans des messagers communs : la stratégie repose sur le clonage de séquences présentes au sein de duplex et non de simples brins correspondant à des séquences singulières ou en excès dans l'une des situations étudiées. Le mélange des populations d'ADNc est précipité puis repris dans une solution contenant du formamide (par exemple 80%). L'hybridation est menée de 16 heures à 48 heures, avantageusement 24 heures. Les produits de cette hybridation sont précipités puis soumis à l'action d'une endonucléase de restriction ayant un site de reconnaissance de l'ADN double-brin dicté par 4 bases. Une telle enzyme de restriction va donc cliver l'ADNc double brin formé lors de l'hybridation en moyenne toutes les 256 bases. Cette enzyme est sélectionnée avantageusement pour générer des sites cohésifs. Des exemples de telles enzymes sont fournis par des enzymes de restriction telles Sau3Al, Hpall, Taql et Msel. Sont donc accessibles à une stratégie de clonage utilisant les sites de restriction clivés les fragments double-brin digérés par ces enzymes. Ces fragments sont de deux types : des fragments parfaitement hybridés, dont les deux brins sont parfaitement complémentaires, et des fragments dont l'hybridation est partielle c'est à dire comprenant une boucle simple brin encadrée par des régions double-brin (Figure 6A). Ces derniers fragments, minoritaires, contiennent les informations d'intérêt. Afin de les séparer des fragments parfaitement hybridés, majoritaires puisque dérivés de la majorité de la longueur des ADNc, des techniques de séparation sur gel ou sur toute autre matrice appropriée sont utilisées. Ces techniques mettent à profit le retard de migration, électrophorétique ou lors de gel filtration notamment, des fragments d'ADN qui contiennent une boucle d'ADN simple-brin. Ainsi les populations de fragments minoritaires qui contiennent les informations désirées peuvent être séparées de façon préparative des populations de fragments majoritaires correspondant aux régions d'ADN identiques dans les deux populations. Cette variante, qui permet d'isoler au sein d'une même population les empreintes positives et négatives liées à des différences qualitatives, peut également être appliquée à des hétéroduplexes ARN/ADN. A cet égard, un exemple de retard de migration d'un hétéroduplexe ARN/ADN dans lequel une partie de l'ARN n'est pas appariée, par rapport à un hétéroduplexe homologue dans lequel toutes les séquences sont appariées est illustré sur le modèle grb2/grb33 décrit dans les exemples (voir notamment la figure 8, puits 2 et 3).

### Identification et/ou Clonage

A partir des populations d'acides nucléiques générées par hybridation, les régions caractéristiques des différences qualitatives (e.g., des épissages alternatifs différentiels) peuvent être identifiées par toute technique connue de l'homme du métier.

Identification et/ou clonage à partir des hétéroduplexes ARN/ADN

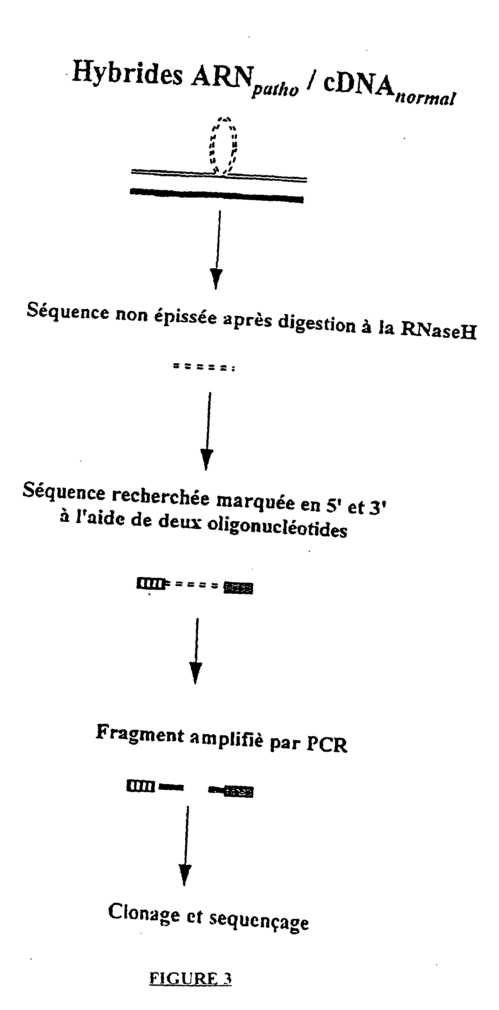

Ainsi, dans le cas des hétéroduplex ARN/ADN (première variante du procédé), ces régions se présentent essentiellement sous forme de régions d'ARN non-appariées (boucles d'ARN), comme représenté sur la Figure 3. Ces régions peuvent donc être identifiées et clonées par séparation des hétéroduplexes, et des acides nucléiques simple-brin (ADN, ARN) (excès d'acide nucléique n'ayant pas réagi), digestion sélective des ARN double-brins (domaines engagés dans les hétéroduplex), puis séparation des ARN simple-brin résultant et des ADN simple-brins.

A cet égard, selon une première approche illustrée sur la Figure 3, les régions d'ARN non appariées sont identifiées par traitement des hétéroduplex au moyen d'une enzyme capable de digérer sélectivement les domaines des ARN engagés dans des hétéroduplex ARN/ADN. Des enzymes douées de cette propriété sont décrites dans l'art antérieur et sont disponibles dans le commerce. Ce sont les RNases H, telles que en particulier, celle de E. Coli produite sous forme recombinante et disponible dans le commerce (Promega Réf. M4281 ; Life Technologies Réf. 18021). Ce premier traitement génère donc un mélange comprenant les régions d'ARN non appariées simple-brin et les ADNc simple-brin. Les ARNs peuvent être séparés des ADNc par toute technique connue de l'homme du métier, et notamment sur la base du marquage des amorces utilisées pour la préparation des ADNc (voir ci-dessus). Ces ARN peuvent être utilisés comme source de matériel pour l'identification de cibles, de produits génétiques d'intérêt ou toute autre application. Ces ARN peuvent également être convertis en ADNc, puis clonés dans des vecteurs, comme décrit ci-après.

A cet égard, le clonage des ARNs peut être réalisés de différentes façons. L'une consiste à insérer à chaque extrémité des ARN des oligonucléotides servant de matrice à une réaction de transcription inverse en présence des amorces correspondantes. Cet ajout d'amorces se fait selon les techniques bien connues de l'homme du métier grâce à une enzyme, telle que par exemple la ARN ligase qui provient du phage T4 et qui catalyse la formation de liaisons phosphodiester intermoléculaires entre le phosphate en 5' d'une molécule donneuse et l'hydroxyl en 3' d'une molécule acceptrice. Une telle ARN ligase est disponible commercialement (par exemple Life Technologies - GIBCO BRL Réf. 18003). Les ADNc ainsi obtenus peuvent ensuite être amplifiés par les techniques classiques (PCR par exemple) en utilisant les amorces appropriées, comme illustré sur la Figure 3. Cette technique est particulièrement adaptée au clonage des ARN de petite taille (inférieure à 1000 b).

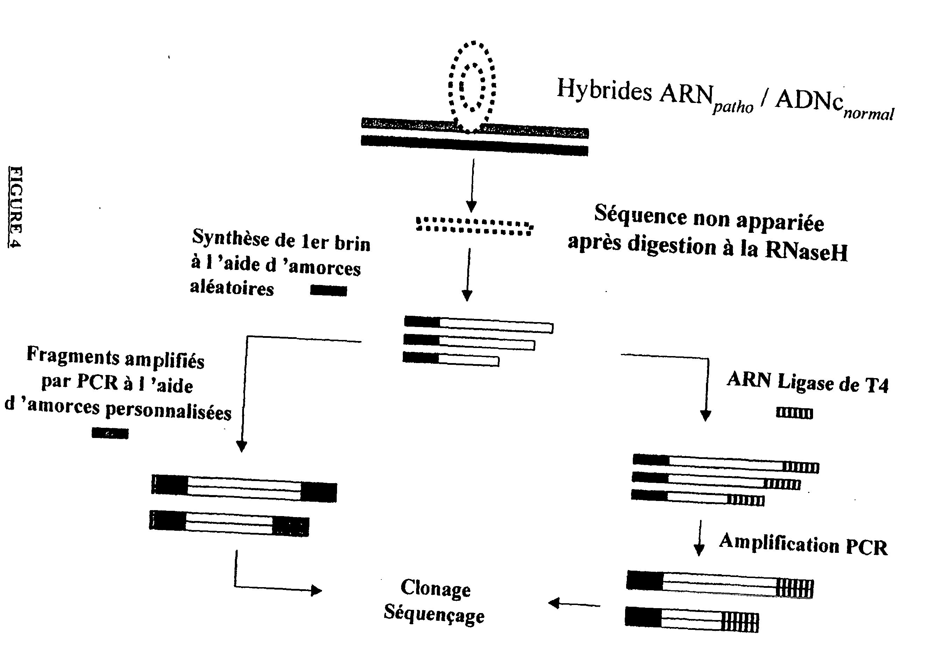

Une autre approche pour le clonage et/ou l'identification des régions d'ARN spécifiques consiste par exemple à réaliser une transcription inverse, sur le produit de digestion par une enzyme spécifique des ARN engagés dans des double-brins, telle la Rnase H, en utilisant des amorces aléatoires, qui vont initier la transcription au hasard à l'intérieur des ARNs. Les ADNc obtenus sont ensuite amplifiés selon les techniques classiques de biologie moléculaire, par exemple par PCR en utilisant des amorces grâce à des oligonucléotides ajoutés aux extrémités des ADNc grâce à l'action de l'ARN ligase du phage T4 (disponible commercialement ; par exemple chez Life Technologies - GIBCO BRL ref. 18003). Cette seconde technique est illustrée sur la Figure 4 et dans les exemples. Cette technique est plus particulièrement adaptée aux ARNs de taille importante, et permet d'obtenir une partie de l'information de séquence, suffisante pour reconstituer par la suite la totalité de la séquence de départ.

Une autre approche pour le clonage et /ou l'identification des régions d'ARN spécifiques repose également sur la réalisation d'une transcription inverse en utilisant des amorces aléatoires (figure 4). Néanmoins, selon cette variante, les amorces utilisées sont au moins en partie des amorces semi-aléatoires, c'est-à-dire des oligonucléotides comprenant:
- une région aléatoire (de dégénérescence),
- une zone minimale d'amorçage présentant un degré de contrainte défini, et
- une zone stabilisatrice.

De préférence, il s'agit d'oligonucléotides comprenant, dans l'orientation 5' ->3' :
- une zone stabilisatrice comprenant 8 à 24 nucléotides déterminés, de préférence de 10 à 18 nucléotides. Cette zone stabilisatrice peut elle-même correspondre à la séquence d'un oligonucléotide utilisé pour réamplifier les fragments issus des premières amplifications réalisées à l'aide des amorces semialéatoires de l'invention. En outre, la zone stabilisatrice peut comprendre la séquence d'un ou plusieurs sites, de préférence non-palindromiques, correspondant à des enzymes de restriction. Ceci permet par exemple de faciliter le clonage des fragments ainsi amplifiés. Un exemple particulier de zone stabilisatrice est représenté par la séquence GAG AAG CGT TAT (résidus 1 à 12 de SEQ ID NO:1);
- une région aléatoire ayant de 3 à 8 nucléotides, plus particulièrement de 5 à 7 nucléotides, et
- une zone minimale d'amorçage définie de sorte que l'oligonucléotide s'hybride en moyenne au moins toutes les 60 pb environ, de préférence toutes les 250 pb environ. Plus préférentiellement, la zone d'amorçage comporte de 2 à 4 nucléotides définis, préférentiellement 3 ou 4, tels que par exemple AGGX, où X représente l'une des quatre bases A, C, G ou T. La présence d'une telle zone d'amorçage confère à l'oligonucléotide la capacité d'hybrider en moyenne toutes les 256 paires de bases environ.

De manière particulièrement préférée, il s'agit d'oligonucléotides de formule :
GAGAAGCGTTATNNNNNNNAGGX (SEQ ID NO: 1) où les bases fixées ont été ordonnées de façon à minimiser le bruit de fond dû à des auto-appariements dans des expériences de PCR, où N indique que les quatre bases peuvent être présentes de façon aléatoire à la position indiquée, et où X représente l'une des bases A, C, G ou T. De tels oligonucléotides constituent également un objet de la présente invention.

A cet égard, de façon à augmenter les possibilités d'amorçage sur les ARN à cloner, des réactions en parallèle peuvent être effectuées avec des oligonucléotides tels que : chaque population d'oligonucléotides (A, B, C, D) pouvant être utilisée individuellement ou en combinaison avec une autre.

Après l'étape de transcription inverse, les ADNc sont amplifiés par PCR en utilisant les oligonucléotides A ou B ou C ou D.

Comme indiqué ci-avant, selon la complexité et la spécificité de la population des oligonucléotides souhaitée le nombre de positions dégénérées peut varier de 3 à 8, de préférence de 5 à 7. En deçà de 3 les hybridations sont restreintes et au delà de 8 la population d'oligonucléotides est trop complexe pour assurer une bonne amplification de bandes spécifiques.

Par ailleurs, la longueur de l'extrémité 3' fixe (la zone d'amorçage contrainte) de ces oligonucléotides peut également être modifiée : si les amorces décrites plus haut, avec 4 bases fixées, permettent d'amplifier en moyenne des fragments de 256 paires de bases, des amorces avec 3 bases fixes permettent d'amplifier des fragments plus courts (64 paires de bases en moyenne). Dans un premier mode préféré de l'invention, on utilise des oligonucléotides dans lesquels la zone d'amorçage comprend 4 bases fixes. Dans un autre mode préféré de l'invention, on utilise des oligonucléotides ayant une zone d'amorçage de trois bases fixes. En effet, les exons ayant une taille moyenne de 137 bases, ceux-ci sont avantageusement amplifiés avec de tels oligonucléotides. A cet égard, voir également les oligonucléotides de séquence SEQ ID NO: 2, 3 et 4, par exemple.

Enfin, généralement, l'étape d'identification et/ou de clonage des ARN met en oeuvre les différentes méthodes de PCR et de clonage, de manière à obtenir l'information la plus complète.

### . Identification et/ou clonage à partir des hétérotriplexes.

Dans le cas des hétérotriplexes (autre variante du procédé), les régions de différences qualitatives (insertions, délétions, épissages différentiels) se présentent essentiellement sous forme de régions d'ADN double-brin, comme représenté sur la Figure 5. Ces régions peuvent donc être identifiées et clonées par traitement en présence d'enzymes appropriées telles qu'une enzyme permettant de digérer les ARN, puis une enzyme permettant de digérer les ADN simple-brin. Les acides nucléiques ainsi obtenus sont donc directement sous forme d'ADN double-brin et peuvent être clonés dans tout vecteur approprié, tel le vecteur pMos-Blue (Amersham, RPN 5110), par exemple. Cette méthodologie est à différencier des approches déjà décrites utilisant des ARNs ou oligonucleotides de séquences prédéterminées, modifiés pour exercer une activité nucléasique (Landgraf et al. (1994) Biochemistry, 33, 10607-10615).

### . Identification et/ou clonage à partir des homoduplexes ADN/ADN (figure 6).

Les fragments isolés par leurs structures atypiques sont ensuite additionnés, à chacune de leurs extrémités, d'adaptateurs, ou linkers, ayant des sites de restriction clivés à l'une de leurs extrémités. Cette étape peut être réalisée selon les techniques connues de l'homme du métier, par exemple par ligation avec l'ADN ligase du phage T4. Les sites de restriction ainsi introduits sont choisis compatibles avec les sites des fragments d'ADNc. Les linkers introduits sont des séquences d'ADNc double-brin, de séquences connues, permettant de dériver des amorces pour réaliser des amplifications enzymatiques (PCR). Puisque l'étape suivante consiste à amplifier les deux brins qui présentent entre eux les différences qualitatives à identifier, il est nécessaire d'utiliser des linkers dont les extrémités 5' sont phosphorylées. Ainsi après dénaturation thermique des ADNc double-brin additionnés de linkers, chacune des extrémités de ces ADNc est liée de façon covalente avec une séquence d'amorçage spécifique. Après PCR à l'aide des amorces spécifiques appropriées, deux catégories d'ADNc double-brin sont obtenues: des fragments qui contiennent des séquences spécifiques de différences qualitatives qui distinguent les deux situations physiopathologiques, et des fragments qui comprennent l'empreinte négative de ces événements d'épissages. Le clonage de ces fragments permet d'obtenir une banque d'épissages alternatifs dans laquelle, pour chaque événement d'épissage, des empreintes positives et négatives sont présentes. Dans cette banque, sont donc accessibles non seulement les exons et les introns alternatifs mais aussi les jonctions spécifiques créées par excision de ces séquences épissées. Dans une même banque, ces différentes informations génétiques peuvent provenir des deux situations physiopathologiques sans discrimination. Par ailleurs, de façon à vérifier le caractère différentiel des épissages identifiés et de façon à déterminer dans quelle situation ceux-ci sont spécifiquement recrutés, les clones de la banque peuvent être hybridés avec des sondes dérivées de chacune des populations totales des ARNm.

Deux usages principaux peuvent être envisagés pour les fragments d'ADNc issus des différences qualitatives identifiées :
- Leur clonage dans des vecteurs appropriés de façon à constituer des banques représentatives des différences qualitatives qui existent entre les deux situations physiopathologiques étudiées,
- Leur utilisation en tant que sondes afin de cribler une banque d'ADN permettant d'identifier les événements épissés de façon différentielle.

Les vecteurs utilisés dans l'invention peuvent être notamment des plasmides, cosmides, phages, YAC, HAC, etc. Ces acides nucléiques peuvent ainsi être conservés tels quels ou introduits dans des microorganismes adaptés au vecteur de clonage utilisé, afin d'être multipliés et/ou conservés sous forme de cultures.

Les méthodes telles que décrites ci-dessus sont généralement mises en oeuvre, pour chaque échantillon, sur une période de temps de moins de deux mois, en particulier moins de 6 semaines. Par ailleurs, ces différentes méthodes peuvent être automatisées afin de réduire la durée totale et de faciliter le traitement de nombreux échantillons.

A cet égard, un autre objet de l'invention concerne les acides nucléiques identifiés et/ou clonés par les méthodes de l'invention. Comme indiqué ci-dessus, ces acides nucléiques peuvent être des ARN ou des ADNc. Plus généralement, l'invention concerne une composition d'acides nucléiques, comprenant essentiellement des acides nucléiques correspondant aux épissages alternatifs distinguant deux situations physiologiques. Plus particulièrement, ces acides nucléiques correspondent aux épissages alternatifs identifiés dans un échantillon biologique test et non présents dans le même échantillon biologique dans une situation de référence. L'invention a également pour objet l'utilisation des acides nucléiques ainsi clonés comme produit thérapeutique ou diagnostic, ou comme outil de criblage de molécules actives, comme indiqué ci-après.

Les différentes méthodes exposées ci-dessus aboutissent donc toutes au clonage de séquences d'ADNc qui représentent l'information génétique épissée différentiellement entre deux situations physiopathologiques. L'ensemble des clones issus de l'une de ces méthodes permet donc la constitution d'une banque représentative des différences qualitatives qui existent entre deux situations étudiées.

### Génération de banques qualitatives

A cet égard, l'invention concerne en outre un procédé de préparation d'une banque d'acides nucléiques représentatifs d'un état physiologique donné d'un échantillon biologique. Ce procédé comprend avantageusement le clonage d'acides nucléiques représentatifs des marqueurs qualitatifs d'expression génétiques (par exemple des épissages alternatifs) dudit état physiologique et non présents dans un état de référence, dans des banques spécifiques de différences qualitatives qui existent entre les 2 états étudiés.

Ces banques sont constituées d'ADNc insérés dans des vecteurs plasmidiques ou phagiques. Ces banques peuvent être présentées sur des filtres de nitrocellulose ou tout autre support connu de l'homme de l'art, tels des chips ou biopuces.

L'une des caractéristiques et en même temps l'une des originalités du criblage différentiel qualitatif est que cette technique aboutit à non pas une mais avantageusement deux banques différentielles qui représentent l'ensemble des différences qualitatives qui existent entre deux situations données : Paire de banque (voir figure 1D).

Ainsi, l'invention concerne de préférence toute composition ou banque d'acides nucléiques, susceptible d'être obtenue par hybridation entre une population d'ARN provenant d'un premier échantillon biologique et une population d'ADNc provenant d'un deuxième échantillon biologique. Plus préférentiellement, les banques ou compositions de l'invention comprennent des acides nucléiques représentatifs des différences qualitatives d'expression entre deux échantillons biologiques, et sont produites par un procédé comprenant (i) une étape au moins d'hybridation entre une population d'ARN provenant d'un premier échantillon biologique et une population d'ADNc provenant d'un deuxième échantillon biologique, (ii) la sélection des acides nucléiques représentatifs des différences qualitatives d'expression et, éventuellement (iii) le clonage desdits acides nucléiques.

En outre, après constitution de telles banques, il est possible d'effectuer une étape de sélection des clones pour améliorer la spécificité des banques obtenues. En effet, il est possible que certains mésappariements observés ne soient pas uniquement dus à différences qualitatives (e.g. à des épissages alternatifs différentiels), mais puissent résulter de défaut(s) de la transcription inverse par exemple. Bien que ces événements ne soient pas généralement significatifs, il est préférable de les éliminer ou de les réduire préalablement au clonage des acides nucléiques. Pour ce faire, les clones de la banque peuvent être hybridés avec les populations d'ADNc des deux situations physiologiques étudiées (voir étape (c) ci-dessus). Les clones hybridant de façon non différentielle avec les deux populations peuvent être considérés comme non-spécifiques et éventuellement éliminés ou traités en deuxième priorité (en effet, l'apparition d'une nouvelle isoforme dans l'échantillon test ne signifie pas toujours que l'isoforme initiale présente dans l'échantillon de référence a disparu de cet échantillon test). Les clones n'hybridant qu'avec une seule des deux populations ou hybridant de façon préférentielle avec l'une des populations sont considérés comme spécifiques et peuvent être sélectionnés en première priorité pour constituer des banques enrichies ou affinées.

Un affinage peut également être réalisé par hybridation et validation de clones avec des sondes provenant d'un nombre statistiquement relevant d'échantillons pathologiques.

La présente demande décrit également toute banque d'acides nucléiques comprenant des acides nucléiques spécifiques d'épissages alternatifs caractéristiques d'une situation physiologique. Ces banques sont avantageusement constituées d'ADNc, généralement double brin, correspondant aux régions d'ARN spécifiques d'un épissage alternatif. Ces banques peuvent être constituées des acides nucléiques, généralement dans un vecteur de clonage ou de cultures cellulaires contenant lesdits acides nucléiques.

Le choix des ARN de départ détermine en partie les caractéristiques des banques obtenues :
- les ARN des deux situations A et B sont des ARNm ou des ARN totaux matures isolés selon les techniques connues de l'homme de l'art. Les banques sont alors des banques de criblage différentiel qualitatif dites restreintes, car restreintes aux différences qualitatives qui caractérisent les ARN matures des deux situations physiopathologiques.
- Les ARN de l'une des situations sont des ARNm ou totaux matures alors que les ARN de l'autre situation sont des ARN prémessagers, non maturés par épissage, isolés selon les techniques connues de l'homme de l'art, à partir de noyaux cellulaires. Dans ce cas les banques obtenues sont des banques de criblage différentiel dites complexes, puisque non restreintes aux différences entre ARN matures mais comprenant tout le répertoire des épissages transcrits dans une situation et éliminés dans l'autre, dont tous les introns.
enfin, les ARN peuvent provenir d'une seule situation physiopathologique et dans ce cas le criblage différentiel implique les ARN matures et les prémessagers d'un même échantillon. Dans ce cas, les banques obtenues sont des banques de criblage différentiel qualitatif autologues. L'intérêt de telles banques est qu'elles rassemblent exclusivement le répertoire des introns transcrits dans une situation donnée. Leur hybridation avec une sonde provenant d'ARN matures d'une autre situation détermine rapidement si à cette situation est caractérisée par une rétention d'introns tout en permettant aisément leur identification.

Généralement, les banques sont générées par étalement, sur milieu solide (notamment sur milieu gélosé), d'une culture cellulaire transformée par les acides nucléiques clonés. La transformation est réalisée par toute technique connue de l'homme du métier (transfection, phosphate de calcium, électroporation, infection par des bactériophages, etc). La culture cellulaire est généralement une culture de bactéries, telles que par exemple les bactéries E. coli. Il peut également s'agir de cultures de cellules eucaryotes, notamment de cellules eucaryotes inférieures (levures par exemple). Cet étalement peut être réalisé sur boite ou sur tout autre support adapté, en conditions stériles. En outre, ces cultures étalées en milieu gélosé peuvent être stockées sous forme congelée par exemple (dans du glycérol ou autre agent adapté). Ces banques peuvent naturellement être utilisées pour la production de "répliques", c'est-à-dire de copies selon les techniques habituelles détaillées ci-après. En outre, ces banques servent généralement à préparer une banque amplifiée, c'est-à-dire une banque comprenant chaque clone sous forme amplifiée. Une banque amplifiée est préparée comme suit : à partir de la culture étalée, tous les clones cellulaires sont récupérés et sont conditionnés pour être conservés sous forme congelée ou au froid, dans tout milieu adapté. Cette banque amplifiée est avantageusement réalisée à partir de cultures de bactéries E.coli, et conservée à 4°C, en conditions stériles. Cette banque amplifiée permet la préparation et la reproduction illimitée de toute banque ultérieure contenant ces clones, sur différents supports, pour différentes applications. Une telle banque permet en outre l'isolement et la caractérisation de tout clone d'intérêt. Chacun des clones constituant les banques de l'invention est en effet un élément caractéristique d'une situation physiologique, et constitue donc une cible particulièrement intéressante pour différentes études telles que la recherche de marqueurs, la préparation d'anticorps, le diagnostic, le traitement pour transfert de gènes, etc. Ces différentes applications sont discutées plus en détail plus loin. La banque est généralement préparée comme décrit ci-dessus par étalement des cultures dans un milieu gélosé, sur un support adapté (boite de pétri par exemple). L'intérêt d'utiliser un milieu gélosé est que chaque colonie peut être séparée et individualisée. A partir de cette culture, des répliques à l'identique peuvent être préparées en quantités importantes par simple "réplique" sur tout support approprié selon les techniques de l'homme de l'art. Ainsi, la réplique peut être réalisée au moyen de filtres, membranes (nylon, nitrocellulose, etc) permettant l'accrochage des cultures. Les filtres peuvent ensuite être stockés en l'état, à 4°C par exemple, sous forme desséchée, dans tout type de conditionnement qui n'altère pas les acides nucléiques. Les filtres peuvent également être traités de manière à éliminer les cellules, protéines, etc, et à ne conserver que des composants tels que les acides nucléiques. Ces traitement peuvent comprendre notamment des protéases, des détergents, etc. Les filtres traités peuvent également être conservés dans tout dispositif ou toute condition adaptés aux acides nucléiques.

Les banques d'acides nucléiques peuvent également être préparées directement à partir des acides nucléiques, par dépôt sur des biopuces ou tout autre dispositif approprié.

La présente demande décrit également toute banque comprenant des oligonucléotides spécifiques d'épissages alternatifs distinguant deux situations physiologiques. Il s'agit avantageusement d'oligonucléotides simple-brin, comprenant de 5 à 100-mères, de préférence moins de 50-mères, par exemple autour de 25-mères environ.

Ces oligonucléotides sont spécifiques d'épissages alternatifs représentatifs d'une situation ou d'un type de situation physiologique. Ainsi, de tels oligonucléotides peuvent être par exemple des oligonucléotides représentatifs d'événements d'épissages alternatifs caractéristiques de situations d'apoptose. Il a en effet été décrit dans la littérature que certains épissages alternatifs étaient observés dans le cadre de situations apoptotiques. Il s'agit par exemple d'épissages dans les gènes Bclx, Bax, Fas ou Grb2 notamment. A partir des données publiées et des séquences accessibles dans la littérature et/ou sur bases de données, il est possible de créer des oligonucléotides spécifiques des formes épissées et non épissées. Ces oligonucléotides peuvent par exemple être crées selon la stratégie suivante :
(a) identification d'une protéine ou d'un événement d'épissage caractéristique d'une situation d'apoptose et de la séquence du domaine épissé. Cette identification peut être basée sur des données publiées ou par compilation de séquences accessibles sur bases de données;
(b) synthèse artificielle d'un ou plusieurs oligonucléotides correspondant à une ou plusieurs régions de ce domaine, qui permettent donc par hybridation de mettre en évidence la forme non épissée dans les ARN d'un échantillon test;
(c) synthèse artificielle d'un ou plusieurs oligonucléotides correspondant à la région de jonction entre les deux domaines séparés par le domaine épissé. Ces oligonucléotides permettent donc par hybridation de mettre en évidence la forme épissée dans les ARN d'un échantillon test;
(d) reproduction des étapes (a) à (c) ci-dessus avec d'autres protéines ou événements d'épissages caractéristiques d'une situation d'apoptose;
(e) transfert sur un premier support approprié du ou des oligonucléotides spécifiques des formes apoptotiques des messagers identifiés ci-avant et, sur un autre support approprié, du ou des oligonucléotides spécifiques des formes non-apoptotiques.

Les deux supports ainsi obtenus peuvent être utilisés pour tester l'état physiologique de cellules ou échantillons tests, et notamment leur état apoptotique, par hybridation d'une préparation d'acides nucléiques de ces cellules ou échantillons.

D'autres banques similaires peuvent être générées avec des oligonucléotides spécifiques d'états physiopathologiques différents (neurodégénérescence, toxicité, prolifération, etc.) et ainsi permettre un élargissement des domaines d'applications.

Des banques d'introns ou d'exons alternatifs peuvent aussi être des banques de données informatiques constituées par analyse systématique des banques de données qui regroupent les informations relatives au génome de tel ou tel organisme, tissu ou culture cellulaire. Dans ce cas, les données obtenues par constitution de telles banques virtuelles peuvent être utilisées pour générer des amorces oligonucléotidiques qui seront utilisées pour tester en parallèle deux situations physiopathologiques.

Les données des banques informatiques peuvent également être utilisées pour dériver des sondes nucléotidiques générales, représentatives d'une classe de protéines ou encore spécifiques d'une séquence définie. Ces sondes peuvent ensuite être appliquées sur les banques de clones issues des différentes techniques de clonage des introns et exons alternatifs afin d'obtenir une image de la complexité de ces banques moléculaires et de déterminer rapidement si telle ou telle classe de protéines ou telle ou telle séquence déterminée est épissée différentiellement entre deux états physiopathologiques distincts.

Une autre banque ou compositions d'acides nucléiques selon l'invention est une banque antisens, réalisée à partir des séquences identifiées selon les méthodes de l'invention (DATAS). Pour la réalisation de ce type de banques, ces séquences sont clonées de façon à être exprimées en fragments d'ARN correspondant à une orientation antisens par rapport aux ARN messagers sur lesquels ont été réalisés DATAS. On aboutit ainsi à une banque dite antisens. Cette approche utilise de préférence la variante de clonage qui permet une orientation des fragments clonés. L'intérêt d'une telle banque antisens est de permettre la transfection de lignées cellulaires et de suivre l'altération de tout phénotype qu'il soit d'ordre morphologique, enzymatique ou suivi par l'utilisation de gènes rapporteurs ou de résistance à un agent de sélection. L'analyse des variations phénotypiques consécutives à l'introduction d'un vecteur d'expression antisens se fait généralement après sélection de clones dits stables, c'est à dire permettant une réplication coordonnée du vecteur d'expression et du génome de l'hôte. Cette coordination est permise par l'intégration du vecteur d'expression dans le génome cellulaire ou, lorsque le vecteur d'expression est épisomal, par pression de sélection. Cette pression de sélection se fait par traitement de la culture cellulaire transfectée avec un agent toxique qui ne peut être détoxifié que lorsque le produit d'un gène porté par le vecteur d'expression est exprimé dans la cellule. Il s'ensuit une synchronisation entre la réplication de l'hôte et celle du transgène. Avantageusement, on utilise des vecteurs épisomaux dérivés du virus Epstein-Barr qui permettent l'expression dans une même cellule de 50 à 100 copies du vecteur (Deiss et al, 1996, EMBO J., 15, 3861-3870 ; Kissil et al, 1995, J. Biol. Chem, 270, 27932-27936).

L'intérêt de ces banques antisens alliées aux séquences DATAS qu'elles contiennent est d'identifier non seulement quel gène a eu son expression inhibée pour amener au phénotype sélectionné mais aussi d'identifier quelle isoforme d'épissage de ce gène a été affectée. Lorsque le fragment antisens cible un exon donné, il peut en être déduit que le domaine protéique et donc la fonction impliquant ce domaine s'oppose au phénotype observé. En cela le couplage de DATAS avec une approche antisens représente un raccourci vers la génomique fonctionnelle.

### Puces à ADN

L'invention concerne également tout support (membrane, filtre, biopuce, chip, etc) comprenant une banque ou une composition d'acides nucléiques telle que définie ci-dessus. Il peut s'agir plus particulièrement d'une banque cellulaire ou d'une banque d'acides nucléiques. L'invention concerne également tout kit ou support comprenant plusieurs banques selon l'invention. En particulier, il peut être avantageux d'utiliser en parallèle une banque représentative des qualités d'un état physiologique test par rapport à un état physiologique de référence et, à titre de contrôle, une banque représentative des qualités de l'état physiologique de référence par rapport à l'état physiologique test ("paire de banques"). Un kit avantageux selon l'invention comprend donc deux banques qualitatives différentielles de deux situations physiologiques (une "paire de banques"). Selon un mode de réalisation particulier, les kits de l'invention comprennent plusieurs paires de banques telles que définies ci-dessus, correspondant à différents états physiologiques ou à différents échantillons biologiques par exemple. Les kits peuvent comprendre par exemple ces différentes paires de banques déposées en série sur un même support.

### Génération de sondes

Une autre utilisation des compositions d'ADNc selon l'invention, représentatifs des différences qualitatives qui existent entre deux états physiopathologiques, consiste à en dériver des sondes. De telles sondes peuvent en effet être utilisées pour cribler les événements épissés de façon différentielle entre deux situations physiopathologiques.

Ces sondes (voir figure 1D) peuvent être préparées par marquage des populations ou banques d'acides nucléiques selon les techniques classiques, connues de l'homme du métier. Ainsi, il peut s'agir de marquage enzymatique, radioactif, fluorescent, immunologique, etc. Préférentiellement, il s'agit d'un marquage radioactif ou fluorescent. Ce type de marquage peut être réalisé par exemple en introduisant sur la population d'acides nucléiques (soit après synthèse soit au cours de leur synthèse) des nucléotides marqués, permettant leur révélation par les méthodes conventionnelles.

Une application est donc de cribler une banque génomique classique. Une telle banque peut comprendre selon le vecteur, dérivé d'un phage ou d'un cosmide, des fragments d'ADN de 10kb à 40kb. Le nombre de clones hybridant avec les sondes générées par DATAS et représentatifs des différences d'épissage qui existent entre deux situations reflète donc à peu près le nombre de gènes affectés par des variations d'épissage, selon qu'ils sont exprimés dans l'une ou l'autre des situations étudiées.

De préférence, les sondes de l'invention sont utilisées pour cribler une banque d'ADN génomique (généralement humaine) adaptée à l'identification d'événements d'épissage. De préférence, une telle banque génomique est composée de fragments d'ADN de taille restreinte (généralement clonés dans des vecteurs), de façon à statistiquement ne recouvrir qu'un seul élément épissable différentiellement, c'est à dire un seul exon ou un seul intron. La banque d'ADN génomique est donc préparée par digestion d'ADN génomique avec une enzyme ayant un site de reconnaissance restreint par 4 bases, assurant ainsi la possibilité d'obtenir par digestion ménagée des fragments d'ADN de taille moyenne de 1kb. De tels fragments nécessitent l'obtention de 10⁷ clones pour constituer une banque d'ADN représentative d'un génome d'organisme eucaryote supérieur. Une telle banque constitue également un objet de la présente demande. Cette banque est ensuite hybridée avec les sondes dérivées du criblage différentiel qualitatif. Précisément, de chaque expérimentation considérée et qui compare deux situations physiopathologiques A et B, deux sondes (paire de sondes) sont obtenues. Une sonde enrichie en événements d'épissages caractéristiques de la situation A et une sonde enrichie en marqueurs d'épissage de B. Les clones de la banque génomique qui hybrident préférentiellement avec l'une ou l'autre sonde portent des séquences préférentiellement épissées dans les situations physiopathologiques correspondantes.

Les méthodes de l'invention permettent ainsi l'identification systématique de différences qualitatives d'expression génique. Ces méthodes présentent de nombreuses applications, dans l'identification et/ou le clonage de molécules d'intérêt, en toxicologie, en pharmacologie ou encore en pharmacogénomique par exemple.

### Applications

L'invention concerne donc également l'utilisation des méthodes, acides nucléiques ou banques décrite ci-dessus pour l'identification de molécules d'intérêt thérapeutique ou diagnostique. L'invention concerne plus particulièrement l'utilisation des méthodes, acides nucléiques ou banques décrite ci-dessus pour l'identification de protéines ou domaines protéiques affectés dans une pathologie.

L'un des atouts de ces techniques est en effet d'identifier à l'intérieur d'un messager, et par conséquent de la protéine correspondante, les domaines fonctionnels qui sont affectés dans une pathologie donnée. Cela permet d'assigner à un domaine donné une importance dans le développement ou le maintien d'un état pathologique. L'avantage immédiat de restreindre à un domaine précis d'une protéine l'impact d'une dérégulation pathologique est de proposer celui-ci comme une cible relevante pour un criblage de petites molécules à visée thérapeutique. Ces informations constituent également des clefs qui permettent de concevoir des polypeptides à activité thérapeutique délivrables par thérapie génique; Ces polypeptides peuvent notamment être des anticorps simples chaînes dérivés d'anticorps neutralisants dirigés contre les domaines identifiés par les techniques décrites ci avant.

Plus spécifiquement, les méthodes selon l'invention permettent d'obtenir des molécules qui peuvent:
- être des séquences codantes qui dérivent d'exons alternatifs.
- correspondre à des séquences non codantes portées par des introns épissés différentiellement d'un état physiopathologique à un autre.

De ces deux points, différents enseignements peuvent être tirés.

Les épissages alternatifs d'exons qui différencient deux états physiopathologiques traduisent un niveau de régulation de l'expression génétique qui permet de moduler ( plus précisément d'abolir ou d'instaurer) une ou plusieurs fonctions d'une protéine donnée. Ainsi la plupart des domaines structuraux et fonctionnels (SH2, SH3, PTB, PDZ, et les domaines catalytiques de différentes enzymes...) étant codés par plusieurs exons contigus, deux configurations peuvent se présenter :
i) Les domaines sont tronqués dans la situation pathologique (Zhu, Q. et al, 1994, J. Exp. Med., vol 180, n°2, pp461-470); cela indique que les chemins de signalisation impliquant ces domaines doivent être restaurés dans un but thérapeutique.
ii) Les domaines sont maintenus au cours d'une pathologie alors qu'ils sont absents dans une situation saine; ces domaines peuvent être considérés comme des cibles de criblage de petites molécules chimiques destinées à antagoniser les signaux transduits par l'intermédiaire de ces domaines.

Les séquences épissées différentiellement peuvent correspondre à des régions non-codantes situées en 5' ou en 3' de la séquence codante ou à des introns intervenant entre deux exons codants. Dans les régions non codantes, ces épissages différentiels peuvent traduire une modification de la stabilité ou de la traductibilité du messager (Bloom, T. J., and Beavo, J. A., 1995, Proc. Natl. Acad. Sci. USA, vol93, n° 24, pp 14188-14192; Ambartsumian, N. et al., 1995, Gene, vol 159, n° 1, pp 125-130). Ces phénomènes doivent alors être recherchés sur la base de ces informations et peuvent mettre en évidence que l'accumulation ou la disparition de la protéine correspondante la désigne ainsi comme cible d'intérêt. Lorsque la rétention d'un intron se produit dans une séquence codante, il s'ensuit le plus souvent une troncature de la protéine naturelle par introduction de codon stop dans la phase de lecture (Varesco, L., et al, 1994, Hum. Genet., vol 93, n°3, pp281-286; Canton, H., et al., 1996, Mol. Pharmacol., vol 50, n° 4, pp799-807, Ion, A., et al, 1996, Am. J. Hum. Genet., vol 58, n°6, pp1185-1191). Avant de rencontrer ce codon stop, il se produit généralement une lecture de quelques codons supplémentaires, ce qui aboutit à adjoindre à la partie déjà traduite une séquence spécifique, témoin protéique de l'épissage alternatif. Ces acides aminés supplémentaires peuvent être utilisés pour générer des anticorps spécifiques de la forme alternative caractéristique de la situation pathologique. Ces anticorps peuvent être ensuite utilisés comme outils de diagnostic. La protéine tronquée voit ses propriétés modifiées, voire altérées. Ainsi des enzymes peuvent être amputées de leur domaine catalytique ou de leur domaine régulateur, devenant inactives ou constitutivement activées. Des adaptateurs peuvent perdre leur capacité à connecter différents partenaires d'une cascade de signalisation(Watanabe, K. et al, 1995, J. Biol. Chem., vol 270, n°23, pp13733-13739). Les produits d'épissage des récepteurs peuvent aboutir à des récepteurs qui ont perdu leur capacité à lier leur ligand (Nakajima, T. et al, 1996, Life Sci., vol58, n°9, pp761-768) et peuvent également générer des formes de récepteurs solubles par relargage de leur domaine extracellulaire (Cheng J., 1994, Science, vol263, n° 5154, pp1759-1762). Dans ce cas, des tests diagnostiques peuvent être envisagés, basés sur la circulation dans les différents fluides physiologiques de forme soluble de récepteur à un ligand donné.

L'invention concerne plus particulièrement l'utilisation des méthodes, acides nucléiques ou banques décrits ci-dessus pour l'identification de domaines antigéniques spécifiques de protéines impliquées dans une pathologie. L'invention concerne également l'utilisation des acides nucléiques, protéines ou peptides tels que décrits ci-avant pour le diagnostic de pathologies.

L'invention concerne également une méthode d'identification et/ou de production de protéines ou domaines protéiques impliqués dans une pathologie comprenant :
(a) l'hybridation des ARN messagers d'un échantillon pathologique avec les ADNc d'un échantillon sain, ou l'inverse, ou les deux en parallèle,
(b) l'identification, dans les hybrides formés, des régions correspondant aux différences qualitatives (non appariées (ARN) ou appariées (ADN double brin)), spécifiques de l'état pathologique par rapport à l'état sain,
(c) l'identification et/ou la production de la protéine ou domaine protéique correspondant à une ou plusieurs régions identifiées en (b).

Les régions identifiées correspondent généralement à des épissages différentiels, mais il peut également s'agir d'autres altérations génétiques telles que insertion(s) ou délétion(s), par exemple.

La ou les protéines ou domaines protéiques peuvent être isolés, séquencés, et utilisés dans des applications thérapeutiques ou diagnostiques, notamment pour la préparation d'anticorps.

A titre d'exemple plus spécifique, le criblage différentiel qualitatif de l'invention permet avantageusement de mettre en évidence des gènes suppresseurs de tumeurs. En effet, de nombreux exemples indiquent que l'un des modes d'inactivation des gènes suppresseurs au cours de la progression tumorale est une inactivation par modulation de l'épissage de formes alternatives.

Ainsi, dans les carcinomes pulmonaires à petites cellules, le gène de la protéine p130 qui appartient à la famille RB (protéine du rétinoblastôme) est muté à un site consensus d'épissage. La conséquence de cette mutation est l'élimination de l'exon 2 et une non synthèse de protéine due à la présence d'un codon stop précoce. Cette observation a été la première à souligner l'importance des membres de la famille RB dans la tumorigénèse. De même, dans certains cancers du poumon non à petites cellules, le gène de la protéine p161NK4A, protéine qui est un inhibiteur des kinases cyclines-dépendantes cdk4 et cdk6 est muté dans un site donneur d'épissage. Le résultat de cette mutation est la production d'une protéine tronquée à demi-vie courte ce qui a pour conséquence l'accumulation des formes phosphorylées, inactives, de RB. Par ailleurs, WT1, le gène suppresseur de tumeur de Wilms, est transcrit en plusieurs ARN messagers générés par épissages alternatifs. Dans les cancers du sein, les proportions relatives des différents variants sont modifiées par rapport au tissu sain, fournissant des outils diagnostics et des pistes pour comprendre l'importance des différents domaines fonctionnels de WT1 dans la progression tumorale. Ce même phénomène de modification des rapports entre différentes formes d'ARN messagers et d'isoformes protéiques lors de la transformation cellulaire est retrouvé pour la neurofibrine NF1. En outre, cette notion de modulation des phénomènes d'épissage qui signe la progression tumorale est soutenue également par l'exemple de HDM2 dont 5 épissages alternatifs sont détectés dans les carcinomes ovariens et pancréatiques et dont les expressions augmentent selon le stade d'avancement tumoral. D'autre part, dans les cancers de la tête et du cou, l'un des mécanismes d'inactivation de p53 implique une mutation dans un site consensus d'épissage.

Ces quelques exemples listés illustrent tout l'intérêt des techniques de l'invention basées sur la recherche systématique des variations d'épissage qui distinguent une tumeur donnée du tissu sain voisin. Les résultats qui en découlent permettent en effet non seulement la caractérisation de gènes suppresseurs de tumeurs déjà connus mais également, compte tenu de l'aspect original et systématique des techniques de criblage différentiel qualitatif, l'identification de nouvelles variations d'épissages spécifiques de tumeurs affectant vraisemblablement de nouveaux gènes suppresseurs de tumeurs.

L'invention a donc également pour objet un procédé d'identification et/ou de clonage de gènes suppresseurs de tumeurs ou d'altérations génétiques (e.g., d'épissages) au sein de gènes suppresseurs de tumeurs, tel que défini ci-avant. Ce procédé peut avantageusement comprendre les étapes suivantes :
(a) l'hybridation des ARN messagers d'un échantillon de tumeur avec les ADNc d'un échantillon sain, ou l'inverse, ou les deux en parallèle,
(b) l'identification, dans les hybrides formés, des régions spécifiques de l'échantillon tumoral par rapport à l'état sain,
(c) l'identification et/ou le clonage de la protéine ou domaine protéique correspondant à une ou plusieurs régions identifiées en (b).

Les propriétés suppresseur de tumeur des protéines ou domaines identifiés peuvent ensuite être testées dans différents modèles connus. Ces protéines, ou leur forme native (possédant l'épissage observé dans le tissu sain), peuvent ensuite être utilisées dans des applications thérapeutiques ou diagnostiques, notamment en thérapie génique antitumorale.

La présente demande a donc également pour objet non seulement les différents aspects de mise en oeuvre de la technologie mais aussi l'exploitation des informations qui en découlent à des fins de recherche, de développement de criblage de petites molécules chimiques, de développement d'outils de thérapie génique ou de diagnostic.

A ce titre, l'invention concerne également l'utilisation des méthodes, acides nucléiques ou banques décrits ci-dessus en génotoxicologie, c'est-à-dire pour anticiper (prédire) le potentiel toxique de composés tests.

Les programmes génétiques engagés lors du traitement de cellules ou de tissus par des agents toxiques sont en grande partie corrélés aux phénomènes d'apoptose ou mort cellulaire programmée. L'importance des phénomènes d'épissage alternatif dans la régulation de ces voies apoptotiques est bien illustrée par la littérature. Cependant, aucune technologie génomique décrite jusqu'à présent ne permettait de rechercher systématiquement et d'isoler de manière exhaustive les variations de séquences dues à des épissages alternatifs et distinguant deux situations physiopathologiques données. Les techniques de criblage différentiel qualitatif développées dans la présente invention permettent de regrouper l'ensemble des différences d'épissage qui existent entre deux situations dans des banques d'ADNc. La comparaison des séquences d'ARN (par exemple les ARN messagers) d'un tissu (ou d'une culture cellulaire) traité ou non avec un composé toxique de référence permet d'établir des banques d'ADNc qui regroupent les différences qualitatives de l'expression génique qui
caractérisent l'action toxique étudiée. Ces banques d'ADNc peuvent ensuite être hydridées avec des sondes dérivées d'ARN extrait des mêmes tissus ou cellules traités avec un produit chimique dont on veut évaluer le potentiel toxique. La plus ou moins grande capacité de ces sondes à s'hybrider avec les informations génétiques spécifiques d'une situation toxique de référence permet de lui assigner un potentiel toxique. Par ailleurs, outre l'application de DATAS à la génération et l'utilisation de banques de différences qualitatives induites par des agents toxiques, une partie de l'invention consiste également à démontrer que des dérégulations dans l'épissage de certains ARNs messagers peuvent être induites par certains agents toxiques, à des doses inférieures aux IC50 mesurées dans des tests de cytotoxicité et d'apoptose connus de l'homme de l'art. De telles dérégulations (ou dysrégulations) peuvent être utilisées comme marqueurs pour le suivi de la toxicité et/ou de l'efficacité de molécules (chimiques ou génétiques).

L'invention concerne donc également toute méthode de détection ou de suivi du potentiel toxique et/ou thérapeutique d'un composé basée sur la détection de formes et/ou de profils d'épissages induits par ce composé sur un échantillon biologique. Elle concerne en outre l'utilisation de toute modification de formes et/ou de profils d'épissages comme marqueur pour le suivi de la toxicité et/ou de l'efficacité de molécules.

L'évaluation ou le suivi du potentiel toxique peut s'effectuer plus particulièrement selon deux approches :

Selon la première approche, le criblage différentiel qualitatif peut être réalisé entre un tissu ou une culture cellulaire de référence, d'une part non traité et d'autre part traité par le produit dont on veut évaluer la toxicité. L'analyse des clones qui représentent les différences qualitatives spécifiquement induites par le produit permet ensuite éventuellement de détecter dans ces clones des événements caractéristiques d'ADNc impliqués dans les phénomènes liés à la toxicité comme l'apoptose.

L'apparition de ces marqueurs est suivie selon la dose et la durée du traitement par le produit et permet une approche de son profil toxicologique.

La présente demande a donc également pour objet un procédé d'identification, par criblage différentiel qualitatif selon les techniques présentées ci-dessus, de marqueurs de toxicité induits dans un système biologique modèle par un composé chimique dont on veut tester le potentiel toxique. A cet égard, l'invention concerne notamment un procédé d'identification et/ou de clonage d'acides nucléiques spécifiques d'un état toxique d'un échantillon biologique donné comprenant la préparation de banques différentielles qualitatives entre les ADNc et les ARN de l'échantillon après ou sans traitement par un composé toxique test, et la recherche de marqueurs de toxicité spécifiques des qualités de l'échantillon après traitement.

Selon la deuxième approche, des abaques pour différentes classes de produits toxiques rassemblent leur profil de toxicité selon les doses employées et selon les durées des traitements pour un tissu ou un modèle cellulaire de référence. A chaque point de ces abaques, des banques d'ADNc caractéristiques des différences génétiques qualitatives peuvent être établies. Ces banques sont des banques différentielles qualitatives, i.e., elles sont obtenues par extraction des informations génétiques du point choisi dans les abaques et du point correspondant au modèle tissulaire ou cellulaire contrôle. Comme cela est illustré dans les exemples, le criblage différentiel qualitatif repose sur l'hybridation d'ARNm extraits d'une situation avec les ADNc issues d'une autre. Comme indiqué plus haut, le criblage différentiel qualitatif peut être également mené à partir d'ARN totaux ou d'ARN nucléaires contenant les prémessagers.

A cet égard, l'invention concerne un procédé de détermination ou d'évaluation de la toxicité d'un composé test sur un échantillon biologique donné comprenant l'hybridation :
- de banques différentielles entre les ADNc et les ARN dudit échantillon biologique à l'état sain et à un ou différents stades de toxicité résultant d'un traitement dudit échantillon avec un composé toxique de référence, avec,
- une préparation d'acides nucléiques de l'échantillon biologique traité par ledit composé test, et
- l'évaluation du potentiel toxique du composé test par analyse du degré d'hybridation avec les différentes banques.

Selon ce procédé, pour chaque situation (doses de composé et/ou temps d'incubation), deux hybridations réciproques sont avantageusement réalisées, entre :
- les ARN de la situation A (test) et les ADNc de la situation B (référence) ( rA/cB)
- les ARN de la situation B (référence) et les ADNc de la situation A (test) (rB/cA).

A chaque situation toxique de référence, à chaque point des abaques, correspondent donc deux banques de criblage différentiel qualitatif. L'une de ces banques regroupe les variations qualitatives, c'est à dire notamment les épissages alternatifs, spécifiques de la situation normale de référence alors que l'autre banque rassemble les épissages spécifiques des événements toxiques. Ces banques sont répliquées sur des supports solides tels des filtres de nylon ou de nitrocellulose ou avantageusement sur des chips. Ces banques formées initialement par des fragments d'ADNc de longueur variable (selon les événements d'épissages concernés) peuvent être optimisées par l'emploi d'oligonucléotides dérivés des séquences isolées initialement.

Lorsqu'un composé chimique est proposé pour un développement pharmaceutique, il peut être appliqué sur les mêmes modèles tissulaires ou cellulaires que ceux répertoriés dans les abaques de toxicité. Des sondes moléculaires peuvent ensuite être réalisées à partir d'ARNm extraits des échantillons biologiques traités par le composé chimique d'intérêt. Ces sondes sont ensuite hybridées à des filtres portant l'ADNc des banques rA/cB et rB/cA. Par exemple, la banque rA/cB peut contenir les séquences spécifiquement présentes dans la situation normale et la banque rB/cA les éléments d'épissage alternatifs spécifiques de la situation de toxicité. L'innocuité ou la toxicité du composé chimique est alors aisément évaluée en fonction des profils d'hybridation d'une sonde dérivée d'ARNm extraits du modèle tissulaire ou cellulaire de référence traité par le composé testé:
- une hybridation efficace avec la banque rA/cB et aucun signal sur la banque rB/cA révèle une absence de toxicité du composé sur le modèle étudié
- l'hybridation de la sonde avec des clones de la banque rB/cA indique une toxicité induite par le composé testé.

Des exemples d'application de constitutions de telles banques peuvent être fournis par des modèles de culture d'hépatocytes, telle la lignée HepG2, de cellules épithéliales rénales, telle la lignée HK-2, ou de cellules endothéliales, telle la lignée ECV304, traités par des agents toxiques tel l'éthanol, la camptothécine ou le PMA.

Un exemple de choix peut également être fourni par l'utilisation en cosmétologie de modèles de culture de peau traités ou non par des agents toxiques ou irritants.

La présente demande a donc également pour objet des banques de criblage différentiel (entre les ADNc et les ARN), réalisées à partir d'organes, de tissus ou de cultures cellulaires de référence traités avec des composés chimiques représentatifs de grandes classes d'agents toxiques selon les abaques décrites dans la littérature. L'invention concerne aussi l'étalement de ces banques sur des filtres ou sur des supports connus de l'homme de l'art (nitrocellulose, nylon...). Avantageusement ces supports peuvent être des chips ou puces qui définissent ainsi des puces de génotoxicité. L'invention concerne en outre l'exploitation qui peut être faite du séquençage des différents clones qui constituent ces banques dans le but d'élucider les mécanismes mis en jeu par l'action des différents toxiques, ainsi que l'utilisation de ces banques pour les hybrider avec des sondes provenant de cellules ou de tissus traités par un composé chimique ou un produit pharmaceutique dont on veut évaluer la toxicité. Avantageusement, l'invention concerne des banques d'acides nucléiques telles que définies ci-avant, préparées à partir de cellules de la peau traitées en différentes conditions toxiques. L'invention concerne en outre un kit comprenant ces différentes banques différentielles de la peau.

L'invention concerne également l'utilisation des méthodes, acides nucléiques ou banques décrits ci-dessus pour évaluer (prédire) ou améliorer le potentiel thérapeutique de composés tests (génopharmacologie).

Dans cette utilisation, le principe mis en application est très proche de celui décrit précédemment. Des banques différentielles de référence sont établies entre les ADNc et les ARN d'une culture cellulaire ou d'un organe dans une situation contrôle et de leur équivalent mimant un modèle de pathologie. L'efficacité thérapeutique d'un produit peut alors être évaluée en suivant sa capacité à antagoniser les variations qualitatives de l'expression génique qui sont spécifiques du modèle pathologique. Cela est mis en évidence par la modification du profil d'hybridation d'une sonde issue du modèle pathologique sur les banques de références: sans traitement, la sonde n'hybride qu'avec la banque qui contient les signatures spécifiques de la maladie. Après traitement avec un produit efficace, la sonde bien que provenant du modèle pathologique hybride préférentiellement avec l'autre banque, qui porte les signatures du modèle équivalent sain.

A cet égard, l'invention concerne également un procédé de détermination ou d'évaluation de l'efficacité thérapeutique d'un composé test sur un échantillon biologique donné comprenant l'hybridation :
- de banques différentielles entre les ADNc et les ARN dudit échantillon biologique à l'état sain et à (différents stades de développement de) l'état pathologique avec,
- une préparation d'acides nucléiques de l'échantillon biologique traité par ledit composé test, et
- l'évaluation du potentiel thérapeutique du composé test par analyse du degré d'hybridation avec les différentes banques.

Un exemple d'une telle application peut être fourni par un modèle d'apoptose mimant certains aspects de la neurodégénérescence qui sont antagonisés par des facteurs trophiques de référence. Ainsi les cellules dérivées de phéochromocytômes PC12 différenciées en corps neuronaux en présence de NGF entrent en apoptose par retrait de ce facteur de croissance. Cette apoptose est accompagnée par expression de nombreux marqueurs de mort cellulaire programmée dont plusieurs sont régulés par épissage alternatif et dont l'apparition est inhibée par action d'IGF1. Deux banques issues de criblage différentiel qualitatif sont établies à partir d'ARNm extraits de cellules PC12 différenciées entrées en apoptose par retrait de NGF d'une part et à partir de PC12 différenciées sauvegardées de l'apoptose par ajout d'IGF1 d'autre part. Sur ces banques peuvent être hybridées des sondes réalisées à partir d'ARNm extraits de PC12 différenciées entrées en apoptose et dont la survie est améliorée par traitement avec un produit neuroprotecteur à tester. L'efficacité de l'inversion des caractéristiques qualitatives induites par le composé test peut donc être appréciée par la capacité de la sonde à hybrider spécifiquement les clones spécifiques de la banque représentative des cellules dont la survie est améliorée. Ce test peut par la suite être utilisé pour tester l'efficacité de dérivés du composé ou de tout autre nouvelle famille de composés neuroprotecteurs et en améliorer le profil pharmacologique.

Dans un mode de réalisation particulier, le procédé de l'invention permet d'évaluer l'efficacité d'une composé test neuroprotecteur par hybridation avec une banque différentielle selon l'invention entre une cellule nerveuse saine et cette cellule présentant un modèle de neurodégénérescence.

Dans un autre mode, il s'agit de tester un composé anti-tumoral sur des banques différentielles établies à partir d'un échantillon de cellules tumorales et un échantillon sain.

Comme indiqué ci-avant, le procédé de l'invention peut en outre être utilisé pour améliorer les propriétés d'un composé, en testant différents dérivés pour leur capacité à induire un profil d'hybridation proche de la banque représentative de l'échantillon sain.

L'invention concerne également l'utilisation des méthodes, acides nucléiques ou banques décrits ci-dessus en pharmacogénomique, i.e., pour évaluer (prédire) la réponse d'un patient à un composé ou traitement test.

La pharmacogénomique a pour ambition d'établir des profils génétiques de patients afin de déterminer quel traitement est susceptible d'être couronné de succès pour une pathologie donnée. Les techniques décrites dans la présente invention permettent à cet égard d'établir des banques d'ADNc représentatives des différences qualitatives qui existent entre une situation pathologique qui répond à un traitement donné et une autre qui répond peu ou mal, susceptible d'être l'objet d'une autre stratégie thérapeutique. Ces banques de références établies, elles peuvent être hybridées avec des sondes réalisées à partir d'ARN messagers de patients. Les résultats d'hybridation permettent de savoir quel patient a un profil d'hybridation correspondant à la situation de répondeur ou de non répondeur et ainsi d'affiner le choix de traitement.

Dans cette application, le but est d'une part de proposer en fonction du patient le traitement le plus approprié, le plus susceptible d'être couronné de succès et d'autre part d'enrôler dans un traitement les patients les plus susceptibles d'y répondre avec succès. Comme dans les autres applications, deux banques de criblage différentiel qualitatif sont réalisées: l'une à partir d'un modèle ou d'un échantillon pathologique connu pour répondre à un traitement, l'autre à partir d'un autre modèle ou échantillon pathologique qui répond peu ou mal à l'action thérapeutique. Ces deux banques sont ensuite hybridées avec des sondes provenant d'ARNm extraits de biopsies de différents patients. Selon que ces sondes hybrident préférentiellement avec les épissages alternatifs spécifiques de l'une ou l'autre situation, les patients peuvent être répartis en répondeurs et en non répondeurs au traitement de référence qui a défini les modèles de départ.

A cet égard, l'invention concerne également un procédé de détermination ou d'évaluation de la réponse d'un patient à un composé ou traitement test comprenant l'hybridation :
- de banques différentielles entre les ADNc et les ARN d'un échantillon biologique répondeur audit composé/traitement et d'un échantillon biologique non-répondeur ou mal-répondeur audit composé/traitement, avec,
- une préparation d'acides nucléiques d'un échantillon biologique pathologique du patient, et
- l'évaluation du potentiel répondeur du patient par analyse du degré d'hybridation avec les différentes banques.

Un exemple de choix de l'apport du criblage différentiel qualitatif à la pharmacogénomique est constitué par un criblage différentiel qualitatif entre deux tumeurs de même origine histologique, l'une régressant lors du traitement par un composé antitumoral (par exemple un transfert d'un ADNc codant pour la protéine p53 sauvage par thérapie génique), l'autre se montrant réfractaire à ce traitement. La première retombée de la constitution de banques de différences qualitatives entre ces deux situations est de déterminer, par analyse des clones qui constituent ces banques, quels mécanismes moléculaires sont mobilisés lors de la régression du premier modèle et ne sont pas présents dans le deuxième.

Ensuite, l'utilisation de filtres ou tout autre support présentant les ADNc de ces banques permet de réaliser des hybridations avec des sondes dérivées d'ARNm de biopsies de tumeurs dont on veut anticiper la réponse audit traitement. Ces résultats permettent ainsi de proposer un enrôlement optimisé des patients dans un protocole clinique.

Un exemple particulier de ce procédé consiste à déterminer la réponse de tumeurs à un traitement par le gène suppresseur de tumeur p53. ll a en effet été décrit que certains patients et certaines tumeurs répondent plus ou moins bien à ce type de traitement (Roth et al., Nature Medicine, 2 (1995) 958). Il est donc important de pouvoir déterminer quels types de tumeurs et/ou quels patients sont sensibles à un traitement par thérapie génique par p53 sauvage, afin d'optimiser le traitement et de favoriser l'enrôlement des patients dans les essais cliniques en cours. Le procédé de l'invention permet avantageusement de faciliter ces étapes en proposant des banques spécifiques des qualités de cellules répondeuses et de cellules non répondeuses à p53. Des exemples de modèles cellulaires p53-sensibles ou résistants sont décrits par exemple par Sabbatini et al. (Genes Dev. 9 (1995) 2184) ou par Roemer et al. (Oncogene 12 (1996) 2069). L'hybridation de ces banques avec des sondes dérivées de biopsies de patients permet aisément d'évaluer leur potentiel répondeur. En outre les banques spécifiques permettent également d'identifier des acides nucléiques impliqués dans la réponse à p53.

La présente demande concerne donc également l'établissement de banques de criblage différentiel à partir d'échantillons pathologiques, ou de modèles de pathologie, qui répondent différemment à au moins un agent pharmacologique. Ces banques peuvent être des banques restreintes, complexes ou autologues comme définies ci-dessus. Elle concerne aussi l'étalement de ces banques sur des filtres ou sur des supports connus de l'homme de l'art (nitrocellulose, nylon...). Avantageusement ces supports peuvent être des chips ou puces qui définissent ainsi des puces de pharmacogénomique. L'invention porte encore sur l'exploitation qui peut être faite du séquençage des différents clones qui constituent ces banques dans le but d'élucider les mécanismes qui président aux différences de réponses d'échantillons pathologiques à différents traitement, ainsi que l'utilisation de ces banques pour les hybrider avec des sondes provenant de biopsies provenant de situations pathologiques dont on veut anticiper la réponse au traitement de référence qui définit les banques.

La présente invention décrit ainsi que des variations dans les formes et/ou profils d'épissages constituent des sources de marqueurs de pharmacogénomique, c'est-à-dire des sources de marqueurs permettant la mise en évidence de la capacité et de la manière d'un patient à répondre à des traitements. A cet égard, l'invention a donc également pour objet l'utilisation de l'intervariabilité, entre individus, des isoformes générées par épissage alternatif (analyse du spliceome) comme source de marqueurs de pharmacogénomique. L'invention concerne aussi l'utilisation de modifications d'épissage induites par des traitements comme source de marqueurs de pharmacogénomique. Ainsi, comme expliqué ci-avant, les methodologies DATAS de l'invention permettent de générer des acides nucléiques représentatifs des différences qualitatives entre deux échantillons biologiques. Ces acides nucléiques, ou des formes dérivées (sondes, amorces, acides complémentaires, etc.) peuvent être utilisés pour l'analyse du spliceome de sujets, en vue de mettre en évidence leur capacité/manière de répondre à des traitements, ou leur prédisposition à tel traitement/pathologie, etc.

Ces différents exemples généraux illustrent l'intérêt des banques de criblage différentiel qualitatif dans des études de génotoxicité, génopharmacologie, pharmacogénomique ainsi que dans des recherches de cibles d'intérêt diagnostique ou thérapeutique. Ces banques sont issues du clonage des différences qualitatives qui existent entre deux situations physiopathologiques. Puisqu'une autre utilisation des ADNc représentatifs de ces différences qualitatives est de constituer des sondes destinées à cribler une banque d'ADN génomique dont les caractéristiques ont été décrites ci avant, une telle approche peut être également mise en oeuvre pour toute étude de génotoxicité, génopharmacologie et pharmacogénomique ainsi que d'identification de gène. Dans les études de génotoxicité par exemple, les clones génomiques restreints par la taille de leurs insertions statistiquement à un seul intron ou à un seul exon sont classés sur des filtres en fonction de leur hybridation avec des sondes DATAS provenant de l'analyse différentielle qualitative entre une population cellulaire ou un tissu de référence et les mêmes cellules ou tissu traités par un composé toxique de référence. Ces clones représentatifs des différentes classes de toxicité étant sélectionnés, il peut ensuite être procédé à une hybridation de ces clones avec une sonde dérivée des ARN messagers totaux d'une même population cellulaire ou d'un même tissu traité par un composé dont on veut apprécier le potentiel toxique.

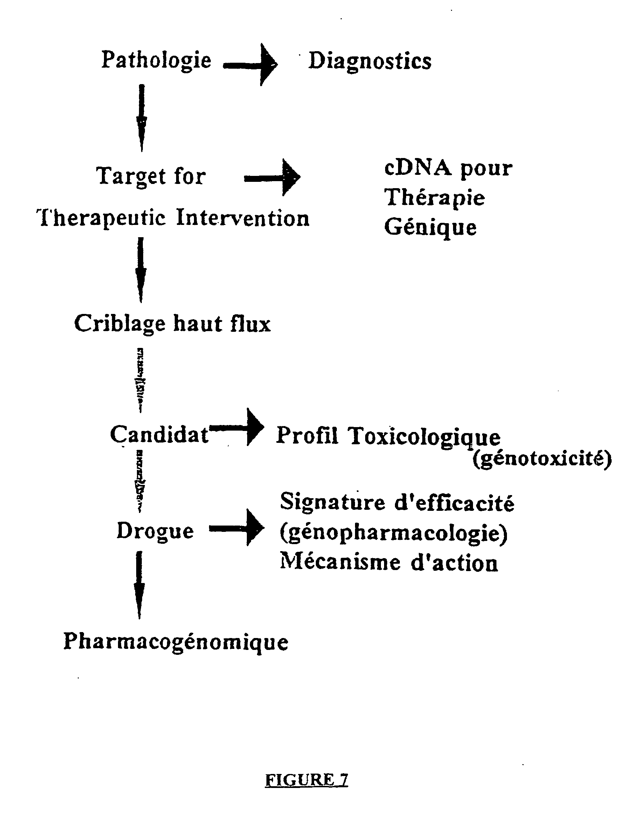

D'autres avantages et applications de la présente invention apparaîtront à la lecture des exemples qui suivent, qui doivent être considérés comme illustratifs et non limitatifs. Les champs d'application de l'invention sont représentés sur la figure 7.

### LEGENDE DES FIGURES

Figure 1. Représentation schématique des criblages différentiels selon l'invention (figure 1A) utilisant une (figure 1B) ou deux (figure 1C) hybridations, et utilisation des acides nucléiques (figure 1D).
Figure 2. Représentation schématique décrivant l'obtention d'hybrides ARN/ADN permettant de caractériser les séquences ARN simple brin, signatures spécifiques de l'état pathologique ou de l'état sain.
Figure 3. Représentation schématique décrivant un moyen permettant d'isoler et de caractériser par séquençage les séquences d'ARN simple brin spécifiques d'une situation pathologique ou d'une situation saine.
Figure 4. Représentation schématique décrivant un autre moyen permettant de caractériser par séquençage tout ou une partie des ARNs simple brin spécifiques d'une situation pathologique ou d'une situation saine.
Figure 5. Représentation schématique permettant d'isoler les produits d'épissages alternatifs grâce à des structures R-loop
Figure 6. Représentation schématique du criblage différentiel qualitatif par restriction de boucles (formation d'homoduplexes ADNcdb/ADNc et extractions des informations, Figure 6A) et description des informations obtenues (Figure 6B).
Figure 7. Apports du criblage différentiel qualitatif aux différentes étapes de la recherche et du développement pharmaceutique.
Figure 8. Isolation d'un domaine différentiellement épissé dans le modèle grb2/grb33. A) Production des ARNs synthétiques de grb2 et de grb33. B) Suivi des premières étapes de DATAS conduisant à la caractérisation d'un fragment ARN correspondant au domaine différentiellement épissé ; 1 : ARN de grb2, 2 : Hybridation entre l'ARN de grb2 et l'ADNc de grb33, 3 : Hybridation entre l'ARN de grb2 et l'ADNc de grb2, 4 : Hybridation entre l'ARN de grb2 et de l'eau, 5 : Surnageant après passage sur billes Streptavidine de (2), 6 : Surnageant après passage sur billes Streptavidine de (3), 7: Surnageant après passage sur billes Streptavidine de (4), 8 : Digestion du duplex ARN grb2 / ADNc grb33 à la Rnase H, 9 : Digestion du duplex ARN grb2 / ADNc grb2 à la Rnase H, 10 : Digestion de l'ARN grb2 à la Rnase H, 11 : pareil que (8) après passage sur colomne d'exclusion, 12: pareil que (9) après passage sur colomne d'exclusion, 13 : pareil que (10) après passage sur colomne d'exclusion.
Figure 9. Représentation des populations d'ARNs non appariées issues de la digestion par la Rnase H à partir de duplexes ARN / ADNc simple brin provenant de cellules HepG2 traitées ou non à l'ethanol.
Figure 10. Représentation des populations d'ADNc double brin générées par une des variantes de DATAS. 1 à 12 : PCRs à partir de populations de boucles d'ARNs issues de la digestion à la Rnase H, 13 : PCR à partir d'ADNc total.
Figure 11. Application de la variante de DATAS faisant intervenir les ADNc double brin sur le modèle grb2/grb33. A) Analyse sur gel d'agarose des complexes après hybridation : 1 : ADNc double brin grb2 / ARN grb33, 2 : ADNc double brin grb2 /ARN grb2, 3 : ADNc double brin grb2 / eau. B) Digestion des échantillons 1,2 et 3 de A) par la nucléase S1 et la nucléase " Mung Bean " : 1 à 3 : complexes 1 à 3 avant traitement au glyoxal ; 4 à 6 : complexes 1 à 3 après traitement au glyoxal ; 7 à 9 : Digestions de 1 à 3 par nucléase S1 ; 10 à 12 : Digestions de 1 à 3 par nucléase Mung Bean.
Figure 12. Application de la variante de DATAS faisant intervenir les ADNc simple brin et la Rnase H sur un système de cellules HepG2 traitées ou non à l'ethanol 0,1M pendant 18 heures. Les inserts clonés ont été transférés sur membrane après electrophorèse sur gel d'agarose et soumis à hybridation à l'aide de sondes correspondant aux situations traitées (Tr) ou non (NT).
Figure 13. Mode opératoire pour évaluer le potentiel toxique d'un produit.
Figure 14. Mode opératoire pour suivre l'efficacité d'un produit.
Figure 15. Mode opératoire pour étudier la susceptibilité d'une situation pathologique à un traitement.
Figure 16. Analyse d'hybridation différentielle de clones issus de DATAS à partir d'ARNs extraits de cellules induites et d'ADNc extraits de cellules non induites. A) Utilisation de colonies bactériennes déposées et lysées sur membrane. B) Southem Blot effectué à partir d'une sélection de clones de A.
Figure 17. Séquences nucléotidique et peptidique de ΔSHC (SEQ ID NO: 9 et 10).
Figure 18. Tests de cytotoxicité et d'apoptose sur cellules HepG2 traitées A) à l'ethanol ; B) à la camptothécine ; C) au PMA.
Figure 19. Réactions de RT-PCR effectuées à partir d'ARNs extraits de cellules HepG2 traitées ou non (N.T) par l'ethanol (Eth.), la camptothécine (Camp.) et le PMA (PMA) permettant l'amplification de fragments correspondants à des domaines de MACH-a, BCL-X, FASR et beta-actine comme contrôle de normalisation.

Dans les exemples et la description de l'invention, il est fait références aux séquences de la Liste de Sequences, qui contient le texte libre suivant:
<223> OLIGO
<223> OLIGO
<223> OLIGO
<223> OLIGO
<223> OLIGO
<223> OLIGO
<223> OLIGO
<223> OLIGO
<223> OLIGO
<223> OLIGO
<223> OLIGO
<223> OLIGO

### EXEMPLES

### 1. CLONAGE DIFFÉRENTIEL DES EPISSAGES ALTERNATIFS ET AUTRES MODIFICATIONS QUALITATIVES DES ARNS EN UTILISANT DES ADNc SIMPLE-BRINS

Les ARN messagers correspondant à deux situations, l'une normale (mN) et l'autre pathologique (mP), sont isolés à partir de biopsies ou de cellules en culture. Ces ARN messagers sont convertis en ADN complémentaires (cN) et (cP) à l'aide de reverse transcriptase (RT). Des hybrides mN/cP et cN/mP sont ensuite réalisés en phase liquide (se reporter au schéma de la figure 2 illustrant un des deux cas aboutissant à la formation de cN/mP).

Ces hybrides sont avantageusement réalisés en émulsion phénolique (technique PERT ou Phenol Emulsion DNA Reassociation Technique) maintenue par thermocycles (Miller, R.,D. and Riblet, R., 1995, Nucleic Acids Research, vol 23, n°12, pp 2339-2340). Typiquement, cette étape d'hybridation est réalisée entre 0,1 à 1 µg d'ARN polyA⁺ et 0,1 à 2µg d'ADN complémentaire dans une émulsion formée d'une phase aqueuse (tampon phosphate de sodium 120mM, NaCl 2,5M, EDTA 10mM) et d'une phase organique représentant 8% de la phase aqueuse et constituée de phénol bidistillé.

Une autre technique est également avantageusement employée de façon à obtenir des hétéroduplex : à l'issue de la transcription inverse, l'ADNc néosynthétisé est séparé de l'amorce oligodT biotinylée sur colonne d'exclusion. 0,1 à 2µg de cet ADNc est coprécipité avec 0,1 à 1µg d'ARN polyA⁺ en présence de 0,3M d'acétate de sodium et de deux volumes d'éthanol. Ces acides nucléiques coprécipités sont repris dans 30µl d'un tampon d'hybridation qui contient 80% de formamide, 40mM de PIPES (piperazinebis(2-ethanesulfonic acid)) ph6,4, 0,4M de NaCl et 1mM d'EDTA.

Les acides nucléiques en solution sont dénaturés par chauffage 10mn à 85°C puis leur hybridation est réalisée pendant au moins 16h et jusqu'à 48h à 40°C.

L'intérêt de la technique d'hybridation en formamide est de permettre des conditions de plus forte sélectivité lors de l'appariement des brins d'ADNc et d'ARN.

A l'issue de chacune de ces deux techniques d'hybridation, nous disposons d'héteroduplex ARN/ADN dont la perfection d'appariement dépend de l'efficacité de la RT à synthétiser la longueur totale des ADNc. Demeurent également sous forme de simples brins les régions d'ARN (et d'ADN) qui correspondent aux épissages alternatifs qui différencient les deux états physiopathologiques étudiés.

Le but de la méthode est ensuite de caractériser l'information génétique portée par ces boucles d'épissage.

Pour cela, les hétéroduplex sont purifiés par capture des ADNc (amorcés avec des oligodT biotinylés) grâce à des billes portant des groupements streptavidines. Avantageusement ces billes sont de billes douées de propriétés magnétiques, ce qui permet de les séparer des ARN non engagés dans les hétéroduplex par action d'un séparateur magnétique. De telles billes et de tels séparateurs sont disponibles commercialement.

Sont isolés à ce stade de la procédure les hétéroduplex et les ADNc non engagés dans des hybridations avec des ARN. Ce matériel est ensuite soumis à l'action de la RNaseH qui va spécifiquement hydrolyser les régions d'ARN hybridées avec les ADNc. Les produits résultant de cette hydrolyse sont d'une part les ADNc et d'autre part les fragments d'ARN qui correspondent aux boucles d'épissage ou aux régions non hybridées du fait du rendement partiel de la transcriptase inverse. Les fragments d'ARN sont séparés de l'ADN par séparation magnétique selon le même mode opératoire que celui mentionné plus haut et par digestion avec de la DNase exempte de toute contamination par une activité Rnase.

### 1.1. Validation de la technique DATAS sur les variants d'épissage du gène Grb2

Une mise en évidence de la faisabilité de cette approche a été réalisée sur un système in-vitro utilisant un ARN correspondant à la région codante de Grb2 d'une part et un ADNc simple brin complémentaire à la région codante de Grb3.3. Grb2 est un gène possédant une phase codante de 651 paires de bases. Grb33 est une isoforme de grb2 générée par épissage alternatif et comprenant une délétion de 121 paires de bases dans le domaine fonctionnel SH2 de grb2 (Fath et.al, Science (1994), 264, 971-4). Les ARNs de Grb2 et de Grb33 sont synthétisés selon les techniques connues de l'homme du métier à partir d'un plasmide contenant la séquence codante de Grb2 ou de Grb33 sous contrôle du promoteur T7 à l'aide du kit RiboMax (Promega). L'analyse des produits démontre une synthèse homogène (figure 8A). Dans un but de visualisation, l'ARN de Grb2 a également été rendu radioactif par incorporation d'une base marquée lors de la transcription in-vitro à l'aide du kit RiboProbe (Promega). Les ADNc de Grb2 et de Grb3.3 ont été synthétisés par transcription inverse à partir des ARNs synthétiques produits ci-dessus, du kit Superscript II (Life Technologies) et d'une amorce oligonucleotidique biotinylée commune à Grb2 et à Grb33 correspondant au complémentaire de la séquence (618-639) de Grb2. Les ARNs et ADNcs ont été traités selon les indications des fournisseurs (promega, Life Technologies), purifiés sur colonne d'exclusion (Rnase free sephadex G25 ou G50, 5 Prime, 3 Prime) et quantifiés par spectrophotométrie.

Les premières étapes de DATAS ont été appliquées en associant, en suspension 10ng d'ARN marqué de Grb2 avec :
1. 100 ng d'ADNc de grb33 biotinylé,
2. 100 ng d'ADNc de grb2 biotinylé,
3. de l'eau
dans 30 µl d'un tampon d'hybridation qui contient 80% de formamide, 40 mM PIPES (pH 6,4), 0,4 M NaCl, 1 mM EDTA. Les acides nucléiques sont dénaturés par chauffage 10 mn à 85 °C, puis l'hybridation est réalisée pendant 16 heures à 40°C. Après capture à l'aide de billes streptavidine, les échantillons sont traités à la RNase H comme décrit précédemment.

L'analyse de ces étapes est réalisée par electrophorèse sur gel d'acrylamide à 6% suivi d'un traitement des gels à l'aide d'un Instant imager (Packard Instruments) permettant la qualification et la quantification des espèces issues de l'ARN de grb2 marqué (figure 8B). Ainsi, les puits 2,3 et 4 indiquent que les duplexes grb2/grb33 et grb2/grb2 se sont formés de façon quantitative. La migration du complexe grb2/grb33 est retardée par rapport à celle de l'ARN de grb2 (puits 2) alors que celle du complexe grb2/grb2 est augmentée (puits 3). Les puits 5,6 et 7 correspondent aux échantillons non retenus par les billes streptavidine démontrant que 80% des complexes grb2/grb33 et grb2/grb2 ont été retenus sur les billes alors que l'ARN de grb2 seul, non biotinylé, se retrouve exclusivement dans le surnageant des billes. Le traitement à la Rnase H libère, outre les nucléotides libres qui migrent plus vite que le bleu de Bromophénol (BPB) une espèce migrant en deçà du bleu de xylène Cyanol (XC) (marqué par une flèche sur la figure) et ce spécifiquement dans le puits 8 correspondant au complexe grb2/grb33 par rapport aux puits 9 et 10 qui correspondent au complexe grb2/grb2 et à l'ARN de grb2. Les puits 11,12 et 13 correspondent aux puits 8,9 et 10 après passage des échantillons sur une colomne d'exclusion pour éliminer les nucléotides libres. La migration observée dans les puits 8 et 11 est celle attendue pour une molécule d'ARN correspondant à la délétion de 121 nucléotides différenciant grb2 de grb33.

Ce résultat montre bien la possibilité d'obtenir les boucles d'ARN générées par la formation d'hétéroduplex entre deux séquences dérivées de deux isoformes d'épissage.

### 1.2. Application de la technique DATAS à la génération de banques qualitatives de cellules hépatiques dans un état sain et toxique

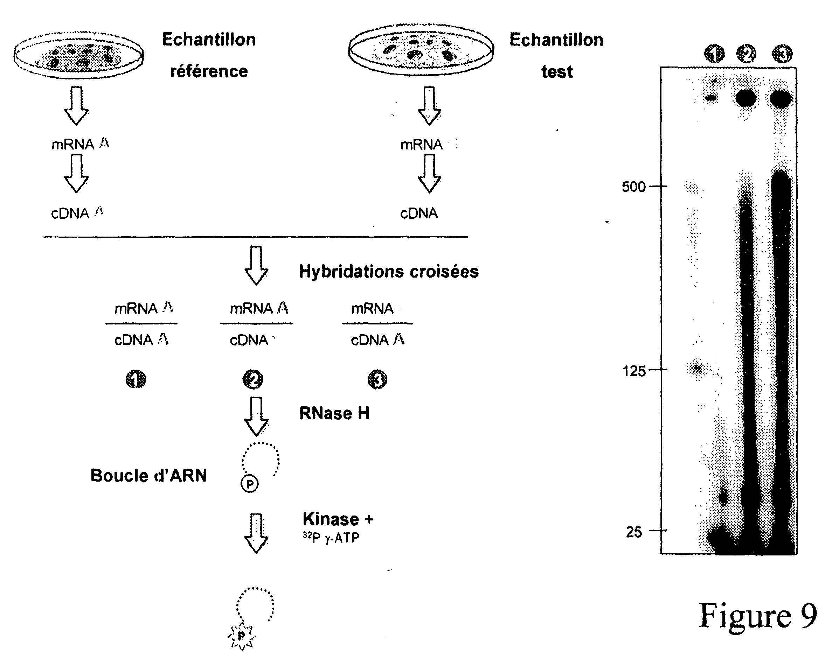

Une situation plus complexe a été étudiée. Dans le cadre de l'application de la technologie DATAS comme outil prédictif de toxicité de molécules, des cellules humaines de type hépatocytaire, HepG2, ont été traitées par de l'ethanol 0,1 M pendant 18 heures. Les ARNs ont été extraits à partir des cellules traitées ou non. La variante de DATAS décrite ci-dessus (préparation d'ADNc sb biotinylés, hybridations croisées en phase liquide, application d'un champs magnétique pour séparer les espèces, traitement RNaseH) a été appliquée avec les cellules non traitées en situation de référence (ou situation A) et les cellules traitées en situation test (ou situation B) (figure 9). Les ARNs extraits n'étant pas marqués radioactivement , la visualisation des populations d'ARN générées par digestion à la RnaseH est réalisée en effectuant une réaction d'échange du phosphate en 5' des ARNs par un phosphate marqué, à l'aide de polynucleotide kinase de T4 et de gamma-P³²ATP. Ces marquages sont ensuite déposés sur un gel d'acrylamide/urée et analysés par exposition à l'aide d'un Instant Imager (Packard Instruments). Des signatures complexes issues des hybridations A/B et B/A peuvent alors être visualisées avec un premier groupe de signaux migrant faiblement dans le gel et correspondant à des séquences d'acides nucléiques de taille importante et un deuxième groupe de signaux migrant entre 25 et 500 nucleotides. Ces signatures sont d'intensité beaucoup plus faible à partir de la situation A/A suggérant que l'ethanol peut induire une reprogrammation de l'épissage des ARNs, traduite par l'existence de signaux en A/B et B/A.

### 1.3. Clonage et Préparation de banques à partir des acides nucléiques identifiés.

Plusieurs variantes expérimentales sont ensuite envisageables pour cloner ces fragments d'ARN résistant à l'action de la Rnase H:
A. Une première approche consiste à isoler ces boucles et à les cloner (Figure 3).
   Selon cette approche, il est procédé à une ligation d'oligonucléotides à chacune des extrémités par action de la RNA ligase selon les conditions connues de l'homme de l'art. Ces oligonucléotides sont ensuite utilisés comme amorces pour effectuer une RT PCR. Les produits de PCR sont clonés et criblés avec des sondes d'ADN complémentaires totales correspondant aux deux situations physiopathologiques d'intérêt. Seuls les clones hybridant préférentiellement avec une seule des deux sondes contiennent les boucles d'épissage qui sont ensuite séquencées et/ou utilisées pour générer des banques.
B. La seconde approche (Figure 4) consiste à effectuer une transcription inverse sur l'ARN simple brin libéré des hétéroduplex après action de la RNaseH, initiée à l'aide d'amorces au moins en partie aléatoires. Ainsi, il peut s'agir d'amorces aléatoires en 3' et en 5', d'amorces aléatoires en 3' et déterminées en 5', ou encore d'oligonucléotides semi-aléatoires, c'est-à-dire comprenant une zone de dégénérescence et une zone définie.

Selon cette stratégie, les amorces sont donc susceptibles de s'hybrider soit n'importe où sur l'ARN simple brin, soit à chaque succession de bases fixée par le choix de l'amorce semi-aléatoire. Une PCR avec des amorces correspondant aux oligonucléotides décrits ci-dessus permet ensuite d'obtenir des séquences dérivées des boucles d'épissage.

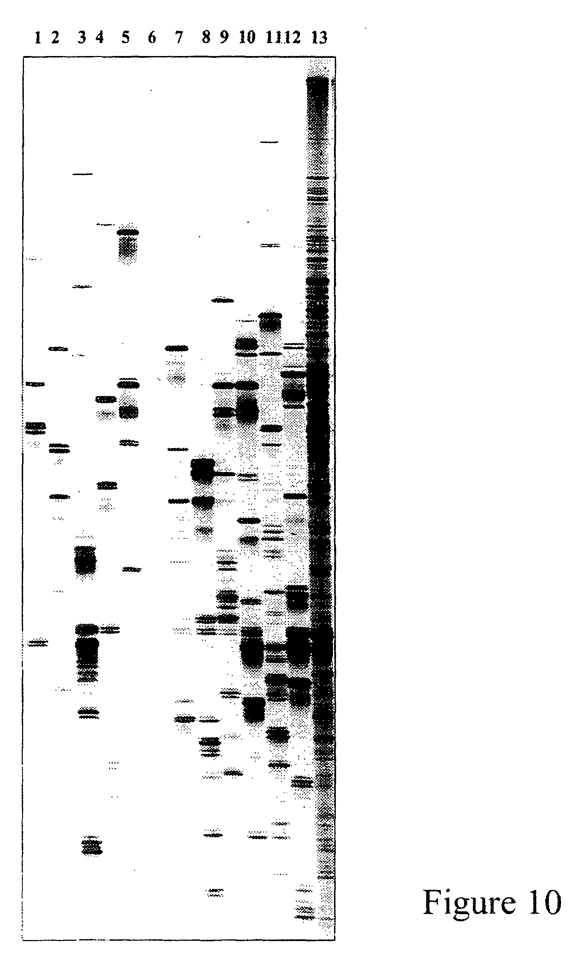

La figure 10 (puits 1 à 12) montre l'analyse sur gel d'acrylamide des fragments de PCR obtenus à partir de plusieurs essais DATAS et couplée à l'utilisation des oligonucléotides semi-aléatoires suivants:

La comparaison avec la complexité des signaux obtenus en utilisant les mêmes oligonucléotides, mais de l'ADNc total comme matrice (puits 13) démontre que DATAS a permis de filtrer ("profiler") des informations correspondant à des différences qualitatives.

Cette variante a été utilisée afin de cloner un événement correspondant au domaine ARN de grb2 généré par action de la Rnase H à partir du duplex ARN grb2 / ADNc simple brin de grb33 selon le protocole décrit précédemment (exemple 1.1.). A cette fin, un oligonucléotide de séquence : GAGAAGCGTTATNNNNNNNNTCCC (SEQ ID NO: 2), choisi sur le modèle GAGAAGCGTTATNNNNNNNWXYZ (dans lequel N est défini comme précédemment, W, X et Y représentent chacun une base fixe déterminée, et Z représente soit une base déterminée soit un groupe 3'-OH, SEQ ID NO: 3) et sélectionné pour amplifier un fragment dans la délétion de grb2 a été utilisé, permettant de générer un fragment PCR dont le clonage et le séquençage a démontré qu'il était effectivement issu du domaine délété de grb2 (194-281 dans grb2).

Ces deux approches permettent donc la production de compositions d'acides nucléiques représentatifs des épissages différentiels dans les deux situations testées qui peuvent être employées comme sondes
ou pour construire des banques d'ADNc de différences qualitatives. La capacité de la technologie DATAS à générer des banques profilées d'ADNc représentatives de différences qualitatives est également illustrée par l'exemple 1.4. suivant.

### 1.4. Production de banques profilées représentatives de cellules endothéliales humaines

Cet exemple a été réalisé à partir d'une lignée de cellules endothéliales humaines (ECV304). L'analyse qualitative de l'expression génétique a été réalisée à partir d'ARN cytosoliques extraits de cellules en prolifération, d'une part, et de cellules en anoïkis (apoptose par privation de support d'attachement), d'autre part.

Les cellules ECV ont été cultivées en milieu 199 supplémenté en sels de Earle (Life Sciences). Leur mise en anoïkis a été réalisée par passage pendant 4 heures sur boîtes de culture traitées au polyHEMA. Lors de la préparation des ARN, les cellules ont été lysées dans un tampon contenant du Nonidet P-40. Les noyaux sont ensuite écartés par centrifugation. La solution d'extrait cytoplasmique a été ensuite ajustée de manière à fixer de façon spécifique l'ARN à la matrice de silice Rneasy selon les instructions de la société Qiagen. Après lavage, les ARN totaux sont élués dans de l'eau traitée au DEPC. Les ARNs messagers sont préparés à partir des ARNs totaux par séparation sur billes magnétiques Dynabeads oligo (dT)₂₅ (Dynal). Après avoir mis en suspension les billes dans un tampon de fixation, l'ARN total est incubé pendant 5 min à température ambiante. Après séparation magnétique et lavage, les billes sont reprises dans un tampon d'élution pour une incubation à 65°C qui libère les ARNs messagers.

Les synthèses d'ADN premier brin sont effectuées à partir des ARNs messagers en utilisant la Reverse Transcriptase SuperScript II ou ThermoScript (Life Technologies) à l'aide d'amorces olido (dT). Après RnaseH, les nucléotides libres sont éliminés par passage sur colonne Séphadex G50 (5 Prime- 3 Prime). Après extraction au phénol /Chloroforme et précipitation à l'éthanol, les échantillons sont quantifiés par absorbance UV.

Les quantités requises d'ARN et d'ADNc (en l'occurrence 200ng de chaque) sont combinées et précipitées à l'ethanol. Les échantillons sont repris dans un volume de 30µl dans un tampon d'hybridation (Hepes (pH 7.2) 40 mM, NaCl 400mM, EDTA 1mM) supplémenté de formamide désionisée (80% (v/v), sauf indication contraire). Après dénaturation 5 min à 70°C, les échantillons sont incubés sur la nuit à 40°C.

Les billes Streptavidine (Dynal) sont lavées puis reconditionnées dans un tampon de fixation (2X= Tris-HCl (pH 7,5) 10 mM, NaCl 2M, EDTA 1 mM). Les échantillons d'hybridation sont amenés à un volume de 200 µl avec de l'eau puis ajustés à 200 µl de billes pour une incubation de 60 min à 30°C. Après capture sur aimant et lavages des billes, celles-ci sont reprises dans 150 µl de tampon RnaseH puis incubées pendant 20 min à 37°C. Après capture sur aimant, les régions non hybridées ont été relarguées dans le surnageant qui est traité à la Dnase puis extrait au phénol acide / chloroforme puis précipité à l'éthanol. Les précipitations à l'éthanol de faibles quantités d'acides nucléiques sont effectuées à l'aide d'un polymère commercial SeeDNA (Amersham Pharmacia Biotech) permettant de récupérer de façon quantitative des acides nucléiques à partir de solutions très diluées (de l'ordre du ng/ml).

La synthèse d'ADNc à partir des échantillons d'ARNs provenant de l'action de la RnaseH est effectuée à partir d'hexanucléotides aléatoires à l'aide de Superscript II Reverse Transcriptase. L'ARN est ensuite dégradé à l'aide d'un mélange de RnaseH et de Rnase T1. L'amorce, les nucléotides non incorporés et les enzymes sont séparés de l'ADNc à l'aide d'une cartouche " GlassMAX Spin ". L'ADNc correspondant aux boucles d'épissage est ensuite soumis à une réaction de PCR en utilisant des oligonucléotides de type semi-aléatoire déjà décrits plus haut dans l'invention. En l'occurrence les oligonucléotides choisis sont :

La réaction de PCR est réalisée à l'aide de Taq Polymérase sur 30 cycles :
- Dénaturation initiale : 94°C pdt 1 min.
- 94°C pdt 30 s
- 55°C pdt 30s
- 72°C pdt 30s
- Extension finale : 72°C pdt 5 min.

Les produits de PCR ont été clonés dans le vecteur pGEM-T (Proméga) possédant un T flottant aux extrémités 3' afin de faciliter le clonage de fragments issus de l'activité de la Taq Polymérase. Après transformation dans les bactéries JM109 compétentes (Proméga), les colonies obtenues sont repiquées sur filtre de nitrocellulose, et hybridées avec des sondes dérivées de produits de PCR effectuées sur des ADNc totaux des cellules en prolifération d'une part et en anoïkis d'autre part. Pour ces PCR les mêmes oligonucléotides GAGAAGCGTTATNNNNNCCA sont utilisés. Dans une première réalisation expérimentale, 34 clones hybridant préférentiellement avec la sonde des cellules en apoptose et 13 clones hybridant préférentiellement avec la sonde des cellules en prolifération ont été isolés.

Parmi ces 13 clones, 3 clones contiennent le même fragment d'ADNc qui dérive du domaine SH2 de la protéine SHC.

La séquence de ce fragment est la suivante :

L'utilisation d'amorces de PCR qui encadrent le domaine SH2 de SHC (oligo5' : GGGACCTGTTTGACATGAAGCCC (SEQ ID NO:6) ; oligo3': CAGTTTCCGCTCCACAGGTTGC (SEQ ID NO:7)) a permis de caractériser la délétion du domaine SH2 de SHC qui est observée spécifiquement dans les cellules ECV en anoïkis. Avec ce couple d'amorce, un seul produit d'amplification correspondant à un fragment d'ADNc de 382 paires de bases qui contient le domaine SH2 intègre est obtenu à partir d'ARN de cellules ECV en phase exponentielle. Un fragment additionnel de 287 paires de bases est observé lorsque la PCR est réalisée à partir d'ARN de cellules en anoïkis. Ce fragment supplémentaire dérive d'un ARN messager dérivé du messager de SHC mais présentant une délétion.

La séquence de cette délétion est la suivante :

Cette délétion correspond aux bases 1198 à 1293 de la phase ouverte du messager codant pour les formes de 52kDa et 46kDa de la protéine SHC (Pelicci, G. et al, 1992, Cell, 70, pp93-104).

Les données structurales des domaines SH2 ainsi que la littérature indiquent qu'une telle délétion aboutit à la perte de l'affinité pour les phosphotyrosines puisqu'elle englobe les acides aminés impliqués dans les interactions avec les tyrosine phosphorylées (Waksman, G. et al, 1992, Nature, vol358, pp646-653). Les protéines SHC étant des adapteurs qui connectent différents partenaires par leurs domaines SH2 et PTB (PhosphoTyrosine Binding domain), cette délétion génère donc un dominant négatif naturel de SHC que nous appelons ΔSHC. Les domaines SH2 des protéines dont les gènes sont séquencés étant portés par deux exons, il est vraisemblable que la délétion identifiée par la méthode DATAS correspond à un exon alternatif du gène SHC.

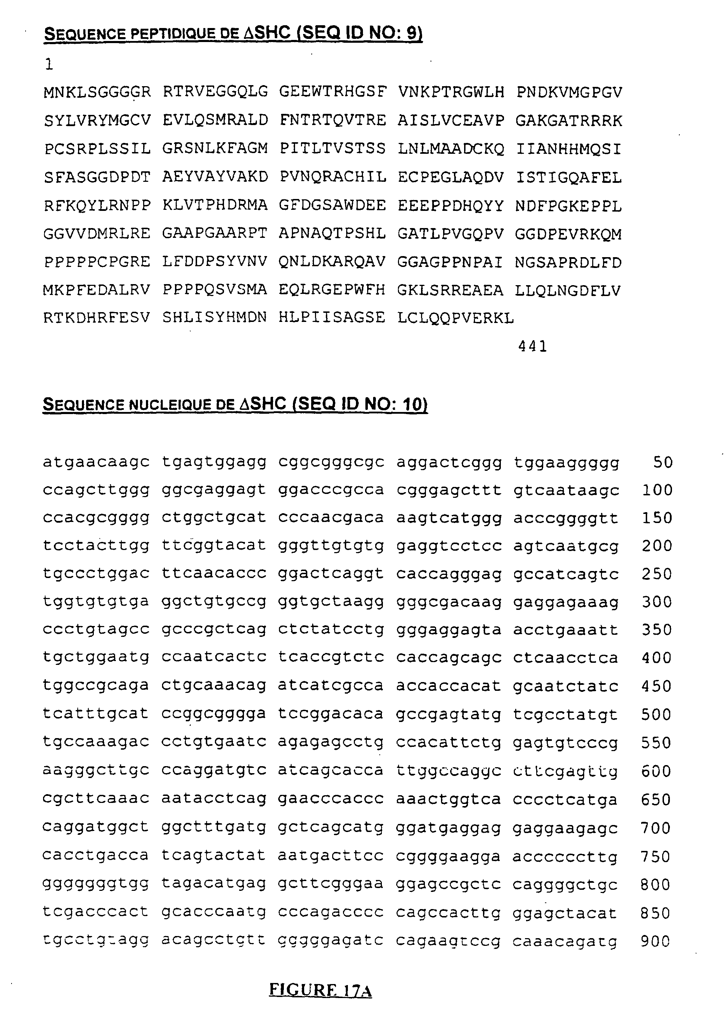

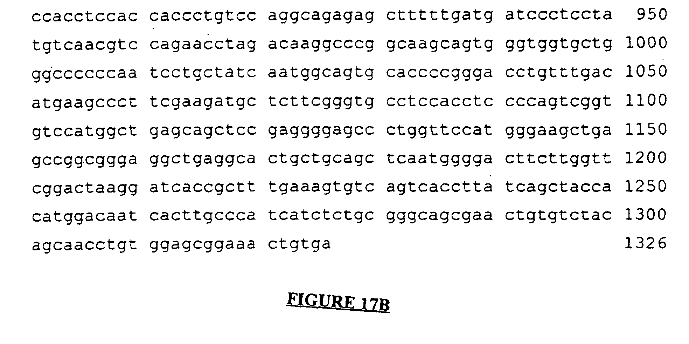

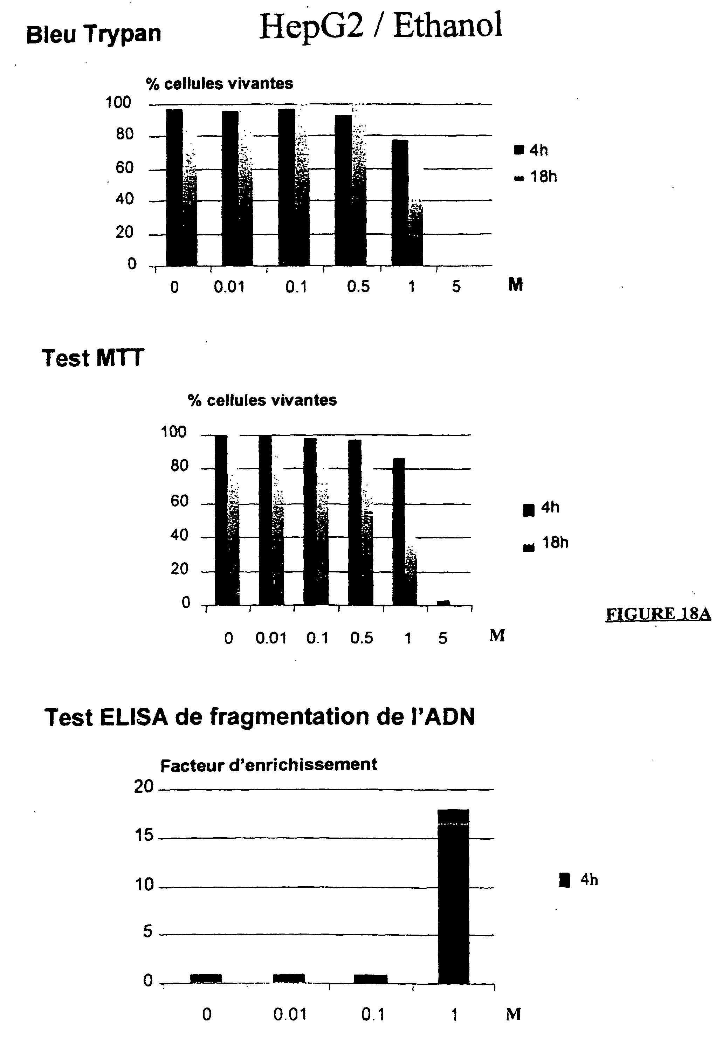

Les séquences protéique et nucléique de ΔSHC sont les représentées sur la Figure 17 (SEQ ID NO: 9 et 10).

Le domaine SH2 de SHC étant impliqué dans la transduction de nombreux signaux impliqués dans la prolifération et la viabilité cellulaires, l'examen de la séquence de ΔSHC permet d'anticiper ses propriétés de dominant négatif sur la protéine SHC et sa capacité d'interférer avec différent signaux cellulaires.

L'invention concerne également cette nouvelle forme épissée de SHC, le domaine protéique correspondant à l'épissage, tout anticorps ou sonde nucléique permettant sa détection dans un échantillon biologique, et leurs utilisation diagnostique ou thérapeutique, par exemple.

L'invention concerne en particulier tout variant de SHC comprenant au moins une délétion correspondant aux bases 1198 à 1293, plus particulièrement une délétion de la séquence SEQ ID NO: 8. L'invention concerne plus spécifiquement le variant ΔSHC ayant la séquence SEQ ID NO: 9, codé par la séquence SEQ ID NO: 10.

L'invention concerne aussi toute sonde nucléique, oligonucléotide ou anticorps permettant d'identifier le variant ΔSHC ci-dessus, et/ou toute altération du rapport SHC/ΔSHC dans un échantillon biologique. Il peut s'agir notamment d'une sonde ou oligonucléotide complémentaire de tout ou partie de la séquence SEQ ID NO: 8, ou d'un anticorps dirigé contre le domaine protéique codé par cette séquence. De telles sondes, oligonucléotides ou anticorps permettent de détecter la présence de la forme non épissée (e.g., SHC) dans un échantillon biologique.

Les matériels peuvent en outre être utilisés en parallèle avec des sondes, oligonucléotides et/ou anticorps spécifiques de la forme épissée (e.g., ΔSHC), c'est-à-dire correspondant par exemple à la région de jonction résultant de l'épissage (localisée autour du nucléotide 1198 de la séquence SEQ ID NO: 10).

De tels matériels peuvent être utilisés pour le diagnostic de pathologies liées à une immunodépression (cancer, traitement immunosuppresseur, SIDA, etc.).

L'invention concerne aussi tout procédé de criblage de molécules basé sur le blocage (i) du domaine épissé dans la protéine SHC (notamment pour induire un état de tolérance immunitaire par exemple dans les maladies autoimmunes ou les rejets de greffes et les cancers) ou (ii) des gains de fonction acquis par la protéine ΔSHC.

L'invention concerne en outre l'utilisation thérapeutique de ΔSHC, et notamment pour le traitement de cellules cancéreuses ou de cancers (ex vivo ou in vivo) dans lesquels une hyperphosphorylation de la protéine SHC peut être mise en évidence, par exemple. A cet égard, l'invention concerne aussi tout vecteur, notamment viral, comprenant une séquence codant pour ΔSHC. Il s'agit préférentiellement d'un vecteur capable de transfecter des cellules cancéreuses ou en prolifération, telles que des cellules musculaires lisses, des cellules endothéliales (resténose), des fibroblastes (fibroses), de préférence d'origine mammifère, notamment humaine. Comme vecteur viral, on peut citer notamment des vecteurs adénoviraux, rétroviraux, AAV, herpès, etc.

### 2. CLONAGE DIFFÉRENTIEL DES EPISSAGES ALTERNATIFS ET AUTRES MODIFICATIONS QUALITATIVES DES ARNS EN UTILISANT DES ADNc DOUBLE-BRINS (FIGURE 5).

Les ARNs messagers correspondants aux situations normales (mN) et pathologiques (mP) sont produits, ainsi que les ADNs complémentaires double brin correspondants (dsN et dsP) par des protocoles classiques de biologie moléculaire. Des structures de type "R-loop" sont alors obtenues en hybridant mN avec dsP et mP avec dsN dans une solution contenant 70% de formamide. Les domaines nucléiques différentiellement épissés entre la situation N et P resteront sous forme d'ADN double brin. Les simples brins d'ADN déplacés sont alors traités au glyoxal afin d'éviter le redéplacement du brin d'ARN lors du retrait de la formamide. Après retrait de la formamide et du glyoxal puis traitement à la RNAseH, nous nous retrouvons avec des structures de type abeille, les ADNs simples brin non appariés représentant les ailes de l'abeille et le domaine double brin appareillé d'intérêt représentant le corps de l'abeille. L'utilisation d'enzymes qui dégradent spécifiquement l'ADN simple brin comme la nucléase S1 ou la Mung Bean nucléase permet l'isolation de l'ADN resté sous forme double brin qui est ensuite cloné puis séquencé. Cette deuxième technique permet l'obtention directe d'une empreinte ADN double brin du domaine d'intérêt comparativement au premier protocole qui produit une empreinte ARN de ce domaine.

Cette approche a été réalisée sur le modèle grb2/grb33 décrit précédemment. L'ADN double brin de grb2 a été produit par amplification PCR à partir de l'ADNc simple brin de grb2 et de deux amorces nucléotidiques correspondant a la séquence (1-22) de grb2 et à la séquence complémentaire de (618-639) de grb2. Ce fragment PCR a été purifié sur gel d'agarose, nettoyé sur colonne d'affinité (JetQuick, Genomed) et quantifié par spectrophotométrie. Dans le même temps, deux ARNs synthétiques correspondant aux phases de lecture de grb2 et de grb33 ont été produits à partir de vecteurs plasmidiques comportant les cDNAs de grb2 ou de grb33 sous contrôle du promoteur T7, à l'aide du kit RiboMax (Promega). Les ARNs ont été purifiés selon les instructions du fournisseur et nettoyés sur colomne d'exclusion (Sephadex G50, 5 prime-3 prime). 600 ng de l'ADN double brin de grb2 (1-639) ont été associés avec :
1. 3 µg d'ARN de grb33
2. 3 µg d'ARN de grb2
3. de l'eau
dans trois réactions différentes, dans le tampon suivant :
100 mM PIPES (pH 7,2), 35 mM NaCl, 10 mM EDTA, 70% formamide déionisé (Sigma)

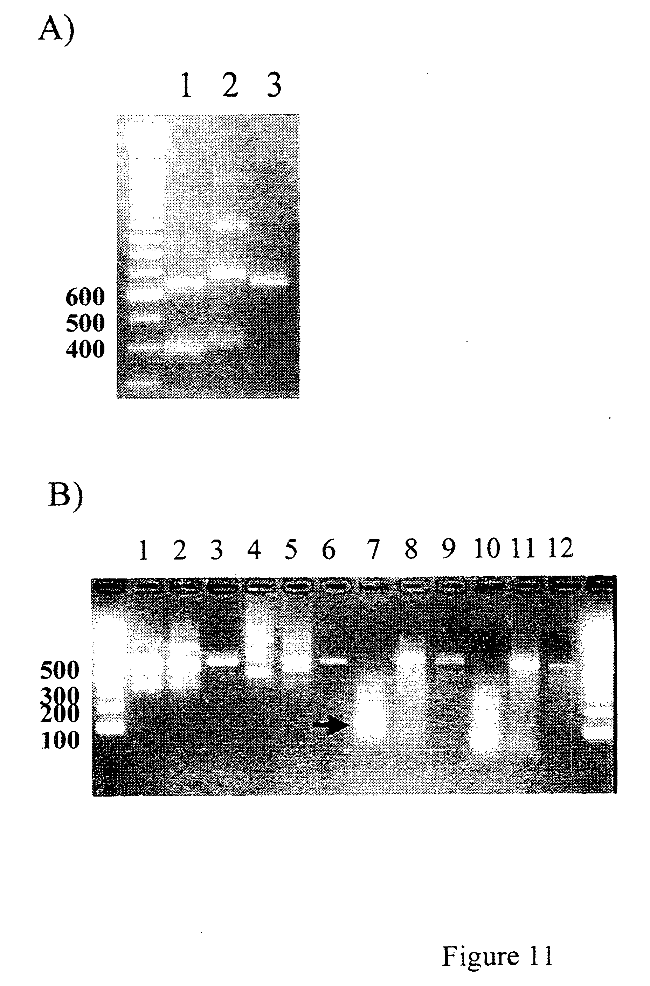

Les échantillons ont été amenés à 56 °C puis refroidis à 44 °C par incrément de -0,2 °C toutes les 10 minutes. Ils sont ensuite conservés à 4 °C. L'analyse sur gel d'agarose révèle des modifications de migrations dans les puits 1 et 2 par rapport au puits 3 contrôle (Figure 11A) indiquant la formation de nouveaux complexes. Les échantillons sont ensuite traités au glyoxal déionisé (Sigma) (5% v/v ou 1M) pendant 2 h à 12 °C. Les complexes sont ensuite précipités à l'éthanol (0,1M NaCl, 2 volumes d'ethanol), lavés à l'ethanol 70%, séchés puis repris dans de l'eau. Ils sont enfin traités par la RnaseH (Life Technologies), puis par une enzyme capable de dégrader spécifiquement l'ADN simple brin. La nuclease S1 et la nucléase " Mung Bean " présentent cette propriété et sont disponibles commercialement (Life Technologies, Amersham). De telles digestions (incubations de 5 minutes dans les tampons fournis avec les enzymes) ont été analysées sur gel d'agarose (figure 11B). Des digestions significatives sont uniquement obtenues à partir des complexes issus de la réaction 1 (grb2/grb33) (figure 11B, puits 7 et 10). Ces digestions semblent plus complètes avec la nucléase S1 (puits 7) qu'avec la nucléase " Mung Bean " (puits 10). Ainsi, la bande correspondant à une taille légèrement supérieure à 100 paires de bases (indiquée par une flèche dans le puits 7) a été purifiée, clonée dans le vecteur pMos-Blue (Amersham), puis séquencée. Ce fragment correspond au domaine de 120 paires de bases de grb2, délété dans grb33.

Cette approche peut maintenant être effectuée à partir d'une population totale d'ARN messager et d'une population totale d'ADNc double brin produite selon les techniques connues de l'homme de métier. La population d'ARN de la situation de référence est hybridée à la population d'ADNc double brin de la situation test et réciproquement. Après application du protocole décrit ci-dessus, les digestions sont déposées sur gel d'agarose afin d'isoler et de purifier les bandes correspondant à des tailles variant entre 50 et 300 paires de bases. Ces bandes sont ensuite clonées dans un vecteur (pMos-Blue, Amersham) pour donner lieu à une banque d'inserts enrichis en des événements de différences qualitatives.

### 3. CONSTRUCTION DE BANQUES ISSUES DE CRIBLAGES DIFFÉRENTIELS QUALITATIFS.

Les deux exemples décrits ci-dessus aboutissent aux clonages d'ADNc représentatifs de toute ou partie des séquences épissées différentiellement entre deux situations physiopathologiques données. Ces ADNc permettent la constitution de banques par insertion de ces ADNc dans des vecteurs plasmidiques ou phagiques. Ces banques peuvent être présentées sur des filtres de nitrocellulose ou tout autre support connu de l'homme de l'art, tels des chips ou biopuces ou membranes. Ces banques peuvent être conservées au froid, à l'abri de la lumière. Ces banques, une fois déposées et fixées sur support par les techniques classiques, peuvent être traitées par des composés pour éliminer les bactéries hôtes qui permettent la production des plasmides ou des phages. Ces banques peuvent également avantageusement être constituées de fragments d'ADNc correspondant aux ADNc clonés mais préparés par PCR de façon à ne déposer sur filtre que les séquences dérivées des événements d'épissages alternatifs.

L'une des caractéristiques et en même temps l'une des originalités du criblage différentiel qualitatif est que cette technique aboutit de façon avantageuse non pas à une mais à deux banques différentielles ("paire de banque") qui représentent l'ensemble des différences qualitatives qui existent entre deux situations données. En particulier, l'une des banques d'épissage différentiel de l'invention représente la signature des qualités de la situation physiologique test par rapport à la situation physiologique de référence, et l'autre banque représente la signature des qualités de la situation physiologique de référence par rapport à la situation physiologique test. Ce couple de banques est également désigné paire de banques ou "banque différentielle d'épissage".

L'un des apports du criblage différentiel qualitatif étant de permettre d'évaluer le potentiel toxique d'un composé, comme cela est indiqué dans le chapitre suivant, un bon exemple de mise en oeuvre de la technologie est l'obtention par DATAS de clones d'ADNc correspondant à des séquences spécifiques de cellules HepG2 naïves, d'une part, et traitées par de l'éthanol, d'autre part. Ces cellules présentent des signes de cytotoxicité et une dégradation de leur ADN par fragmentation internucléosomale à partir de 18h en présence de 1M d'éthanol. De façon à obtenir des marqueurs précoces de la toxicité éthanolique, les ARN messagers ont été préparés à partir de cellules naïves et de cellules traitées pendant 18h par de l'éthanol à la concentration de 0,1M. Après mise en oeuvre de la variante de DATAS qui utilise l'ADNc simple brin et la Rnase H, les ADNc clonés obtenus ont été amplifiés par PCR, soumis à une électrophorèse sur gel d'agarose et ensuite transférés sur un filtre de nylon selon les techniques connues de l'homme de l'art. Pour chaque ensemble de clones spécifiques d'une part des différences qualitatives spécifiques de l'état naïf et d'autre part des séquences spécifiques des cellules traitées par l'éthanol, deux répliques identiques de filtres sont effectuées. Ainsi les empreintes de chaque ensemble de clones sont hybridées d'une part avec une sonde spécifique des cellules non traitées et d'autre part avec une sonde spécifique des cellules traitées par 0.1M d'éthanol pendant 18h.

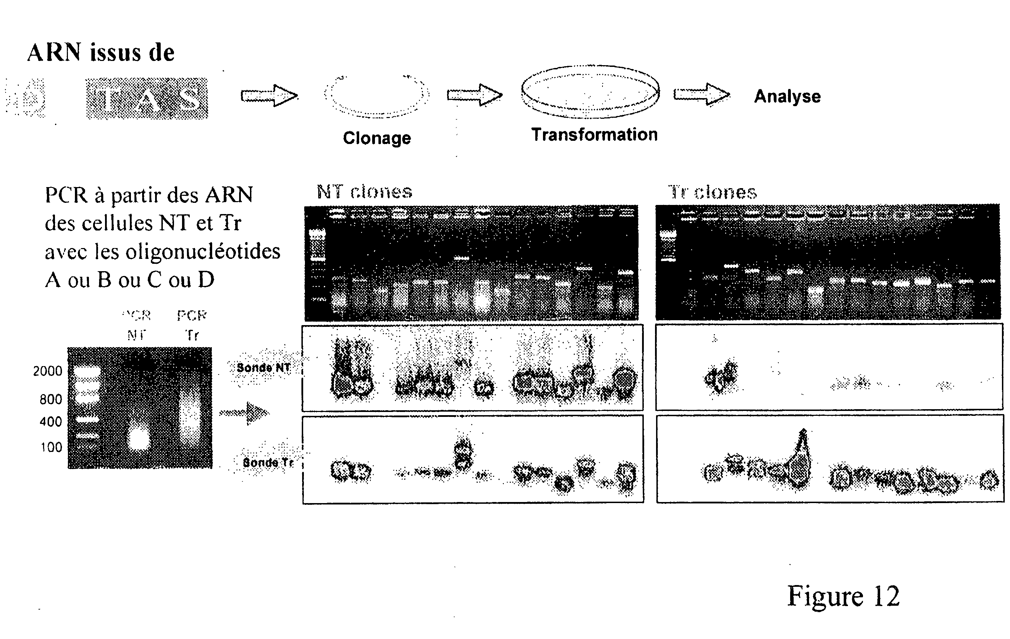

Le profil d'hybridation différentiel obtenu et présenté sur la figure 12 permet d'apprécier la qualité de la soustraction effectuée lors de la mise en oeuvre de la technique DATAS. Ainsi les clones issus de l'hybridation de l'ARNm de cellules non traitées (NT) avec l'ADNc de cellules traitées (Tr) et qui doivent correspondre à des différences qualitatives spécifiques de la situation naïve hybrident préférentiellement avec une sonde représentant la population totale des ARN messagers des cellules non traitées. Réciproquement, les clones issus des produits résistant à la RNase H ayant agi sur les hétéroduplex ARN(Tr)/ADNc(NT) hybrident préférentiellement avec une sonde dérivée de la population totale des ARN messagers des cellules traitées.

Les deux ensembles de clones spécifiques d'une part de la situation traitée et d'autre part de la situation non traitée représentent un exemple de banques de différences qualitatives caractéristiques de deux états cellulaires distincts.

### 4. UTILISATIONS ET APPORTS DES BANQUES DIFFÉRENTIELLES QUALITATIVES.

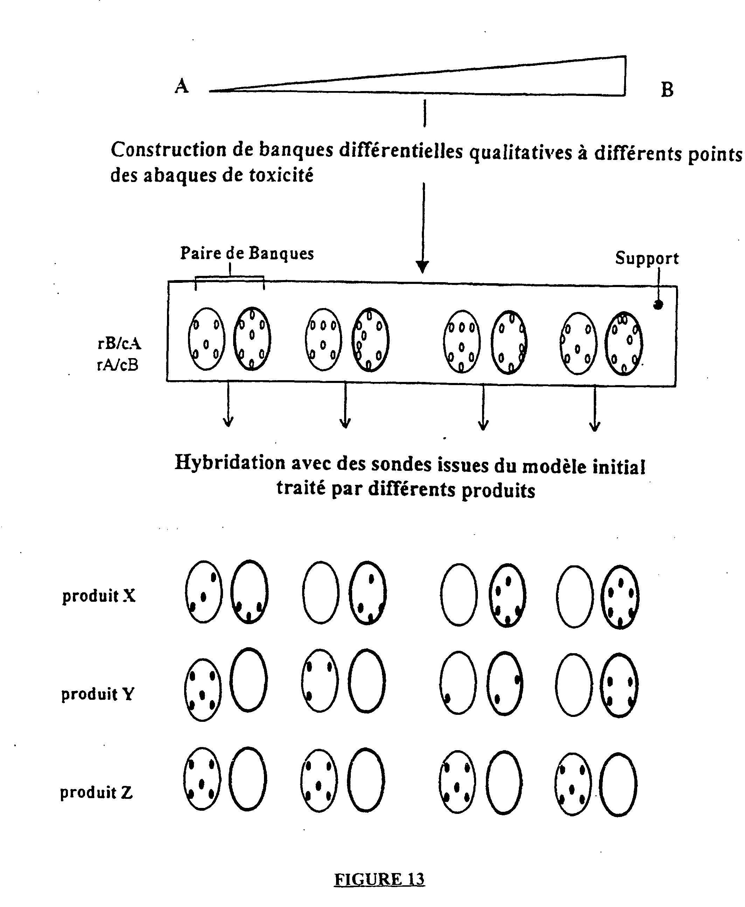

Les possibilités d'utilisation des banques différentielles d'épissage de l'invention sont illustrées notamment sur les Figures 13 à 15. Ainsi, ces banques sont utilisables pour :

### 4.1. L'évaluation du potentiel toxique d'un composé (figure 13) :

Dans cet exemple, la situation de référence est désignée A et la situation toxique est désignée B. Des abaques de toxicité sont obtenues par traitement de la situation A en présence de différentes concentrations d'un composé toxique de référence, pendant des périodes variables. A différents points les abaques de toxicité, des banques différentielles qualitatives sont construites (paires de banque), dans cet exemple, des banques restreintes rA/cB et rB/cA. Les paires de banque sont avantageusement déposées sur un support. Le support est ensuite hybridé avec des sondes issues de l'échantillon biologique initial traité par différentes doses de composés test : Produits X, Y et Z. L'hybridation est révélée et fait apparaître le potentiel toxique des produits test : dans cet exemple, le produit Z présente une forte toxicité et le produit Y offre un profil intermédiaire. La faisabilité de cette constitution d'abaques de toxicité est bien illustrée par l'exemple de constitution de banques de criblage différentiel qualitatif décrit ci-avant et mettant en jeu l'éthanol et des cellules HepG2.

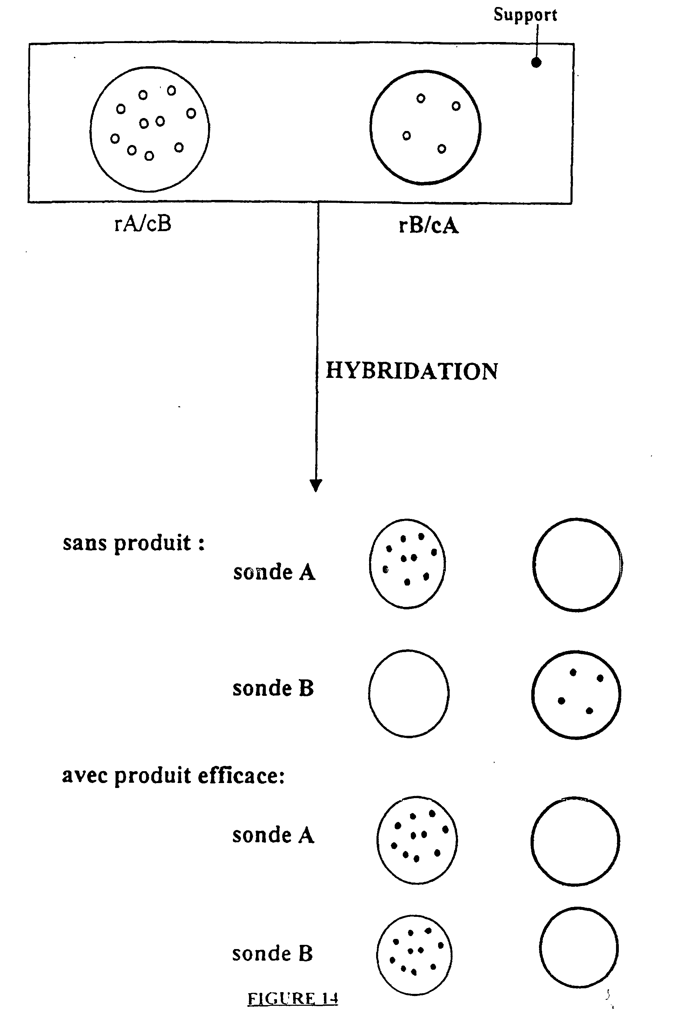

### 4.2. L'évaluation de l'efficacité d'une composition pharmaceutique (figure 14) :

Dans cet exemple, une paire de banques restreintes selon l'invention est réalisée à partir d'un modèle pathologique B et d'un modèle sain A (ou du modèle pathologique traité avec un produit actif de référence). Les banques différentielles rA/cB et rB/cA sont, le cas échéant, déposées sur un support. Cette paire de banque regroupe les différences d'épissage entre les deux situations. Cette paire de banque permet d'évaluer l'efficacité d'un composé test, c'est-à-dire de déterminer sa capacité à générer un profil de type "sain" (rA/cB) à partir du profil de type pathologique (rB/cA). Dans cet exemple, la paire de banque est hybridée avec des sondes préparées à partir des situations A et B avec ou sans traitement par le composé test. Le profil d'hybridation qui peut être obtenu est présenté sur la figure 14. La faisabilité de cette application est la même que celle de la constitution de banques de différences qualitatives caractéristiques de situations saines et toxiques présentée plus-haut. La situation toxique est remplacée par l'état pathologique et il est possible d'apprécier la capacité d'un composé test à produire une sonde hybridant avec plus ou moins de préférence avec les situations de référence ou pathologique.

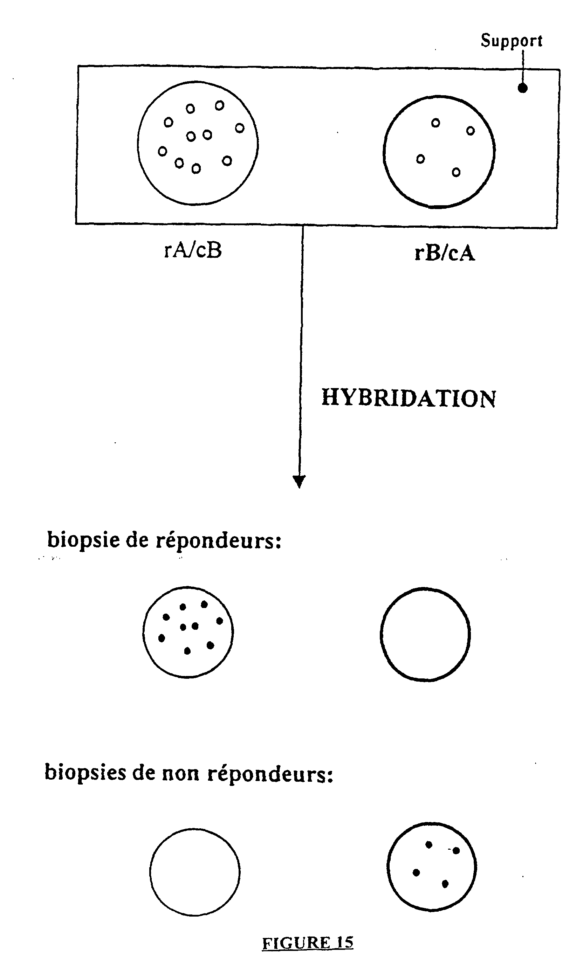

### 4.3. L'anticipation de la réponse d'un échantillon pathologique à un traitement (figure 15) :

Dans cet exemple, une paire de banque restreinte selon l'invention est réalisée à partir de deux modèles pathologiques, dont l'un répond à un traitement par un produit donné (le gène p53 sauvage par exemple) : situation A ; et l'autre y est réfractaire : situation B. Cette paire de banque (rA/cB ; rB/cA) est déposée sur un support.

Cette paire de banque est ensuite utilisée pour déterminer la sensibilité d'un échantillon pathologique test à ce même produit. Pour cela, cette paire de banque est hybridée avec des sondes provenant de biopsies de patients dont on souhaite anticiper la réponse au traitement de référence. Le profil d'hybridation d'une biopsie de répondeur et d'une biopsie de non-répondeur est présenté sur la figure 15.

### 4.4 L'identification de ligands pour des récepteurs orphelins

L'activation de récepteurs membranaires ou nucléaires par leurs ligands pourrait induire spécifiquement des dysrégulations dans l'épissage de certains ARNs. L'identification de ces événements par les méthodes DATAS de l'invention permet de disposer d'un outil (marqueurs, banques, kits, etc.) de suivi d'activation de récepteurs, utilisables pour la recherche de ligands naturels ou synthétiques de récepteurs, en particulier orphelins. Selon cette application, des marqueurs associés aux dysrégulations sont identifiés et déposés sur des supports. L'ARN total de cellules, (sur)exprimant le récepteur à l'étude, traitées ou non par différentes compositions et/ou composés tests, est extrait et utilisé comme sonde dans une hybridation avec les supports. La mise en évidence d'une hybridation avec certains, voire la totalité des marqueurs déposés sur le support, indique que le récepteur à l'étude a été activé, et donc que le composition/composé correspondant constitue ou comprend un ligand dudit récepteur.

### 4.5 L'identification de cibles d'intérêt thérapeutique :

Celle-ci se fait par identification de gènes dont l'épissage est modifié dans une pathologie ou dans un modèle de pathologie et plus précisément l'identification des exons ou introns modifiés. Cette approche doit permettre de donner accès aux séquences qui codent les domaines fonctionnels altérés lors de pathologies ou de tout phénomène physiopathologique adressant les phénomènes de prolifération, différenciation ou apoptose par exemple.

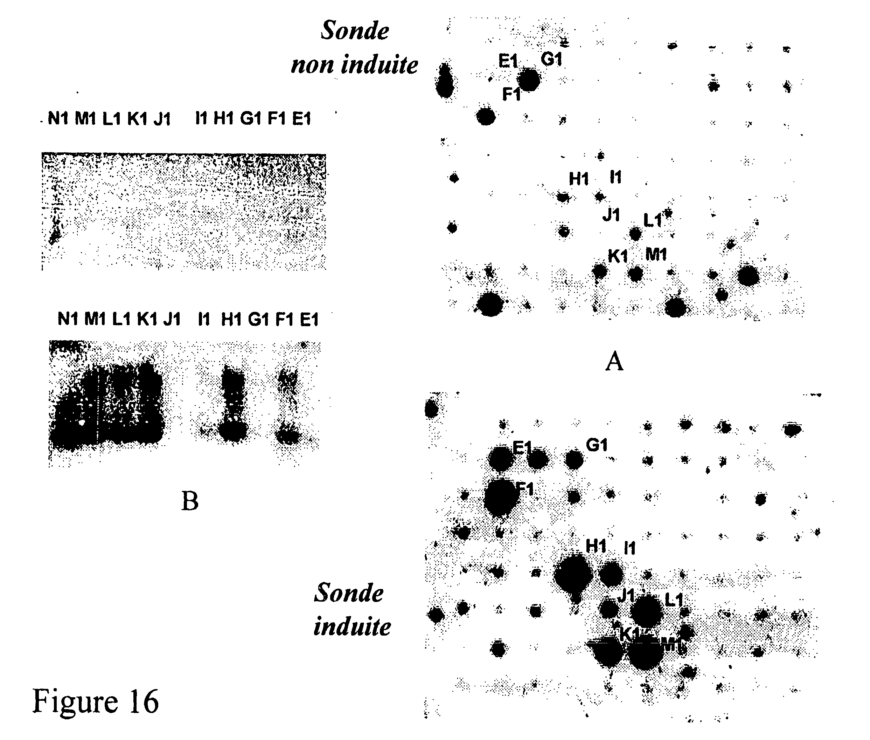

Un exemple de l'apport du criblage différentiel qualitatif à l'identification de gènes différentiellement épissés est fourni par l'application de DATAS à un modèle d'induction d'apoptose par induction de l'expression de la forme sauvage de p53. Ce modèle cellulaire a été établi par transfection d'un système d'expression inductible pour le gène suppresseur de tumeur p53. De façon à identifier les différences qualitatives qui sont associés spécifiquement à l'apoptose induite par p53, DATAS a été mise en oeuvre à partir des ARN messagers extraits de cellules induites et non induites. Pour ces expériences 200ng d'ARN polyA⁺ et 200ng d'ADNc ont été utilisés lors de la formation des hétéroduplex. Une centaine de clones a été obtenue à partir de chacune des hybridations croisées. L'hybridation de ces clones bactériens puis des fragments d'ADNc qu'ils contiennent avec des sondes représentatives des ARN messagers totaux des situations de départ a permis l'identification de séquences spécifiquement exprimées lors de la forte induction de p53 qui aboutit à la mort cellulaire (figure 16).

Ces fragments dérivent de séquences exoniques ou introniques qui modulent la qualité du message présent et permettent de proposer les domaines fonctionnels auxquels ils participent ou qu'ils interrompent comme des cibles d'intervention pour induire ou inhiber la mort cellulaire.

Une telle approche aboutit également à la constitution d'une paire de banques qui rassemblent des événements différentiellement épissés entre une situation non apoptotique et une situation apoptotique. Cette paire de banque peut être utilisée pour tester le pouvoir hybridant d'une sonde dérivée d'une autre situation physiopathologique ou d'un traitement particulier. Le résultat d'une telle hybridation donnera des indications sur l'engagement éventuel du programme d'expression génétique de la situation testée vers l'apoptose.

Comme il ressort de la description ci-avant, l'invention concerne également :
- toute sonde nucléique, tout oligonucléotide, tout anticorps dirigé contre une séquence identifiée par la technique décrite dans la présente demande et caractérisés en ce qu'ils permettent de caractériser une situation pathologique,
- l'utilisation des informations issues de l'utilisation des techniques décrites pour la recherche de molécules organiques à visée thérapeutique par la mise en place de criblages caractérisés en ce qu'ils ciblent des domaines splicés différentiellement entre une situation saine et une situation pathologique ou bien caractérisés en ce qu'ils sont basés sur l'inhibition des gains de fonctions acquis par la protéine résultant d'un épissage différentiel,
- l'utilisation des informations issues des techniques décrites dans la présente demande pour des applications de thérapie génique,
- l'utilisation d'ADNc transférés par thérapie génique caractérisés en ce qu'ils ont des propriétés antagonistes ou agonistes sur des voies de signalisation cellulaires définies,
- toute constitution et toute utilisation de banques moléculaires d'exons ou d'introns alternatifs à des fins:
   . de diagnostic ou de réactifs commerciaux pour la recherche
   . de créer ou de rechercher des molécules, polypeptides, acides nucléiques pour application thérapeutique.
- toute constitution et toute utilisation de banques virtuelles informatiques regroupant des exons ou introns alternatifs caractérisés en ce que ces banques permettent de concevoir des sondes nucléiques ou des amorces oligonucléotidiques dans le but de caractériser les épissages alternatifs qui différentient deux états physiopathologiques distincts.
- toute composition pharmaceutique ou diagnostique comprenant des polypeptides, acides nucléiques sens ou anti-sens, ou des molécules chimiques capables d'interférer avec les produits d'épissage alternatifs mis en évidence et clonés par les techniques de l'invention,
toute composition pharmaceutique ou diagnostique comprenant des polypeptides, acides nucléiques sens ou anti-sens, ou des molécules chimiques capables de restaurer un épissage représentatifs d'une situation normale par opposition à l'événement alternatif caractéristique d'une situation pathologique.

### 5. DEREGULATIONS DES MÉCANISMES D'EPISSAGE DES ARNS PAR DES AGENTS TOXIQUES.

Cet exemple montre que les différences de formes et/ou profils d'épissages peut être utilisée comme marqueur pour le suivi et/ou la détection de toxicité et/ou d'efficacité de composés.

Les effets d'agents toxiques sur les dérégulations d'épissages des ARNs a été testé de la manière suivante. Des cellules hépatocytaires, HepG2, ont été traitées par différentes doses de trois composés toxiques (ethanol, camptothecine, PMA (Phorbol 12-Myristate 13-Acetate)). Deux tests de cytotoxicité (Bleu de Trypan, MTT) ont été réalisés à différents temps : 4h et 18h pour l'ethanol ; 4h et 18h pour la camptothecine ; 18h et 40h pour le PMA.

Le Bleu de Trypan est un colorant qui peut être incorporé par les cellules vivantes. Un simple comptage des cellules "bleues" et "blanches" sous microscope permet de déterminer le pourcentage de cellules vivantes après traitement ou pourcentage de survie. Les points sont effectués en triplicates.

Le test MTT est un test colorimétrique qui mesure la capacité des cellules vivantes à convertir les sels solubles de tetrazolium (MTT) en un précipité insoluble de formazan. Ces cristaux de formazan, bleu foncé, peuvent être dissous et leur concentration déterminée par mesure d'absorbance à 550 nm. Ainsi, après ensemencement de plaques 24 puits par 150000 cellules sur la nuit, puis traitement des cellules par les composés toxiques, est ajouté 50 µl de MTT (Sigma) (à 5 mg/ml dans du PBS). La réaction de formation des cristaux de formazan s'effectue en 5 h dans l'incubateur à CO2 (37°C, 5% CO2, 95% humidité). Après addition de 500 µl de solution de solubilisation (Hcl O,1N dans Isopropanol-Triton X-100 (10%)), les cristaux sont dissous sous agitation et les absorbances mesurées à 550 à 660nm. Les points sont effectués en triplicates avec les contrôles (viabilité, mort cellulaire, blancs) appropriés.

Un test d'apoptose ou mort cellulaire programmée a également été réalisé par mesure de la fragmentation d'ADN via l'utilisation d'anticorps anti-histone et de mesures d'ELISA. Le test utilisé est Cell Death ELISA Plus de Roche.

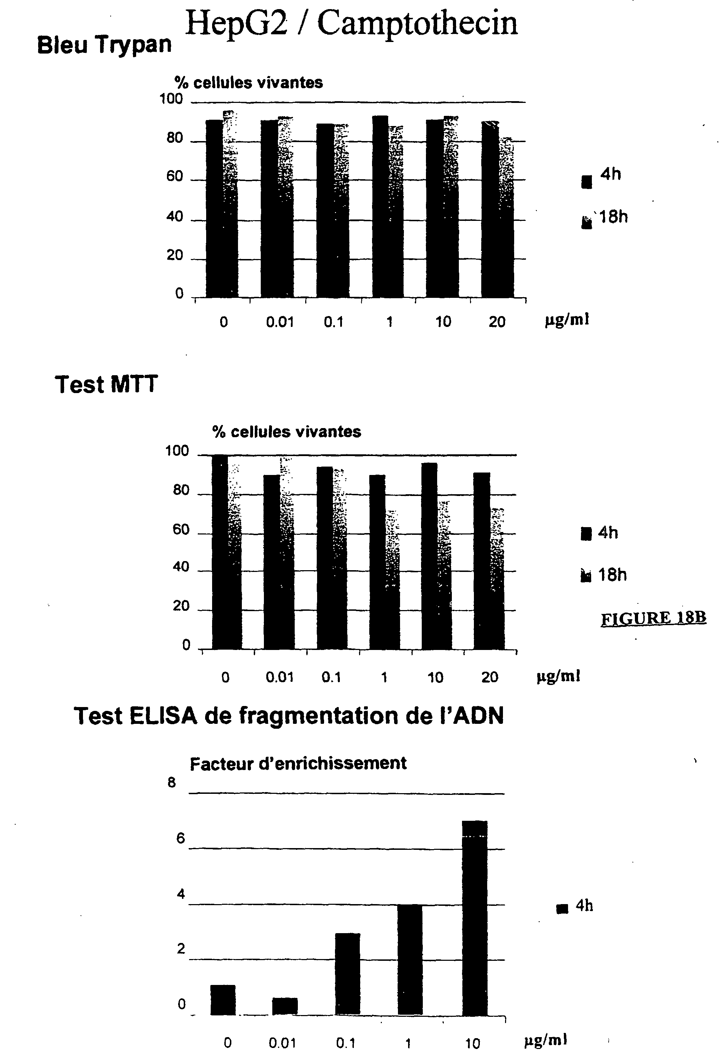

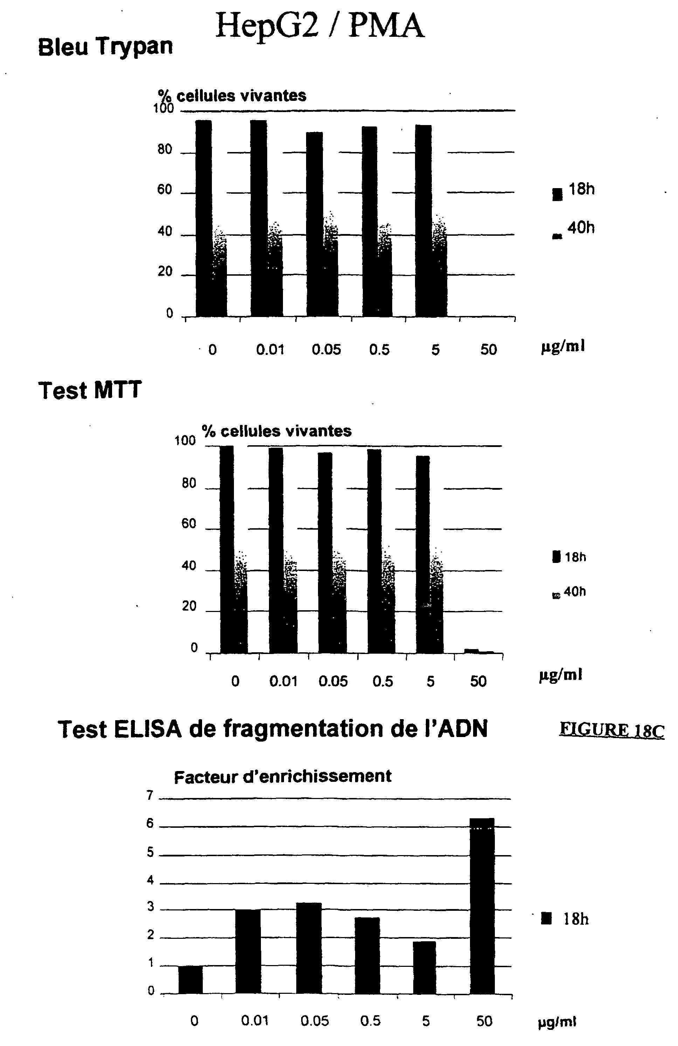

Les résultats de ces trois tests (Figures 18 A,B,C) ont permis de déterminer que les doses suivantes :
- ethanol : 0,1 M
- camptothecine : 1 µg/ml
- PMA : 50 ng/ml
étaient bien inférieures aux IC50s mesurées.

Les cellules HepG2 ont ainsi été traitées par ces trois composés à ces trois doses pendant 4 h pour l'ethanol et la camptothecine et 18 h pour le PMA. Les ARNs messagers ont été purifiés par billes Dynal-Oligo-(dT) à partir d'ARNs totaux purifiés selon le kit Rneasy (Quiagen). Des synthèses d'ADNc ont été effectuées à partir de ces ARNs messagers et de la Transcriptase Inverse Superscript (Life Technologies) en utilisant des hexamères aléatoires comme amorces.

Ces premiers brins ont servi de matrices à des réactions d'amplification par PCR (94°C 1mn, 55°C 1mn, 72°C 1mn, 30 cycles) à l'aide des amorces oligonucléotidiques suivantes :
MACH-α:

Ces amorces correspondent à des régions communes aux différentes isoformes décrites de MACH-α (1,2 et 3, amplifiant respectivement 595, 550 et 343 paires de bases). MACH-α (Caspase-8) est une protéase impliquée dans la mort cellulaire programmée (Boldin et.al., Cell (1996), 85, 803-815).
BCL-X :

Ces amorces correspondent à des régions communes aux différentes isoformes décrites de bcl-X (bcl-Xl, bcl-Xs, BCL-Xβ) (Boise et al., Cell (1993) 74, 597-608; U72398 (Genbank)) et doivent amplifier un fragment unique pour ces trois isoformes de 204 paires de bases.
FASR:

Ces amorces correspondent à des régions communes à certaines isoformes de FASR et doivent amplifier un fragment de 478 paires de bases pour la forme sauvage de FasR, 452 pour l'isoforme Δ8 et 415 pour l'isoforme ΔTM.

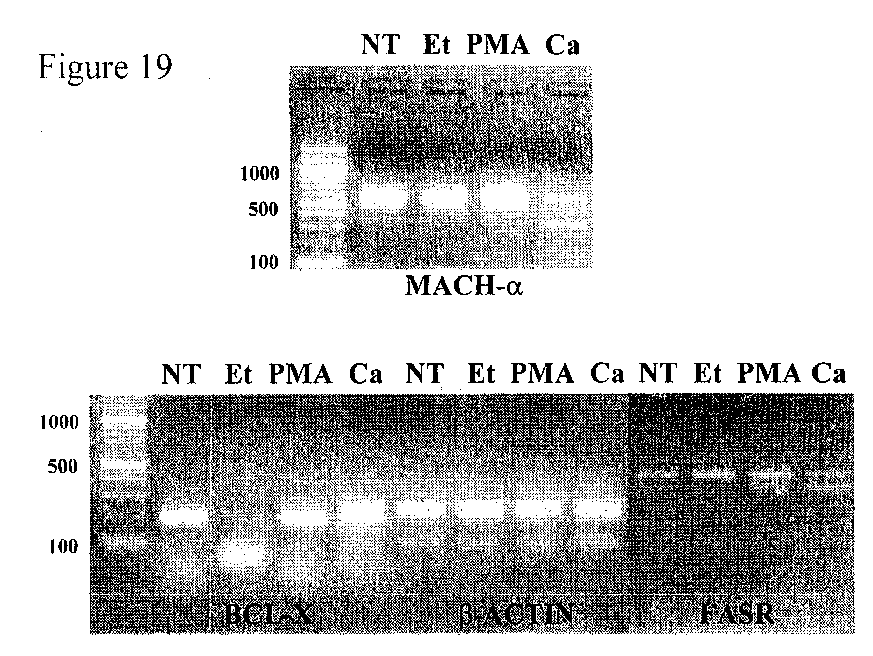

Les résultats rapportés sur la figure 19 indiquent que :
- la camptothécine induit une diminution de l'expression de l'isoforme MACH-α1 et une augmentation de l'isoforme MACH-α3.
- La camptothécine induit l'apparition d'une nouvelle isoforme de bcl-X (bande supérieure du doublet migrant vers 200 paires de bases).
- La camptothécine induit une diminution de la forme sauvage du récepteur de fas, remplacé par une expression d'une isoforme plus courte pouvant correspondre à Fas ΔTM.
- L'éthanol induit la disparition de bcl-x, remplacé par une isoforme plus courte.
- L'éthanol induit une augmentation de la forme longue, sauvage, du récepteur de fas, ceci aux dépens de l'isoforme plus courte.

Ces résultats démontrent que des traitements par des agents toxiques à des doses faibles peuvent induire des dysrégulations d'épissages alternatifs de certains ARNs, ceci de façon spécifique. L'identification de ces dysrégulations au niveau post-transcriptionnel, notamment par l'application de la technologie DATAS permet ainsi de définir un outil prédictif de la toxicité de molécules.

## Revendications

1. Procédé d'identification et/ou de clonage de régions d'acides nucléiques représentatives de différences génétiques qualitatives entre deux échantillons biologiques, comprenant une étape d'hybridation entre les ARN ou d'ADNc double-brin provenant d'un premier échantillon biologique et les ADNc provenant d'un deuxième échantillon biologique et, l'identification et/ou le clonage, à partir des complexes formés par hybridation, de régions d'acides nucléiques non-appariées, lesdites régions comprenant des acides nucléiques représentatifs de différences génétiques qualitatives entre les deux échantillons.

2. Procédé selon la revendication 1 **caractérisé en ce qu'**il comprend :
(a) l'hybridation des ARN provenant du premier échantillon (situation test) avec les ADNc provenant du deuxième échantillon (situation de référence);
(b) l'hybridation des ARN provenant du deuxième échantillon (situation de référence) avec les ADNc provenant du premier échantillon (situation test); et
(c) l'identification et/ou le clonage, à partir des hybrides formés en (a) et (b), d'acides nucléiques correspondant à des différences génétiques qualitatives.

3. Procédé selon la revendication 1 ou 2 **caractérisé en ce que** les hybridations sont réalisées entre des ARN et des ADNc simple-brins et **en ce qu'**il comprend l'identification et/ou le clonage de régions d'ARN non appariées.

4. Procédé selon la revendication 1 **caractérisé en ce qu'**il comprend l'hybridation entre des ADNc double-brin provenant d'un premier échantillon biologique et des ADNc simple-brin provenant d'un deuxième échantillon biologique.

5. Procédé selon les revendications 3 ou 4 **caractérisé en ce que** le clonage des acides nucléiques comprend la transcription inverse et/ou l'amplification au moyen d'amorces aléatoires ou semi-aléatoires, en particulier d'amorces de séquence GAGAAGCGTTATNNNNNNNWXYZ dans laquelle N indique que chacune des quatre bases peut être présente de façon aléatoire à la position indiquée, W, X et Y désignent chacun une base déterminée, et Z désigne soit une base déterminée, soit un groupe 3'-OH.

6. Procédé selon l'une des revendications 1 à 5 **caractérisé en ce que** l'échantillon biologique est composé de cellules, d'un tissu, d'un organe ou d'une biopsie.

7. Procédé selon l'une des revendications 1 à 6 pour l'identification et/ou le clonage d'épissages alternatifs différentiels entre des cellules tumorales et des cellules non-tumorales.

8. Procédé selon l'une des revendications 1 à 6 pour l'identification et/ou le clonage d'épissages alternatifs différentiels entre des cellules traitées par un composé test et des cellules non-traitées.

9. Procédé selon l'une des revendications 1 à 6 pour l'identification et/ou le clonage d'épissages alternatifs différentiels entre des cellules en apoptose et des cellules non-apoptotiques.

10. Procédé selon l'une des revendications 1 à 9 **caractérisé en ce que** l'hybridation est réalisée en phase liquide.

11. Procédé d'identification et/ou de clonage de régions d'acides nucléiques épissées différentiellement entre deux situations physiologiques A et B, comprenant :
(a) la formation d'hétéroduplex en phase liquide entre les ARN messagers provenant de la situation A et les ADNc provenant de la situation B d'une part;
(b) la formation d'hétéroduplex en phase liquide entre les ARN messagers provenant de la situation B et les ADNc provenant de la situation A d'autre part; et
(c) l'identification et/ou le clonage des régions d'ARN non appariées dans les hétéroduplex obtenus en (a) et en (b).

12. Procédé d'identification et/ou de clonage de régions d'acides nucléiques épissées différentiellement entre deux situations physiologiques A et B, comprenant :
(a) la formation d'hétéroduplex entre les ARN messagers provenant de la situation A et les ADNc provenant de la situation B d'une part, les ARN ou les ADNc étant immobilisés sur un support ;
(b) la formation d'hétéroduplex entre les ARN messagers provenant de la situation B et les ADNc provenant de la situation A d'autre part, les ARN ou les ADNc étant immobilisés sur un support ; et
(c) l'identification et/ou le clonage des régions d'ARN non appariées dans les hétéroduplex obtenus en (a) et en (b).

13. Procédé de préparation d'une composition comprenant un support sur lequel sont présentés des acides nucléiques, comprenant :
- la préparation d'acides nucléiques distincts comprenant une séquence complémentaire et spécifique d'exons ou d'introns alternatifs distincts de gènes ou d'ARNm caractéristiques d'une situation physiologique, lesdits exons ou introns étant identifiables notamment selon le procédé de l'une quelconque des revendications 1 à 12, et
- la présentation de ces acides nucléiques sur un support.

14. Procédé selon la revendication 13, **caractérisé en ce que** les acides nucléiques comprennent des ADNc.

15. Procédé selon la revendication 13, **caractérisé en ce que** les acides nucléiques comprennent des oligonucléotides.

16. Procédé selon la revendication 15, **caractérisé en ce que** les acides nucléiques comprennent des oligonucléotides spécifiques de régions de jonction entre deux domaines séparés par un domaine épissé.

17. Procédé selon la revendication 16, **caractérisé en ce que** les oligonucléotides sont simple-brin et possèdent une longueur comprise entre 5 et 100-mères.

18. Procédé selon la revendication 15, 16 ou 17, **caractérisé en ce que** lesdits oligonucléotides sont obtenus par un procédé comprenant :
(a) l'identification d'un événement d'épissage caractéristique d'une situation physiopathologique et de la séquence du domaine épissé ;
(b) la synthèse artificielle d'un ou plusieurs oligonucléotides correspondant à une ou plusieurs régions de ce domaine ;
(c) la synthèse artificielle d'un ou plusieurs oligonucléotides correspondant à la région de jonction entre les deux domaines séparés par le domaine épissé ; et
(d) la reproduction des étapes (a) à (c) ci-dessus avec d'autres événements d'épissages caractéristiques de ladite situation physiopathologique.

19. Procédé selon la revendication 14, **caractérisé en ce que** les acides nucléiques sont clonés dans des vecteurs.

20. Procédé selon la revendication 18, **caractérisé en ce que** la situation physiopathologique est choisie parmi l'apoptose, la neurodégénérescence, la toxicité et la prolifération.

21. Procédé selon l'une des revendications 13 à 20, **caractérisé en ce que** le support est composé d'un filtre, d'une membrane ou d'une puce.

22. Utilisation d'une composition obtenue par un procédé selon l'une des revendications 13 à 21 pour l'identification de molécules d'intérêt thérapeutique ou diagnostique.

23. Utilisation d'une composition obtenue par un procédé selon l'une des revendications 13 à 21 pour l'identification de protéines ou domaines protéiques affectés dans une pathologie.

24. Utilisation selon la revendication 23 pour l'identification de domaines antigéniques spécifiques de protéines impliquées dans une pathologie.

25. Utilisation selon la revendication 22, pour évaluer la toxicité d'un composé.

26. Utilisation selon la revendication 22, pour évaluer l'efficacité thérapeutique d'un composé.

27. Utilisation selon la revendication 22, pour évaluer la réponse d'un patient à un composé ou traitement test.

28. Utilisation d'une composition comprenant un ensemble d'oligonucléotides simple-brin distincts, d'une longueur comprise entre 5 et 100-mères, comprenant une séquence complémentaire et spécifique d'exons ou d'introns alternatifs distincts de gènes ou d'ARNm caractéristiques d'une situation physiologique, lesdits exons ou introns étant identifiables notamment selon le procédé de l'une quelconque des revendications 1 à 12, pour déterminer la capacité d'un sujet à répondre à un composé ou traitement donné.

29. Utilisation d'une composition comprenant un ensemble d'oligonucléotides simple-brin distincts, d'une longueur comprise entre 5 et 100-mères, comprenant une séquence complémentaire et spécifique d'exons ou d'introns alternatifs distincts de gènes ou d'ARNm caractéristiques d'une situation physiologique, lesdits exons ou introns étant identifiables notamment selon le procédé de l'une quelconque des revendications 1 à 12, pour déterminer la prédisposition d'un sujet à une pathologie.

30. Utilisation d'une composition comprenant un ensemble d'oligonucléotides simple-brin distincts, d'une longueur comprise entre 5 et 100-mères, comprenant une séquence complémentaire et spécifique d'exons ou d'introns alternatifs distincts de gènes ou d'ARNm caractéristiques d'une situation physiologique, lesdits exons ou introns étant identifiables notamment selon le procédé de l'une quelconque des revendications 1 à 12, pour déterminer la toxicité d'un composé ou traitement.

31. Utilisation selon l'une des revendications 28 à 30, **caractérisée en ce que** les oligonucléotides sont utilisés comme amorces.

32. Utilisation selon l'une des revendications 28 à 31, **caractérisée en ce que** les oligonucléotides sont spécifiques de régions de jonction entre deux domaines séparés par un domaine épissé.

33. Procédé de détermination ou d'évaluation de la toxicité d'un composé test sur un échantillon biologique donné, comprenant l'hybridation:
- d'une composition obtenue par un procédé selon l'une des revendications 13 à 21, ladite composition comprenant des acides nucléiques spécifiques d'exons ou d'introns alternatifs de gènes ou d'ARNm caractéristiques dudit échantillon biologique non traité à l'état sain et dudit échantillon biologique à un ou différents stades de toxicité résultant d'un traitement dudit échantillon avec un composé toxique de référence, avec,
- une préparation d'acides nucléiques de l'échantillon biologique traité par ledit composé test, et
- l'évaluation du potentiel toxique du composé test par analyse du degré d'hybridation de ladite préparation avec ladite composition.

34. Procédé selon la revendication 33, **caractérisé en ce que** l'échantillon biologique est une culture d'hépatocytes, de cellules épithéliales rénales ou de cellules endothéliales, traitée ou non par un agent toxique.

35. Procédé selon la revendication 33, **caractérisé en ce que** l'échantillon biologique est une culture de peau traitée ou non par des agents toxiques ou irritants.

36. Procédé de détermination ou d'évaluation de l'efficacité thérapeutique d'un composé test sur un échantillon biologique donné comprenant l'hybridation :
- d'une composition obtenue par un procédé selon l'une des revendications 13 à 21, ladite composition comprenant des acides nucléiques spécifiques d'exons ou d'introns alternatifs de gènes ou d'ARNm caractéristiques dudit échantillon biologique non traité et d'un échantillon biologique traité avec un composé thérapeutique de référence, avec
- une préparation d'acides nucléiques de l'échantillon biologique traité par ledit composé test, et
- l'évaluation du potentiel thérapeutique du composé test par analyse du degré d'hybridation de ladite préparation avec ladite composition.

37. Procédé de détermination ou d'évaluation de la réponse d'un patient à un composé ou traitement test comprenant:
- l'hybridation d'une préparation d'acides nucléiques d'un échantillon biologique pathologique dudit patient avec une composition obtenue par un procédé selon l'une des revendications 13 à 21, ladite composition comprenant des acides nucléiques spécifiques d'exons ou d'introns alternatifs de gènes ou d'ARNm caractéristiques d'un échantillon biologique répondeur audit composé/traitement et d'un échantillon biologique non-répondeur ou mal-répondeur audit composé/traitement, et
- l'évaluation du potentiel répondeur du patient par analyse du degré d'hybridation de ladite préparation avec ladite composition.

38. Procédé selon la revendication 37, pour la détermination ou l'évaluation de la réponse d'un patient à un composé ou traitement antitumoral.

39. Procédé selon la revendication 38, pour la détermination ou l'évaluation de la réponse d'un patient à un traitement antitumoral par transfert du gène p53 sauvage.

## Patentansprüche

1. Verfahren zur Identifizierung und/oder Klonierung von Nucleinsäure-Regionen, die für qualitative genetische Unterschiede zwischen zwei biologischen Proben repräsentativ sind, umfassend einen Schritt der Hybridisierung zwischen der RNA oder doppelsträngiger cDNA, die aus einer ersten biologischen Probe stammt, und der cDNA, die aus einer zweiten biologischen Probe stammt, und die Identifizierung und/oder Klonierung, aus den gebildeten Hybridisierungskomplexen, von nicht gepaarten Nucleinsäure-Regionen, wobei besagte Regionen Nucleinsäuren umfassen, die für qualitative genetische Unterschiede zwischen den beiden Proben repräsentativ sind.

2. Verfahren gemäß Anspruch 1, **dadurch gekennzeichnet, dass** es umfasst:
(a) die Hybridisierung von RNA, die aus der ersten Probe (Testzustand) stammt, mit der cDNA, die aus der zweiten Probe (Referenzzustand) stammt;
(b) die Hybridisierung von RNA, die aus der zweiten Probe (Referenzzustand) stammt, mit der cDNA, die aus der ersten Probe (Testzustand) stammt; und
(c) die Identifizierung und/oder Klonierung, aus den in (a) und (b) gebildeten Hybriden, von Nucleinsäuren, die qualitativen genetischen Unterschieden entsprechen.

3. Verfahren gemäß Anspruch 1 oder 2, **dadurch gekennzeichnet, dass** die Hybridisierungen zwischen RNA und einzelsträngiger cDNA erfolgen und dass es die Identifizierung und/oder Klonierung von nicht gepaarten RNA-Regionen umfasst.

4. Verfahren gemäß Anspruch 1, **dadurch gekennzeichnet, dass** es die Hybridisierung zwischen doppelsträngiger cDNA, die aus einer ersten biologischen Probe stammt, und einzelsträngiger cDNA, die aus einer zweiten biologischen Probe stammt, umfasst.

5. Verfahren gemäß den Ansprüchen 3 oder 4, **dadurch gekennzeichnet, dass** die Klonierung der Nucleinsäuren die inverse Transkription und/oder die Amplifikation mit Hilfe zufälliger oder halbzufälliger Primer umfasst, insbesondere Primer mit der Sequenz GAGAAGCGTTATNNNNNNNWXYZ, in der N anzeigt, dass jede der vier Basen an der angegebenen Position in zufälliger Weise stehen kann, W, X und Y jeweils für eine bestimmte Base stehen und Z entweder für eine bestimmte Base oder für eine 3'-OH-Gruppe steht.

6. Verfahren gemäß einem der Ansprüche 1 bis 5, **dadurch gekennzeichnet, dass** die biologische Probe aus Zellen, einem Gewebe, einem Organ oder einer Biopsie besteht.

7. Verfahren gemäß einem der Ansprüche 1 bis 6 zur Identifizierung und/oder Klonierung differenzieller alternativer Spleißungen zwischen Tumorzellen und Nichttumorzellen.

8. Verfahren gemäß einem der Ansprüche 1 bis 6 zur Identifizierung und/oder Klonierung differenzieller alternativer Spleißungen zwischen mit einer Testverbindung behandelten Zellen und nichtbehandelten Zellen.

9. Verfahren gemäß einem der Ansprüche 1 bis 6 zur Identifizierung und/oder Klonierung differenzieller alternativer Spleißungen zwischen Apoptosezellen und nicht apoptotischen Zellen.

10. Verfahren gemäß einem der Ansprüche 1 bis 9, **dadurch gekennzeichnet, dass** die Hybridisierung in flüssiger Phase durchgeführt ist.

11. Verfahren zur Identifizierung und/oder Klonierung von Nucleinsäure-Regionen, die in zwei physiologischen Zuständen A und B differenziell gespleißt sind, umfassend
(a) die Heteroduplex-Bildung in flüssiger Phase zwischen der Messenger-RNA, die vom Zustand A stammt, und der cDNA, die einerseits vom Zustand B stammt;
(b) die Heteroduplex-Bildung in flüssiger Phase zwischen der Messenger-RNA, die vom Zustand B stammt, und der cDNA, die andererseits vom Zustand A stammt;
(c) die Identifizierung und/oder Klonierung der nicht gepaarten RNA-Regionen in den in (a) und (b) erhaltenen Heteroduplexen.

12. Verfahren zur Identifizierung und/oder Klonierung von Nucleinsäure-Regionen, die in zwei physiologischen Zuständen A und B differenziell gespleißt sind, umfassend
(a) die Heteroduplex-Bildung zwischen der Messenger-RNA, die vom Zustand A stammt, und der cDNA, die einerseits vom Zustand B stammt, wobei die RNA oder die cDNA auf einem Träger immobilisiert ist;
(b) die Heteroduplex-Bildung zwischen der Messenger-RNA, die vom Zustand B stammt, und der cDNA, die andererseits vom Zustand A stammt, wobei die RNA oder die cDNA auf einem Träger immobilisiert ist; und
(c) die Identifizierung und/oder Klonierung der nicht gepaarten RNA-Regionen in den in (a) und (b) erhaltenen Heteroduplexen.

13. Verfahren zur Herstellung einer Zusammensetzung, umfassend einen Träger, auf dem Nucleinsäuren präsentiert sind, umfassend:
- die Präparation von unterschiedlichen Nucleinsäuren, umfassend eine Sequenz, die komplementär und spezifisch für alternierende Exons oder Introns ist, die sich von Genen oder mRNA unterscheiden, die für einen physiologischen Zustand charakteristisch sind, wobei besagte Exons oder Introns insbesondere gemäß dem Verfahren nach einem der Ansprüche 1 bis 12 identifizierbar sind, und
- die Präsentation dieser Nucleinsäuren auf einem Träger.

14. Verfahren gemäß Anspruch 13, **dadurch gekennzeichnet, dass** die Nucleinsäuren cDNA umfassen.

15. Verfahren gemäß Anspruch 13, **dadurch gekennzeichnet, dass** die Nucleinsäuren Oligonucleotide umfassen.

16. Verfahren gemäß Anspruch 15, **dadurch gekennzeichnet, dass** die Nucleinsäuren Oligonucleotide umfassen, die für Verbindungsregionen zwischen zwei Domänen spezifisch sind, die von einer gespleißten Domäne getrennt sind.

17. Verfahren gemäß Anspruch 16, **dadurch gekennzeichnet, dass** die Oligonucleotide einzelsträngig sind und eine Länge zwischen einschließlich 5 und 100-mer besitzen.

18. Verfahren gemäß Anspruch 15, 16 oder 17, **dadurch gekennzeichnet, dass** besagte Oligonucleotide mit einem Verfahren erhalten sind, umfassend:
(a) Identifizierung eines für einen physiopathologischen Zustand charakteristischen Spleißereignisses und der Sequenz der gespleißten Domäne;
(b) die künstliche Synthese eines oder mehrerer Oligonucleotide, die einer oder mehreren Regionen dieser Domäne entsprechen;
(c) die künstliche Synthese eines oder mehrerer Oligonucleotide, die der Verbindungsregion zwischen den zwei Domänen entsprechen, die von einer gespleißten Domäne getrennt sind; und
(d) die Wiederholung der obigen Schritte (a) bis (c) mit anderen für besagten physiopathologischen Zustand charakteristischen Spleißereignissen.

19. Verfahren gemäß Anspruch 14, **dadurch gekennzeichnet, dass** die Nucleinsäuren in Vektoren kloniert sind.

20. Verfahren gemäß Anspruch 18, **dadurch gekennzeichnet, dass** der physiopathologische Zustand aus Apoptose, Neurodegeneration, Toxizität und Proliferation ausgewählt ist.

21. Verfahren gemäß einem der Ansprüche 13 bis 20, **dadurch gekennzeichnet, dass** der Träger aus einem Filter, einer Membran oder einem Chip besteht.

22. Verwendung einer Zusammensetzung, die mit einem Verfahren gemäß einem der Ansprüche 13 bis 21 erhalten ist, zur Identifizierung von Molekülen von therapeutischem oder diagnostischem Interesse.

23. Verwendung einer Zusammensetzung, die mit einem Verfahren gemäß einem der Ansprüche 13 bis 21 erhalten ist, zur Identifizierung von Proteinen oder Protein-Domänen, die bei einer Krankheit verändert sind.

24. Verwendung gemäß Anspruch 23 zur Identifizierung von Antigen-Domänen, die für in einer Krankheit implizierte Proteine spezifisch sind.

25. Verwendung gemäß Anspruch 22 zur Bewertung der Toxizität einer Verbindung.

26. Verwendung gemäß Anspruch 22 zur Bewertung der therapeutischen Wirksamkeit einer Verbindung.

27. Verwendung gemäß Anspruch 22 zur Bewertung der Reaktion eines Patienten auf eine Testverbindung oder Testbehandlung.

28. Verwendung einer Zusammensetzung, umfassend eine Gruppe unterschiedlicher einzelsträngiger Oligonucleotide mit einer Länge zwischen einschließlich 5 und 100-mer, umfassend eine Sequenz, die für alternierende Exons oder Introns komplementär und spezifisch ist, die sich von Genen oder mRNA unterscheiden, die für einen physiologischen Zustand charakteristisch sind, wobei besagte Exons oder introns insbesondere gemäß dem Verfahren nach einem der Ansprüche 1 bis 12 identifizierbar sind, um die Fähigkeit einer Person zu bestimmen, auf eine bestimmte Verbindung oder Behandlung zu reagieren.

29. Verwendung einer Zusammensetzung, umfassend eine Gruppe unterschiedlicher einzelsträngiger Oligonucleotide mit einer Länge zwischen einschließlich 5 und 100-mer, umfassend eine Sequenz, die für alternierende Exons oder Introns komplementär und spezifisch ist, die sich von Genen oder mRNA unterscheiden, die für einen physiologischen Zustand charakteristisch sind, wobei besagte Exons oder introns insbesondere gemäß dem Verfahren nach einem der Ansprüche 1 bis 12 identifizierbar sind, um die Prädisposition einer Person für eine Krankheit zu bestimmen.

30. Verwendung einer Zusammensetzung, umfassend eine Gruppe unterschiedlicher einzelsträngiger Oligonucleotide mit einer Länge zwischen einschließlich 5 und 100-mer, umfassend eine Sequenz, die für alternierende Exons oder introns komplementär und spezifisch ist, die sich von Genen oder mRNA unterscheiden, die für einen physiologischen Zustand charakteristisch sind, wobei besagte Exons oder Introns insbesondere gemäß dem Verfahren nach einem der Ansprüche 1 bis 12 identifizierbar sind, um die Toxizität einer Verbindung oder Behandlung zu bestimmen.

31. Verwendung gemäß einem der Ansprüche 28 bis 30, **dadurch gekennzeichnet, dass** die Oliginucleotide als Primer verwendet sind.

32. Verwendung gemäß einem der Ansprüche 28 bis 31, **dadurch gekennzeichnet, dass** die Oliginucleotide für Verbindungsregionen zwischen zwei Domänen spezifisch sind, die von einer gespleißten Domäne getrennt sind.

33. Verfahren zur Bestimmung oder Bewertung der Toxizität einer Testverbindung für eine bestimmte biologische Probe, umfassend die Hybridisierung:
- einer Zusammensetzung, die mit einem Verfahren gemäß einem der Ansprüche 13 bis 21 erhalten ist, wobei besagte Zusammensetzung Nucleinsäuren umfasst, die für alternierende Exons oder Introns von Genen oder mRNA spezifisch sind, die für besagte nichtbehandelte biologische Probe in gesundem Zustand und für besagte biologische Probe in einem oder verschiedenen toxischen Stadien, die aus einer Behandlung besagter Probe mit einer toxischen Referenzverbindung resultieren, charakteristisch sind, mit
- einem Präparat von Nucleinsäuren von der biologischen Probe, die mit besagter Testverbindung behandelt ist, und
- die Bewertung des toxischen Potenzials der Testverbindung durch eine Analyse des Hybridisierungsgrads von besagtem Präparat mit besagter Zusammensetzung.

34. Verfahren gemäß Anspruch 33, **dadurch gekennzeichnet, dass** die biologische Probe eine Kultur von Hepatocyten, von Nierenepithelzellen oder Endothelzellen ist, die nichtbehandelt oder mit einem toxischen Agens behandelt ist.

35. Verfahren gemäß Anspruch 33, **dadurch gekennzeichnet, dass** die biologische Probe eine Hautkultur ist, die nichtbehandelt oder mit toxischen oder irritierenden Agenzien behandelt ist.

36. Verfahren zur Bestimmung oder Bewertung der therapeutischen Wirksamkeit einer Testverbindung auf eine bestimmte biologische Probe, umfassend die Hybridisierung:
- einer Zusammensetzung, die mit einem Verfahren gemäß einem der Ansprüche 13 bis 21 erhalten ist, wobei besagte Zusammensetzung Nucleinsäuren umfasst, die für alternierende Exons oder Introns von Genen oder mRNA spezifisch sind, die für besagte nichtbehandelte biologische Probe und für eine mit einer therapeutischen Referenzverbindung behandelte biologische Probe charakteristisch sind, mit
- einem Präparat von Nucleinsäuren von der biologischen Probe, die mit besagter Testverbindung behandelt ist, und
- die Bewertung des toxischen Potenzials der Testverbindung durch eine Analyse des Hybridisierungsgrads von besagtem Präparat mit besagter Zusammensetzung.

37. Verfahren zur Bestimmung oder Bewertung einer Reaktion eines Patienten auf eine Testverbindung oder Testbehandlung, umfassend
- die Hybridisierung eines Präparats von Nucleinsäuren von einer pathologischen biologischen Probe besagten Patienten mit einer Zusammensetzung, die mit einem Verfahren gemäß einem der Ansprüche 13 bis 21 erhalten ist, wobei besagte Zusammensetzung Nucleinsäuren umfasst, die für alternierende Exons oder Introns von Genen oder mRNA spezifisch sind, die für eine biologische Probe, die auf besagte Zusammensetzung/Behandlung reagiert, und für eine biologische Probe, die auf besagte Zusammensetzung/Behandlung schlecht oder nicht reagiert, charakteristisch sind, und
- die Bewertung des Reaktionspotenzials des Patienten durch eine Analyse des Hybridisierungsgrads besagten Präparats mit besagter Zusammensetzung.

38. Verfahren gemäß Anspruch 37 zur Bestimmung oder Bewertung der Reaktion eines Patienten auf eine Antitumor-Verbindung oder Antitumor-Behandlung.

39. Verfahren gemäß Anspruch 38 zur Bestimmung oder Bewertung der Reaktion eines Patienten auf eine Antitumor-Behandlung durch Übertragung des Wildtyp p53-Gens.

## Claims

1. A method for identifying and/or cloning nucleic acids regions representative of qualitative genetic differences between two biological samples, comprising a step of hybridizing the RNAs or double-stranded cDNAs from a first biological sample with the cDNAs from a second biological sample and, identifying and/or cloning, from the complexes formed by hybridization, unpaired nucleic acid regions, said regions comprising nucleic acids representative of qualitative genetic differences between the two samples.

2. A method according to claim 1, wherein said method comprises:
a) hybridizing the RNAs from the first sample (test situation) with the cDNAs from the second sample (reference situation);
b) hybridizing the RNAs from the second sample (reference situation) with the cDNAs from the first sample (test situation) ; and
c) identifying and/or cloning, from the hybrids formed in (a) and (b), nucleic acids corresponding to qualitative genetic differences.

3. A method according to claim 1 or 2, wherein the hybridizations are performed between RNAs and single stranded cDNAs and wherein the method comprises identifying and/or cloning unpaired RNA regions.

4. A method according to claim 1, wherein the method comprises hybridizing double stranded cDNAs from a first biological sample with single stranded cDNAs from a second biological sample.

5. A method according to claims 3 or 4, wherein cloning nucleic acids comprises reverse transcription and/or amplification by means of random or semi-random primers, particularly primers with sequence GAGAAGCGTTATNNNNNNNWXYZ in which N indicates that each of the four bases may be present randomly at the indicated position, W, X and Y each designate a defined base, and Z designates either a defined base, or a 3'-OH group.

6. A method according to any one of claims 1 to 5, wherein the biological sample consists of cells, a tissue, an organ or a biopsy sample.

7. A method according to any one of claims 1 to 6, for identifying and/or cloning differential alternative splicings between tumoral and non tumoral cells.

8. A method according to any one of claims 1 to 6, for identifying and/or cloning differential alternative splicings between cells treated by a test compound and non treated cells.

9. A method according to any one of claims 1 to 6, for identifying and/or cloning differential alternative splicings between cells undergoing apoptosis and non apoptotic cells.

10. A method according to any one of claims 1 to 9, wherein the hybridization is performed in a liquid phase.

11. A method for identifying and/or cloning differentially spliced nucleic acid regions between two physiological conditions A and B, comprising :
(a) generating heteroduplex structures in a liquid phase between the messenger RNAs derived from condition A and the cDNAs derived from condition B on the one hand;
(b) generating heteroduplex structures in a liquid phase between the messenger RNAs derived from condition B and the cDNAs derived from condition A on the other hand ; and
(c) identifying and/or cloning unpaired RNA regions within the heteroduplex structures obtained in steps (a) and (b).

12. A method for identifying and/or cloning differentially spliced nucleic acid regions occurring between two physiological conditions A and B, comprising :
(a) generating heteroduplex structures between the messenger RNAs derived from condition A and the cDNAs derived from condition B on the one hand, the RNAs or cDNAs being fixed to a support material;
(b) generating heteroduplex structures between the messenger RNAs derived from condition B and the cDNAs derived from condition A on the other hand, the RNAs or cDNAs being fixed to a support material; and
(c) identifying and/or cloning unpaired RNA regions within the heteroduplex structures obtained in steps (a) and (b).

13. A method for preparing a composition comprising a support material on which nucleic acids are displayed, comprising
. preparing distinct nucleic acids comprising a sequence complementary to and specific of distinct alternative exons or introns of genes or mRNAs characteristic of a physiological situation, said exons or introns being identifiable for instance by the method of anyone of claims 1 to 12, and
. displaying said nucleic acids on a support.

14. The method of claim 13, wherein the nucleic acids comprise cDNAs.

15. The method of claim 13, wherein the nucleic acids comprise oligonucleotides.

16. The method of claim 15, wherein the nucleic acids comprise oligonucleotides specific for junction regions between two domains separated by a spliced domain.

17. The method of claim 16, wherein the oligonucleotides are single-stranded and are between 5 and 100-mer in length.

18. The method of claim 15, 16 or 17, wherein said oligonucleotides are obtained by a method comprising:
(a) identifying a splicing event characteristic of a physiopathological condition and the sequence of the spliced domain,
(b) artificially synthesizing one or several oligonucleotides corresponding to one or several regions of said domain,
(c) artificially synthesizing one or several oligonucleotides corresponding to the junction region between the two domains separated by the spliced domain, and
(d) repeating steps (a) to (c) above with other splicing events characteristic of said physiopathological condition.

19. The method of claim 14, wherein the nucleic acids are cloned in vectors.

20. The method of claim 18, wherein the physiopathological condition is selected from apoptosis, neurodegeneration, toxicity and proliferation.

21. The method of any one of claims 13 to 20, wherein the support material is selected from a filter, a membrane and a chip.

22. The use of a composition obtained by a method according to any one of claims 13 to 21 for identifying molecules of therapeutic or diagnostic interest.

23. The use of a composition obtained by a method according to any one of claims 13 to 21 for identifying proteins or protein domains altered in a pathology.

24. The use of claim 23 for identifying antigenic domains specific of proteins involved in a pathology.

25. The use of claim 22 to evaluate the toxicity of a compound.

26. The use of claim 22, to evaluate the therapeutic efficacy of a compound.

27. The use of claim 22 to evaluate the response of a patient to a test compound or treatment.

28. The use of a composition comprising a set of distinct single-stranded oligonucleotides, of between 5 and 100-mer in length, comprising a sequence complementary to and specific of distinct alternative exons or introns of genes or mRNAs characteristic of a physiological situation, said exons or introns being identifiable for instance by the method of anyone of claims 1 to 12, to evaluate the capacity of a subject to respond to a given compound or treatment.

29. The use of a composition comprising a set of distinct single-stranded oligonucleotides, of between 5 and 100-mer in length, comprising a sequence complementary to and specific of distinct alternative exons or introns of genes or mRNAs characteristic of a physiological situation, said exons or introns being identifiable for instance by the method of anyone of claims 1 to 12, to evaluate the predisposition of a subject to a pathology.

30. The use of a composition comprising a set of distinct single-stranded oligonucleotides, of between 5 and 100-mer in length, comprising a sequence complementary to and specific of distinct alternative exons or introns of genes or mRNAs characteristic of a physiological situation, said exons or introns being identifiable for instance by the method of anyone of claims 1 to 12, to evaluate the toxicity of a compound or treatment.

31. The use of any one of claims 28 to 30, wherein the oligonucleotides are used as primers.

32. The use of any one of claims 28 to 31, wherein the oligonucleotides are specific for junction regions between two domains separated by a spliced domain.

33. A method of determining or assessing the toxicity of a test compound to a biological sample, comprising hybridizing:
- a composition obtained by a method according to any one of claims 13 to 21, said composition comprising nucleic acids specific of alternative exons or introns of genes or mRNAs characteristic of said biological sample not treated and in healthy condition and of said biological sample at one or various stages of toxicity resulting from a treatment of said sample with a reference toxic compound, with
- a nucleic acid preparation of the biological sample treated with said test compound; and
- assessing the toxicity of said test compound by analysing the extent of hybridization of said preparation with said composition.

34. A method according to claim 33, wherein the biological sample is a culture of hepatocytes, renal epithelial cells or endothelial cells, either subjected or not to treatment by a toxic agent.

35. A method according to claim 33, wherein the biological sample is a skin culture either subjected or not to treatment by toxic agents or irritants.

36. A method of determining or assessing the therapeutic efficacy of a test compound to a biological sample, comprising hybridizing:
- a composition obtained by a method according to any one of claims 13 to 21, said composition comprising nucleic acids specific of alternative exons or introns of genes or mRNAs characteristic of said biological sample not treated and of a biological sample treated with a reference therapeutic compound, with
- a nucleic acid preparation of the biological sample treated with said test compound; and
- assessing the therapeutic potential of said test compound by analysing the extent of hybridization of said preparation with said composition.

37. A method of determining or assessing the responsiveness of a patient to a test compound or treatment, comprising:
- hybridizing a nucleic acid preparation of a pathological biological sample of the patient with a composition obtained by a method according to any one of claims 13 to 21, said composition comprising nucleic acids specific of alternative exons or introns of genes or mRNAs characteristic of a biological sample responding to said compound/treatment and of a biological sample non- or poorly-responding to said compound/treatment, and
- assessing the responsiveness of the patient by analysing the extent of hybridization of said preparation with said composition.

38. A method according to claim 37 for determining or assessing the response of a patient to an anti-tumoral compound or treatment.

39. A method according to claim 38 for determining or assessing the response of a patient to an antitumoral treatment through wild type p53 gene transfer.
